# EUROPEAN PATENT SPECIFICATION

(11) **EP 4 034 248 B1**
(45) Date of publication and mention of the grant of the patent: **20.05.2026**
(21) Application number: 20790106.7
(22) Date of filing: 24.09.2020
(51) Int. Cl.: A61P 35/00, A61P 37/04, C12Q 1/68

(54) **COMPOSITE BIOMARKER FOR CANCER THERAPY**
ZUSAMMENGESETZTER BIOMARKER FÜR DIE KREBSTHERAPIE
BIOMARQUEUR COMPOSITE POUR LE TRAITEMENT DU CANCER

(30) Priority: 25.09.2019 US 201962905933 P
(43) Date of publication of application: 03.08.2022
(73) Proprietor: Bristol-Myers Squibb Company, Princeton, NJ 08543 (US)
(72) Inventor: SANTUCCI PEREIRA DEL BUONO, Julia, Princeton, New Jersey 08543 (US); NELSON, David Martin, Princeton, New Jersey 08543 (US); KANDOUSSI, Enzo Yacobi, Princeton, New Jersey 08543 (US); FISCHER, Bruce S., Princeton, New Jersey 08543 (US); WIND-ROTOLO, Megan M., Princeton, New Jersey 08543 (US); ISHII, Yuko, Princeton, New Jersey 08543 (US); GREENAWALT, Danielle Marie, Princeton, New Jersey 08543 (US)
(74) Representative: Mewburn Ellis LLP
(86) International application number: PCT/US2020/052531
(87) International publication number: WO 2021/062018

(56) References cited:
- US-A1- 2010 055 111
- MARK AYERS ET AL: "IFN-[gamma]-related mRNA profile predicts clinical response to PD-1 blockade", THE JOURNAL OF CLINICAL INVESTIGATION, vol. 127, no. 8, 26 June 2017 (2017-06-26), GB, pages 2930 - 2940, XP055608325, ISSN: 0021-9738, DOI: 10.1172/JCI91190
- MIE M ET AL: "In vitro and in vivo characterization of KHK2455, a highly potent and selective indoleamine 2,3-dioxygenase 1 (IDO1) inhibitor with a novel mechanism of action", JOURNAL FOR IMMUNOTHERAPY OF CANCER 20171101 BIOMED CENTRAL LTD. NLD, vol. 5, no. Supplement 2, 7 November 2017 (2017-11-07), XP055469477, ISSN: 2051-1426
- MARGOLIS NAAMA ET AL: "Reprogramming lymphocytes for the treatment of melanoma: From biology to therapy", ADVANCED DRUG DELIVERY REVIEWS, ELSEVIER, AMSTERDAM, NL, vol. 141, 15 February 2019 (2019-02-15), pages 104 - 124, XP085750601, ISSN: 0169-409X, [retrieved on 20190702], DOI: 10.1016/J.ADDR.2019.06.005
- J LUKE ET AL: "Interferon [gamma] (IFN-[gamma]) gene signature and tryptophan 2,3-dioxygenase 2 (TDO2) gene expression: a potential predictive composite biomarker for linrodostat mesylate (BMS-986205; indoleamine 2,3- dioxygenase 1 inhibitor [IDO1i]) 1 + nivolumab (NIVO)", ANNALS OF ONCOLOGY., vol. 30, no. S5, 27 September 2019 (2019-09-27), NL, pages v760, XP055751348, ISSN: 0923-7534, DOI: 10.1093/annonc/mdz268
- KRISTEN LAUNING ET AL: "Characterization of IDO1 and TDO2 in pediatric central nervous system tumors | Cancer Immunology Research", SECOND CRI-CIMT-EATI-AACR INTERNATIONAL CANCER IMMUNOTHERAPY CONFERENCE: TRANSLATING SCIENCE INTO SURVIVAL; SEPTEMBER 25-28, 2016; NEW YORK,, 25 September 2016 (2016-09-25), XP055752604, Retrieved from the Internet <URL:https://cancerimmunolres.aacrjournals.org/content/4/11_Supplement/A051> [retrieved on 20201120]

## Description

### FIELD OF THE DISCLOSURE

The present disclosure provides immunotherapies for use in a method for treating a subject afflicted with a cancer.

### BACKGROUND OF THE DISCLOSURE

Human cancers harbor numerous genetic and epigenetic alterations, generating neoantigens potentially recognizable by the immune system (Sjoblom et al., Science (2006) 314(5797):268-274). The adaptive immune system, comprised of T and B lymphocytes, has powerful anti-cancer potential, with a broad capacity and exquisite specificity to respond to diverse tumor antigens. Further, the immune system demonstrates considerable plasticity and a memory component. The successful harnessing of all these attributes of the adaptive immune system would make immunotherapy unique among all cancer treatment modalities.

Until recently, cancer immunotherapy had focused substantial effort on approaches that enhance anti-tumor immune responses by adoptive-transfer of activated effector cells, immunization against relevant antigens, or providing non-specific immune-stimulatory agents such as cytokines. In the past decade, however, intensive efforts to develop specific immune checkpoint pathway inhibitors have begun to provide new immunotherapeutic approaches for treating cancer, including the development of antibodies such as nivolumab and pembrolizumab (formerly lambrolizumab; USAN Council Statement, 2013) that bind specifically to the Programmed Death -1 (PD-1) receptor and block the inhibitory PD-1/PD-1 ligand pathway (Topalian *et al.,* 2012a, b; Topalian *et al.,* 2014; Hamid *et al.,* 2013; Hamid and Carvajal, 2013; McDermott and Atkins, 2013). Approaches for manipulation of lymphocytes used to treat melanoma are described (Margolis et al., Adv Drug Deliv Rev 2019(15):104-124).

PD-1 is a key immune checkpoint receptor expressed by activated T and B cells and mediates immunosuppression. PD-1 is a member of the CD28 family of receptors, which includes CD28, CTLA-4, ICOS, PD-1, and BTLA. Two cell surface glycoprotein ligands for PD-1 have been identified, Programmed Death Ligand-1 (PD-L1) and Programmed Death Ligand-2 (PD-L2), that are expressed on antigen-presenting cells as well as many human cancers and have been shown to downregulate T cell activation and cytokine secretion upon binding to PD-1. Inhibition of the PD-1/PD-L1 interaction mediates potent antitumor activity in preclinical models (U.S. Patent Nos. 8,008,449 and 7,943,743), and the use of antibody inhibitors of the PD-1/PD-L1 interaction for treating cancer has entered clinical trials (Brahmer *et al.,* 2010; Topalian *et al.,* 2012a; Topalian *et al.,* 2014; Hamid *et al.,* 2013; Brahmer *et al.,* 2012; Flies *et al.,* 2011; Pardoll, 2012; Hamid and Carvajal, 2013).

Nivolumab (formerly designated 5C4, BMS-936558, MDX-1106, or ONO-4538) is a fully human IgG4 (S228P) PD-1 immune checkpoint inhibitor antibody that selectively prevents interaction with PD-1 ligands (PD-L1 and PD-L2), thereby blocking the down-regulation of antitumor T-cell functions (U.S. Patent No. 8,008,449; Wang *et al.,* 2014). Nivolumab has shown activity in a variety of advanced solid tumors, including renal cell carcinoma (renal adenocarcinoma, or hypernephroma), melanoma, and non-small cell lung cancer (NSCLC) (Topalian *et al.,* 2012a; Topalian *et al.,* 2014; Drake *et al.,* 2013; WO 2013/173223). Combination treatments comprising nivolumab and the indoleamine 2,3-dioxygenase 1 (IDO1) inhibitor linrodostat (BMS-986205) are currently undergoing clinical trials (ClinicalTrials.gov identifier NCT02658890).

Indoleamine 2,3-dioxygenase (IDO1) is a heme-containing enzyme that catalyzes the O2-dependent oxidation of L-tryptophan to N-formylkynurenine. This reaction is the first and rate-limiting step in the kynurenine pathway, which leads to the production of nicotinamide adenine dinucleotide from the degradation of tryptophan. IDO1 is upregulated by *IFNγ* and further upregulated by anti-PD-1 treatment (e.g. nivolumab) in patients (Moon YW, et al. J Immunother Cancer 2015;3:51; Liu M et al. J Hematol Oncol 2018;11(1):100). IDO1 and TDO2 catalyze the rate-limiting step in the tryptophan pathway to produce kynurenine (KYN) (Moon YW, et al. J Immunother Cancer 2015;3:51; Liu M, et al. J Hematol Oncol 2018;11(1):100). IDO1 and TDO2 were also characterized in pediatric central nervous system tumors (Lauing et al., Cancer Immunol Res 2016:4(11):A051). Linrodostat inhibits metabolism of tryptophan to kynurenine, thereby reducing kynurenine levels and potentially increasing effector T-cell function (Hunt JT, et al. Clin Cancer Res 2017;77(suppl.). Abstract 4964). See, e.g., U.S. Pat. No. 9,643,972. KHK2455 is an IDO1 inhibitor that was shown to inhibit kyn production in preclinical models and to demonstrate synergistic inhibition of tumor growth in a mouse tumor model with anti-CTLA-4 antibody (Mie et al., J Immunotherapy Cancer 2017:5(2):83).

The immune system and response to immuno-therapy are complex. Additionally, anti-cancer agents can vary in their effectiveness based on the unique patient characteristics. Accordingly, there is a need for targeted therapeutic strategies that identify patients who are more likely to respond to a particular anti-cancer agent and, thus, improve the clinical outcome for patients diagnosed with cancer.

### SUMMARY

The present invention is as defined in the appended claims. It is directed to an anti-PD-1 antagonist for use in combination with an indoleamine 2,3-dioxygenase 1 (IDO1) inhibitor in a method for treating a human subject afflicted with a cancer, wherein the subject is identified as exhibiting a combined biomarker comprising (a) a high interferon gamma (IFNγ) inflammatory signature score and (b) a low tryptophan 2,3-dioxygenase 2 (TDO2) gene expression score prior to administration of the anti-PD-1 antagonist and IDO1 inhibitor; and wherein the *IFNγ* inflammatory signature score is determined by measuring the expression of a panel of inflammatory genes comprising IFNy-related genes ("*IFN*γ inflammatory gene panel") in a tumor tissue biopsy sample obtained from the subject, wherein the cancer is a bladder cancer or a squamous cell carcinoma of the head and neck (SCCHN); wherein the inflammatory genes comprise *IFNγ, CXCL10, HLA-DRA, IDO1, STATI, CCR5, CXCL11, GZMA,* and *PRF1*, and wherein the IDO1 inhibitor is selected from linrodostat mesylate ((2R)-N-(4-chlorophenyl)-2-(cis-4-(6-fluoroquinolin-4-yl)cyclohexyl)propanamide), linrodostat, indoximod, p-(3-benzofuranyl)-DL-alanine, p-[3-benzo(b)thienyl]-DL-alanine; 6-nitro-L-tryptophan, and any combination thereof.

The technical disclosure set out below may in some respects go beyond the scope of the claims and describe general subject matter, embodiments and examples that are not explicitly claimed to provide background and context for the claimed invention. Methods of treating cancer disclosed herein form part of the claimed subject matter insofar as they are uses of an anti-PD-1 antagonist in combination with an IDO1 as defined in the appended claims. Any other methods of treatment disclosed herein do not form part of the claimed subject matter and are merely part of the disclosure.

Accordingly, the present disclosure generally provides a method for treating a human subject afflicted with a cancer comprising administering an anti-PD-1 antagonist and an indoleamine 2,3-dioxygenase 1 (IDO1) inhibitor to the subject, wherein the subject is identified as exhibiting a combined biomarker comprising (a) a high IFNγ inflammatory signature score and (b) a low tryptophan 2,3-dioxygenase 2 (TDO2) gene expression score prior to the administration; and wherein the *IFNγ* inflammatory signature score is determined by measuring the expression of a panel of inflammatory genes comprising interferon gamma (*IFNγ*) related genes ("*IFNγ* inflammatory gene panel") in a sample obtained from the subject and calculating a score from the expression values.

The present disclosure also describes a method for treating a human subject afflicted with a cancer comprising (i) identifying a subject exhibiting a combined biomarker comprising (a) a high *IFNγ* inflammatory signature score and (b) a low *TDO2* gene expression score; and, (ii) administering to the subject an anti-PD-1 antagonist and an IDO1 inhibitor; wherein the *IFNγ* inflammatory signature score is determined by measuring the expression of *IFNγ* inflammatory gene panel in a sample obtained from the subject and calculating a score from the expression values.

The present disclosure also describes a method for identifying a human subject afflicted with a cancer suitable for treatment with an anti-PD-1 antagonist and an IDO1 inhibitor, the method comprising measuring an IFNy inflammatory signature score and TDO2 gene expression in a sample obtained from the subject and; wherein the *IFNγ* inflammatory signature score is determined by measuring the expression of an *IFNγ* inflammatory gene panel in a sample obtained from the subject and calculating a score from the expression values. In some aspects, the subject exhibits a combined biomarker comprising (a) a high *IFNγ* inflammatory signature score and (b) a low *TDO2* gene expression score. In some aspects, the method further comprises administering to the subject an anti-PD-1 antagonist and an IDO1 inhibitor.

The present disclosure also provides an IDO1 inhibitor for treating a cancer in combination with an anti-PD-1 antagonist in a human subject in need thereof, wherein the subject is identified as exhibiting a combined biomarker comprising (a) a high *IFNγ* inflammatory signature score and (b) a low *TDO2* gene expression score prior to the administration; and, wherein the *IFNγ* inflammatory signature score is determined by measuring the expression of an *IFNγ* inflammatory gene panel in a sample obtained from the subject and calculating a score from the expression values.

Also disclosed herein is a combined biomarker for identifying a human subject afflicted with a cancer suitable for treatment with an IDO1 inhibitor in combination with treatment using an anti-PD-1 antagonist, wherein the combined biomarker comprises (i) an *IFNγ* inflammatory signature score and (ii) a *TDO2* gene expression measured in a sample obtained from the subject; and, wherein the *IFNγ* inflammatory signature score is determined by measuring the expression of an *IFNγ* inflammatory gene panel in a sample obtained from the subject and calculating a score from the expression values. In some aspects, the subject exhibits a combined biomarker comprising (a) a high *IFNγ* inflammatory signature score and (b) a low *TDO2* gene expression score.

In some aspects, the *IFNγ* inflammatory gene panel consists essentially of 1 inflammatory gene, 2 inflammatory genes, 3 inflammatory genes, 4 inflammatory genes, 5 inflammatory genes, 6 inflammatory genes, 7 inflammatory genes, 8 inflammatory genes, 9 inflammatory genes, 10 inflammatory genes, 11 inflammatory genes, 12 inflammatory genes, 13 inflammatory genes, 14 inflammatory genes, 15 inflammatory genes, 16 inflammatory genes, 17 inflammatory genes, 18 inflammatory genes, 19 inflammatory genes, or 20 inflammatory genes. In some aspects, the inflammatory genes are selected from the group consisting of *IFNγ, CXCL10, HLA-DRA, CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STATI, HLA.E, CCR5, CXCL11, GZMA, PRF1, IR2RG, CD3D, CD2, ITGAL, TAGAP, CIITA, PTPRC, CD3E, GZMK, GZMB, PDCD1, SLAMF6, CXCL13,* and any combination thereof.

In some aspects, the *IFNγ* inflammatory gene panel consists of or consists essentially of
(i) *IFNy, CXCL10, CXCL9, HLA-DRA, IDO1,* and *STAT1;*
(ii) *IFNγ, CXCL10, CXCL9, HLA-DRA, IDO1, STATI, CCR5, CXCL11, GZMA,* and *PRF1;*
(iii) *CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STAT1,* and *HLA.E;* or
(vi) any combination thereof, or any combination of the genes in (i) to (iii).

In the present invention, the inflammatory genes comprise *IFNy, CXCL10, CXCL9, HLA-DRA, IDO1, STATI, CCR5, CXCL11, GZMA,* and *PRF1.*

In some aspects, the high *IFNγ* inflammatory signature score is characterized by an *IFNγ* inflammatory signature score that is greater than an average *IFNγ* inflammatory signature score, wherein the average *IFNγ* inflammatory signature score is determined by averaging the expression the genes of the *IFNγ* inflammatory gene panel in cancer samples obtained from a population of subjects afflicted with the cancer. In some aspects, the average *IFNγ* inflammatory signature score is determined by averaging the expression of the *IFNγ* inflammatory gene panel genes in cancer samples obtained from the population of subjects. In some aspects, the high *IFNγ* inflammatory signature score is characterized by an *IFNγ* inflammatory signature score that is higher than the average *IFNγ* inflammatory signature score of a reference sample.

In some aspects, the reference sample comprises a non-tumor tissue of the subject, a corresponding non-tumor tissue of the subject, or the corresponding tissue of subjects without a tumor. In some aspects, the high *IFNγ* inflammatory signature score is characterized by an *IFNγ* inflammatory signature score that is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 125%, at least about 150%, at least about 175%, at least about 200%, at least about 225%, at least about 250%, at least about 275%, or at least about 300% higher than the average *IFNγ* inflammatory signature score.

In some aspects, the *IFNγ* high inflammatory signature score is characterized by an *IFNγ* inflammatory signature score that is at least about 50% higher than the average *IFNγ* inflammatory signature score. In some aspects, the high *IFNγ* inflammatory signature score is characterized by an *IFNγ* inflammatory signature score that is at least about 75% higher than the average *IFNγ* inflammatory signature score.

In some aspects, the low *TDO2* gene expression score is characterized by a *TDO2* gene expression that is smaller than an average *TDO2* gene expression score, wherein the average *TDO2* gene expression score is determined by averaging the expression of the *TDO2* gene in cancer samples obtained from a population of subjects afflicted with the cancer. In some aspects, the average *TDO2* gene expression score is determined by averaging the expression of the *TDO2* genes in cancer samples obtained from the population of subjects. In some aspects, the low *TDO2* gene expression score is characterized by a *TDO2* gene expression that is lower than the average *TDO2* gene expression score of a reference sample. In some aspects, the reference sample comprises a non-tumor tissue of the subject, a corresponding non-tumor tissue of the subject, or the corresponding tissue of subjects without a tumor. In some aspects, the low *TDO2* gene expression score is characterized by a *TDO2* gene expression score that is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 125%, at least about 150%, at least about 175%, at least about 200%, at least about 225%, at least about 250%, at least about 275%, or at least about 300% lower than the average *TDO2* gene expression score.

In some aspects, the low *TDO2* gene expression score is characterized by a *TDO2* gene expression score that is at least about 50% lower than the average *TDO2* gene expression score. In some aspects, the low *TDO2* gene expression score is characterized by a *TDO2* gene expression score that is at least about 75% lower than the average *TDO2* gene expression score.

In some aspects, the cancer is a tumor. In some aspects, the tumor is a carcinoma. In some aspects, the tumor is selected from bladder cancer, cervical cancer, lung cancer, pancreatic cancer, kidney cancer, head and neck cancer, melanoma, endometrial cancer, hepatocellular carcinoma (HCC), or glioblastoma. In some aspects, the lung cancer is non-small cell lung cancer (NSCLC). In some aspects, the kidney cancer is renal cell carcinoma (RCC). In some aspects, the head and neck cancer is squamous cell carcinoma of the head and neck (SCCHN). In some aspects, the glioblastoma is glioblastoma multiforme (GBM). In some aspects, the tumor is not melanoma. In some aspects, the cancer is a hematological cancer. In some aspects, the hematological cancer is lymphoma. In some aspects, the lymphoma is diffuse large B-cell lymphoma (DLBCL). In the present invention, the cancer is a bladder cancer or a squamous cell carcinoma of the head and neck (SCCHN).

In some aspects, and in the present invention, the sample is a tumor tissue biopsy. In some aspects, the sample is obtained from the stroma of a tumor. In some aspects, the sample is a formalin-fixed paraffin-embedded tissue, a fresh-frozen tissue, or a blood sample.

In some aspects, the expression of the genes in the IFNγ inflammatory gene panel and/or *TDO2* gene expression is determined by detecting the presence gene mRNA, the presence of a protein encoded by the gene, or both. In some aspects, the presence of mRNA encoding the genes in the IFNγ inflammatory gene panel and/or the *TDO2* gene is determined using reverse transcriptase PCR. In some aspects, the presence of the protein encoded by the genes in the IFNγ inflammatory gene panel and/or the *TDO2* gene is determined using an immunohistochemistry (IHC) assay. In some aspects, the IHC assay is an automated IHC assay. In some aspects, the assay is a protein IHC assay including topology that is correlated to the IFNγ inflammatory gene panel.

In some aspects, the anti-PD-1 antagonist is an anti-PD-1 antibody or an antigen-binding portion thereof. In some aspects, the anti-PD-1 antibody or antigen-binding portion thereof cross-competes with nivolumab for binding to human PD-1. In some aspects, the anti-PD-1 antibody or antigen-binding portion thereof binds to the same epitope as nivolumab. In some aspects, the anti-PD-1 antibody is a chimeric, humanized or human monoclonal antibody or a portion thereof. In some aspects, the anti-PD-1 antibody comprises a heavy chain constant region which is of a human IgG1 or IgG4 isotype. In some aspects, the anti-PD-1 antibody comprises nivolumab, pembrolizumab, or an antigen-binding portion thereof. In some aspects, the anti-PD-1 antibody is nivolumab, pembrolizumab, or cemiplimab.

In some aspects, the anti-PD-1 antagonist is an anti-PD-L1 antibody or antigen-binding portion thereof. In some aspects, the anti-PD-L1 antibody comprises avelumab, atezolizumab, durvalumab, or an antigen-binding portion thereof. In some aspects, the anti-PD-L1 antibody is avelumab, atezolizumab, or durvalumab.

In some aspects, the anti-PD-1 or anti-PD-L1 antibody is administered at a dose ranging from at least about 0.1 mg/kg to at least about 10.0 mg/kg body weight once about every 1, once about every 2 weeks, or once about every 3 weeks. In some aspects, the anti-PD-1 or anti-PD-L1 antibody is administered at a dose of at least about 3 mg/kg body weight once about every 2 weeks. In some aspects, the anti-PD-1 or anti-PD-L1 antibody or an antigen-binding portion thereof is administered at a flat dose. In some aspects, the anti-PD-1 or anti-PD-L1 antibody or antigen-binding portion thereof is administered at a flat dose of at least about 200 mg/dose, at least about 220 mg/dose, at least about 240 mg/dose, at least about 260 mg/dose, at least about 280 mg/dose, at least about 300 mg/dose, at least about 320 mg/dose, at least about 340 mg/dose, at least about 360 mg/dose, at least about 380 mg/dose, at least about 400 mg/dose, at least about 420 mg/dose, at least about 440 mg/dose, at least about 460 mg/dose, at least about 480 mg/dose, at least about 500 mg/dose, at least about 520 mg/dose, at least about 540 mg/dose, or at least about 550 mg/dose.

In some aspects, the anti-PD-1 or anti-PD-L1 antibody or antigen-binding portion thereof is administered at a flat dose of about 240 mg/dose. In some aspects, the anti-PD-1 or anti-PD-L1 antibody or antigen-binding portion thereof is administered at a flat dose of about 480 mg/dose. In some aspects, the anti-PD-1 or anti-PD-L1 antibody or antigen-binding portion thereof is administered at a flat dose about once every 1 week, once every 2 weeks, once every 3 weeks, or once every 4 weeks. In some aspects, the anti-PD-1 or anti-PD-L1 antibody or antigen-binding portion thereof is administered at a flat dose or about 240 mg/dose once about every two weeks. In some aspects, the anti-PD-1 or anti-PD-L1 antibody or antigen-binding portion thereof is administered at a flat dose of about 480 mg/dose once about every four weeks. In some aspects, the anti-PD-1 or anti-PD-L1 antibody is administered for as long as clinical benefit is observed or until unmanageable toxicity or disease progression occurs. In some aspects, the anti-PD-1 or anti-PD-L1 antibody is formulated for intravenous administration. In some aspects, the anti-PD-1 or anti-PD-L1 antibody is administered at a subtherapeutic dose.

In some aspects, the IDO1 inhibitor selectively inhibits IDO1. In some aspects, the IDO1 inhibitor does not inhibit TDO2 enzymatic activity. In the present invention, the IDO1 inhibitor is selected from linrodostat mesylate ((2R)-N-(4-chlorophenyl)-2-(cis-4-(6-fluoroquinolin-4-yl)cyclohexyl)propanamide), linrodostat, indoximod, p-(3-benzofuranyl)-DL-alanine, p-[3-benzo(b)thienyl]-DL-alanine; 6-nitro-L-tryptophan, and any combination thereof. In some aspects, the IDO1 inhibitor is linrodostat ((2R)-N-(4-chlorophenyl)-2-(cis-4-(6-fluoroquinolin-4-yl)cyclohexyl)propanamide). In some aspects, the IDO1 inhibitor is a linrodostat salt. In some aspects, the linrodostat salt is linrodostat mesylate. In some aspects, the IDO1 inhibitor is selected from the group consisting of linrodostat, 1-methyl-DL-tryptophan, p-(3-benzofuranyl)-DL-alanine, p-[3-benzo(b)thienyl]-DL-alanine; 6-nitro-L-tryptophan, and any combination thereof. In some aspects, the IDO1 inhibitor is formulated for oral administration. In some aspects, the IDO1 inhibitor is administered at a flat dose of about 25 mg/dose, 50 mg/dose, or 100 mg/dose. In some aspects, the IDO1 inhibitor is administered at a flat dose of about 200 mg/dose.

In some aspects, the IDO1 inhibitor is administered at a flat dose of at least about 50 mg/dose, at least about 60 mg/dose, at least about 70 mg/dose, at least about 80 mg/dose, at least about 90 mg/dose, at least about 100 mg/dose, at least about 120 mg/dose, at least about 140 mg/dose, at least about 160 mg/dose, at least about 180 mg/dose, at least about 200 mg/dose, at least about 220 mg/dose, at least about 240 mg/dose, at least about 260 mg/dose, at least about 280 mg/dose, at least about 300 mg/dose, at least about 330 mg/dose, at least about 340 mg/dose, at least about 360 mg/dose, at least about 380 mg/dose, or at least about 400 mg/dose.

In some aspects, the IDO1 inhibitor is administered at a flat dose of about 100 mg/dose every day. In some aspects, the IDO1 inhibitor is administered at a flat dose of about 200 mg/dose every day.

In some aspects, the anti-PD-1 antagonist is an anti-PD-1 antibody administered intravenously at a 240 mg every two weeks or 480 mg dose every four weeks, and the IDO1 inhibitor is administered orally at a 100 mg or 200 mg dose every day. In some aspects, the anti-PD-1 antagonist is an anti-PD-1 antibody administered intravenously at a 240 mg every two weeks, and the IDO1 inhibitor is administered orally at a 100 mg dose every day. In some aspects, the anti-PD-1 antagonist is an anti-PD-1 antibody administered intravenously at a 480 mg dose every four weeks, and the IDO1 inhibitor is administered orally at a 100 mg dose every day. In some aspects, the anti-PD-1 antagonist is an anti-PD-1 antibody administered intravenously at a 240 mg every two weeks, and the IDO1 inhibitor is administered orally at a 200 mg dose every day. In some aspects, the anti-PD-1 antagonist is an anti-PD-1 antibody administered intravenously at a 480 mg dose every four weeks, and the IDO1 inhibitor is administered orally at a 200 mg dose every day.

In some aspects, the anti-PD-1 antibody is nivolumab and the IDO1 inhibitor is linrodostat mesylate. In some aspects, the cancer is relapsed. In some aspects, the cancer is refractory. In some aspects, the PD-1 antagonist is administered before or after the IDO1 inhibitor. In some aspects, the PD-1 antagonist is administered concurrently with the IDO1 inhibitor. In some aspects, the cancer is refractory following at least one prior therapy comprising administration of at least one anticancer agent. In some aspects, the at least one anticancer agent comprises a standard of care therapy. In some aspects, the at least one anticancer agent comprises an immunotherapy. In some aspects, the cancer is locally advanced. In some aspects, the cancer is metastatic.

In some aspects of the methods and/or compositions disclosed herein, the administering of an anti-PD-1 antagonist and an indoleamine 2,3-dioxygenase 1 (IDO1) inhibitor to the subject treats the cancer. In some aspects, the administering reduces the cancer burden. In some aspects, cancer burden is reduced by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100% compared to the cancer burden prior to the administration.

In some aspects, the subject exhibits progression-free survival of at least about one month, at least about 2 months, at least about 3 months, at least about 4 months, at least about 5 months, at least about 6 months, at least about 7 months, at least about 8 months, at least about 9 months, at least about 10 months, at least about 11 months, at least about one year, at least about eighteen months, at least about two years, at least about three years, at least about four years, or at least about five years after the initial administration.

In some aspects, the subject exhibits stable disease after the administration. In some aspects, the subject exhibits a partial response after the administration. In some aspects, the subject exhibits a complete response after the administration. In some aspects, the administering improves progression-free survival probability by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 105%, at least about 110%, at least about 115%, at least about 120%, at least about 125%, at least about 130%, at least about 135%, at least about 140%, at least about 145%, or at least about 150%, compared to the progression-free survival probability of a subject not exhibiting a combined biomarker comprising (a) a high *IFNγ* inflammatory signature score and (b) a low *TDO2* gene expression score.

In some aspects, the administering improves overall survival probability by at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 125%, at least about 150%, at least about 175%, at least about 200%, at least about 225%, at least about 250%, at least about 275%, at least about 300%, at least about 325%, at least about 350%, or at least about 375%, compared to the overall survival probability of a subject not exhibiting a combined biomarker comprising (a) a high *IFNγ* inflammatory signature score and (b) a low *TDO2* gene expression score.

The present disclosure also provides a kit or object of manufacture for treating a subject afflicted with a cancer, the kit or object of manufacture comprising (a) a dosage of an anti-PD-1 antagonist; (b) a dosage of an IDO1 inhibitor; and (c) instructions for using the anti-PD-1 or anti-PD-L1 antibody and IDO1 inhibitor according to any of the methods disclosed herein.

The present disclosure also provides a gene panel comprising at least the *IFNγ* and *TDO2* genes, for use in (i) identifying a subject suitable for therapy with a combination comprising an anti-PD-1 antagonist and an IDO1 inhibitor; (ii) determining the prognosis of an subject undergoing therapy with a combination comprising an anti-PD-1 antagonist and an IDO1 inhibitor; (iii) initiating, suspending, or modifying the administration of a combination comprising an anti-PD-1 antagonist and an IDO1 inhibitor; or, (iv) a combination therefor.

In some aspects, the gene panel comprises *TDO2,* and at least one of *IFNy, CXCL10, HLA-DRA, CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STATI, HLA.E, CCR5, CXCL11, GZMA, PRF1,* IR2RG, CD3D, CD2, ITGAL, TAGAP, CIITA, PTPRC, CD3E, GZMK, GZMB, PDCD1, SLAMF6, CXCL13 or a combination thereof.

In some aspects, the gene panel consists or consists essentially of
*(i) IFNy, CXCL10, CXCL9, HLA-DRA, IDO1, STATI,* and *TDO2;*
(ii) *IFNy, CXCL10, CXCL9, HLA-DRA, IDO1, STATI, CCR5, CXCL11, GZMA, PRF1* and *TDO2;*
(iii) *CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STAT1,* and *HLA.E;* or
   or,
(iv) any combination thereof or any combination of the genes in (i) to (iii).

### BRIEF DESCRIPTION OF THE DRAWINGS

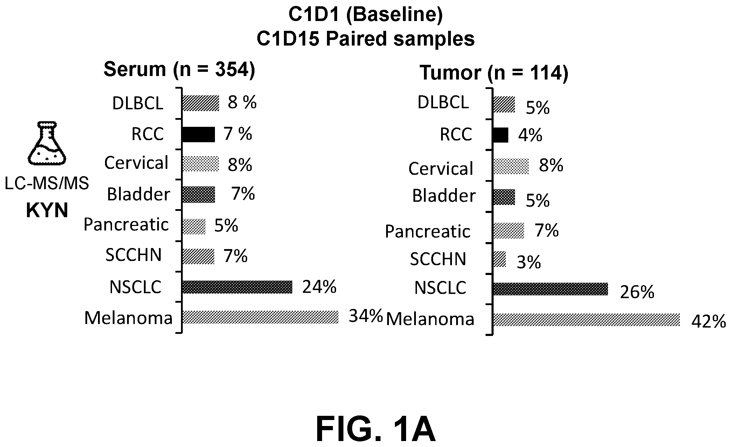

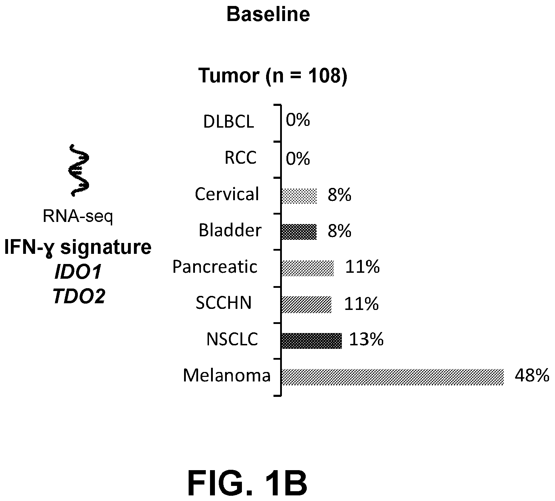

**FIG. 1A** and **FIG. 1B** show the percentage of samples of each tumor type that were obtained from patients, pooled together, and used in subsequent experiments/analyses. These figures depict the biomarker samples used and the assessment conducted with each set of samples. Gene expression results were analyzed in relation to response, progression free survival and overall survival. **FIG. 1A** shows the percentage of each type of tumor in which a tumor and serum sample were collected, frozen, and used in liquid chromatography - mass spectrometry experiments to detect kynurenine (KYN) The serum and tumor samples were collected at the beginning of treatment (cycle 1, day 1; C1D1) and during treatment (cycle 1, day 15; C1D15). **FIG. 1B** shows the percentage of each type of tumor that was formalin-fixed and paraffin-embedded prior to treatment (baseline). These samples were used in RNA-sequencing experiments to determine the IFNγ signature, the expression of *IDO1,* and the expression of TDO2. C1D1 = cycle 1 day 1; C1D15 = cycle 1 day 15; DLBCL = diffuse large B-cell lymphoma; IDO1 = indoleamine 2,3-dioxygenase-1; *IFNγ* = interferon gamma; KYN = kynurenine; LC-MS/MS = liquid chromatography-mass spectrometry; NSCLC = non-small cell lung cancer; OS = overall survival; PFS = progression-free survival; RCC = renal cell carcinoma; seq = sequencing; SCCHN = squamous cell carcinoma of the head and neck; TDO2 = tryptophan 2,3-dioxygenase 2.

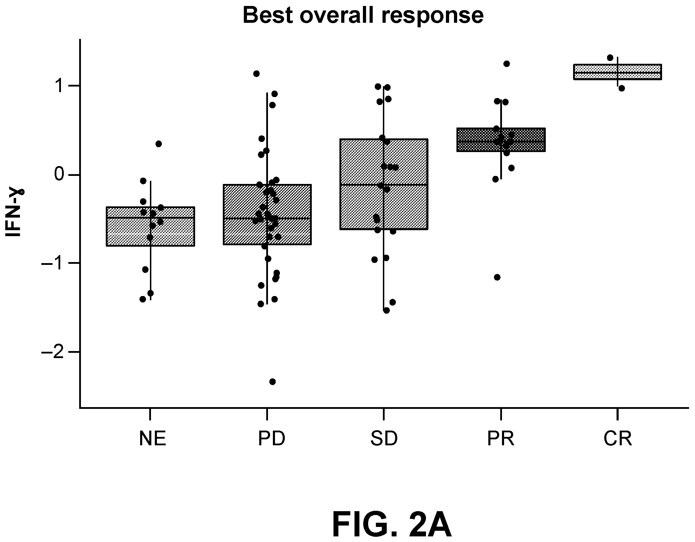

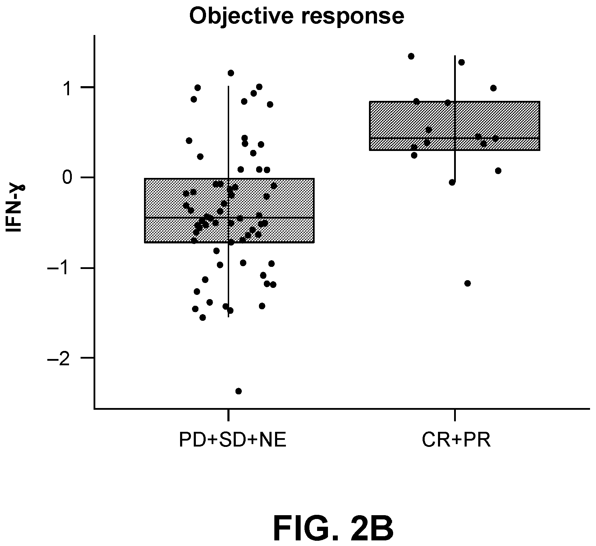

**FIG. 2A** and **FIG. 2B** show the association of IFNγ signature and the clinical response of immuno-oncology naive patients to treatment with nivolumab and the IDO1 inhibitor linrodostat. The best overall response **(****FIG. 2A****)** and objective response **(****FIG. 2B****)** of pooled tumor samples are shown. CR = complete response; *IFNγ* = interferon gamma; NE = not evaluable; PD = progressive disease; PR = partial response; SD = stable disease.

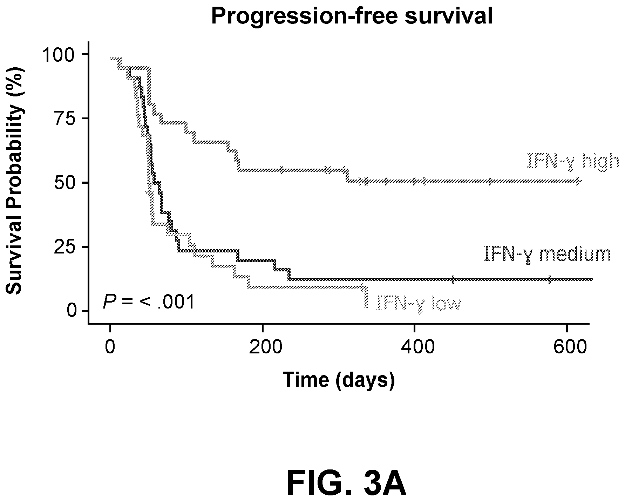

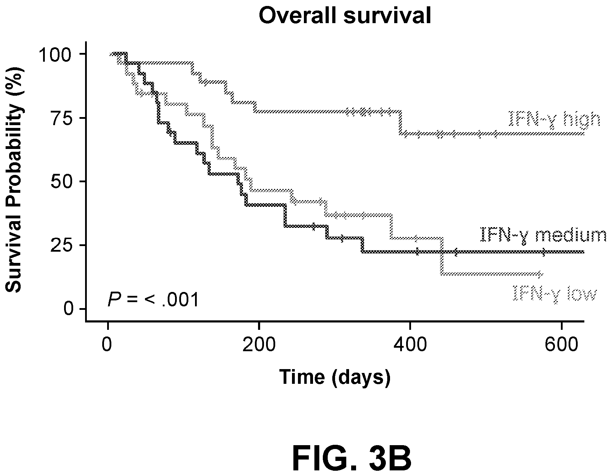

**FIG. 3A** and **FIG. 3B** are survival probability curves of immuno-oncology naive patients with low, medium, or high IFNγ signatures after treatment with nivolumab and linrodostat. A progression-free survival curve **(****FIG. 3A****)** and an overall survival curve **(****FIG. 3B****)** showing the probability of survival (%) corresponding to pooled tumor samples in which the IFNγ signature score was low, medium, or high. Pooled tumor types were evaluated and P values are descriptive based on IFNγ as a continuous variable. Survival curves are visually represented as tertiles. IFNγ = interferon gamma; OS = overall survival; PFS = progression-free survival.

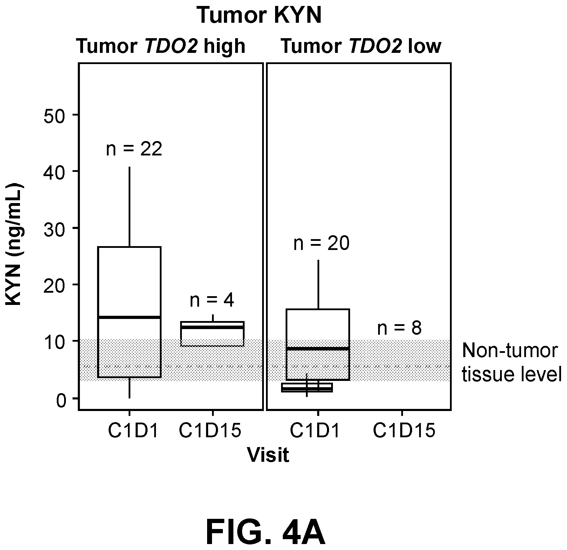

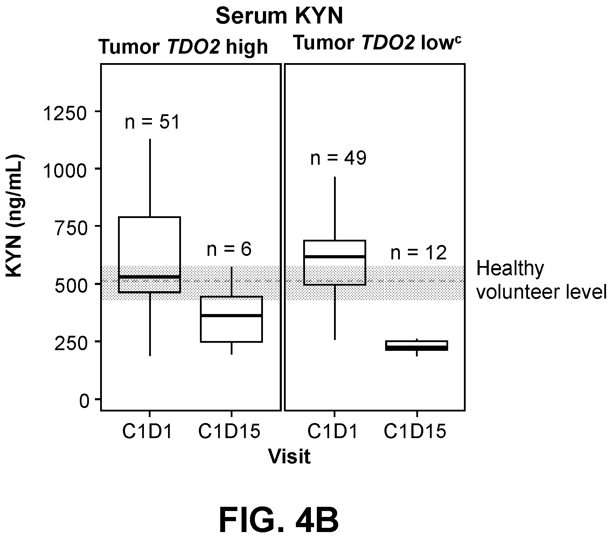

**FIG. 4A** and **FIG. 4B** are box plots showing the association of KYN levels and TDO2 tumor levels in immuno-oncology naive patients during treatment with nivolumab and linrodostat. **FIG. 4A** shows the tumor KYN levels from tumors and **FIG. 4B** shows the KYN levels from serum. The non-tumor tissue level of tumor KYN and the healthy volunteer level of serum KYN are shown by a gray bar. Pooled tumor types were evaluated. n represents the overlap of KYN and TDO2 expression. TDO2 high and low were defined by median TDO2 levels. C1D1 = cycle 1, day 1; C1D15 - cycle 1, day 15; KYN = kynurenine; TDO2 = tryptophan 2,3-dioxygenase 2.

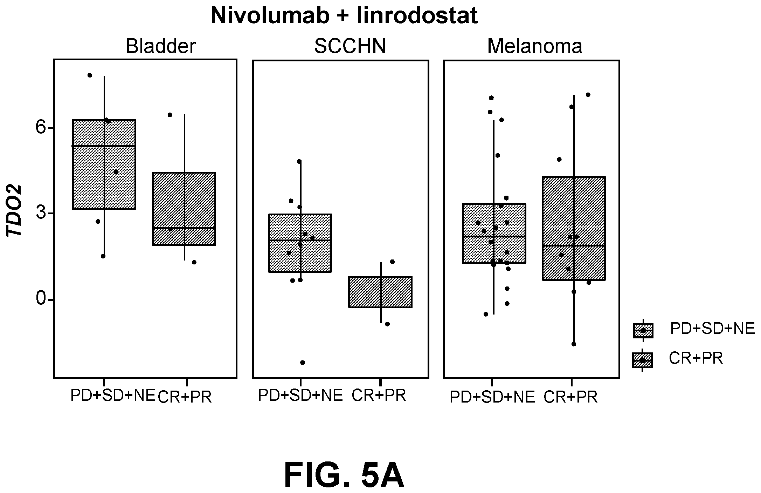

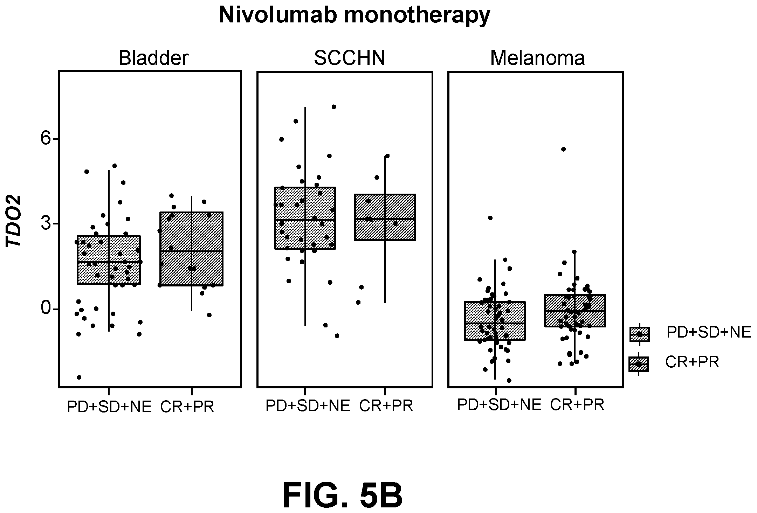

**FIG. 5A** and **FIG. 5B** are box plots showing the association of TDO2 expression with response to nivolumab monotherapy **(****FIG. 5B****)** or nivolumab and linrodostat combination therapy **(****FIG. 5A****)** in immuno-oncology naive patients. TDO2 expression was determined by median. All tumors are responders from the biomarker cohort (Clinical Trial No. NCT02658890). Nivolumab monotherapy data is from CheckMate trials. NE = not evaluable; PD = progressive disease; PR = partial response; SCCHN = squamous cell carcinoma of the head and neck; SD = stable disease; TDO2 = tryptophan 2,3-dioxygenase 2.

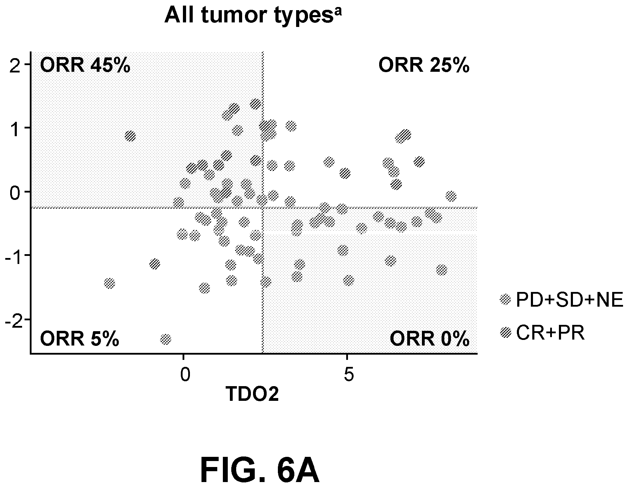

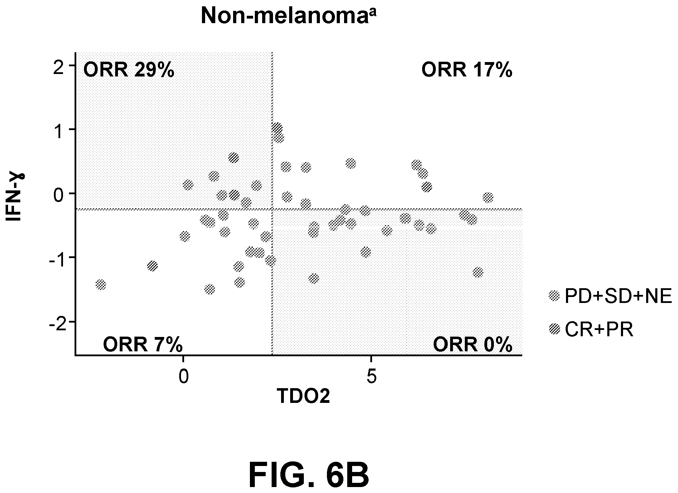

**FIG. 6A** and **FIG. 6B** show the relationship between IFNγ expression and TDO2 expression with the objective response rate in all tumor types **(****FIG. 6A****)** or non-melanoma tumors **(****FIG. 6B****)** from immuno-oncology naive patients treated with nivolumab and linrodostat. Lines denote medians of markers in each cohort. CR = complete response; *IFNγ* = interferon gamma; NE = not evaluable; ORR = objective response rate; PD = progressive disease; PR = partial response; SD = stable disease; TDO2 = tryptophan 2,3-dioxygenase 2.

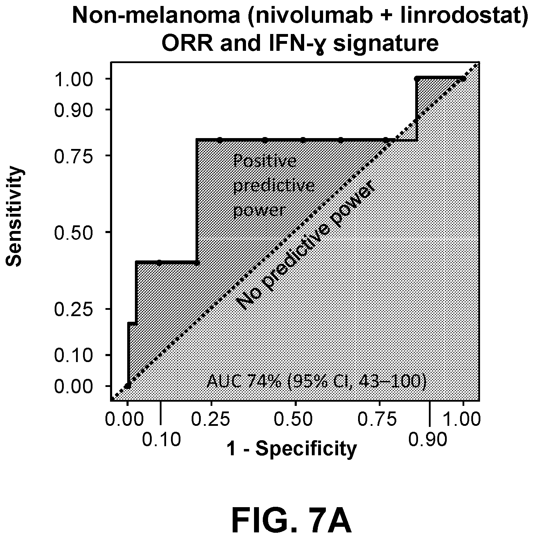

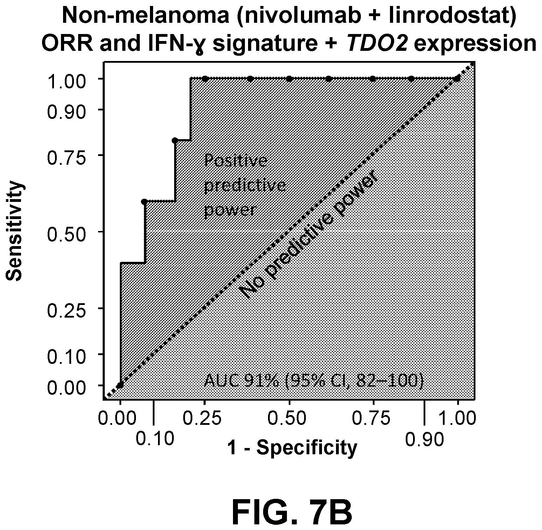

**FIG. 7A** and **FIG. 7B** are receiver operating characteristic curves of sensitivity and specificity for the objective response rate and IFNγ signature **(****FIG. 7A****)** or the objective response rate, *IFNγ* signature, and TDO2 expression **(****FIG. 7B****)** of non-melanoma tumors from immuno-oncology naive patients treated with nivolumab and linrodostat. Analysis was based on total sample size and not limited to the biomarker cohort. AUC = area under the curve; CI = confidence interval; *IFNγ* = interferon gamma; ORR = objective response rate; TDO2 = tryptophan 2,3-dioxygenase 2.

### DETAILED DESCRIPTION OF THE DISCLOSURE

The present disclosure provides methods for treating cancer, making treatment decisions (e.g., decide whether to initiate, suspend, or modify a treatment with a particular therapeutic composition or combination thereof), selecting or excluding patients from treatment, or determining the prognosis of a patient, based on the presence or absence of a composite or combined biomarker comprising (i) an *IFNγ* inflammatory signature score and (ii) a tryptophan 2,3-dioxygenase 2 (*TDO2*) gene expression score.

The composite biomarker disclosed herein has been determined to be predictive of a positive outcome (disease stabilization, partial response, or complete response) when combination treatments comprising an anti-PD-1 antagonist (e.g., an anti-PD-1 antibody such as nivolumab) and an IDO1 inhibitor (e.g., linrodostat mesylate) are administered to a subject afflicted with a cancer, e.g., a tumor, if the IFNyinflammatory signature score is high and the *TDO2* gene expression score is low prior to the administration.

Accordingly, the present disclosure provides methods for treating a human subject afflicted with a cancer, e.g., a tumor, comprising administering an anti-PD-1 antagonist (e.g., anti anti-PD-1 antibody, an anti-PD-L1 antibody, or an antigen binding portion thereof) and an IDO1 inhibitor (e.g., linrodostat mesylate) to the subject, wherein the subject is identified as exhibiting a combined biomarker comprising (a) a high *IFNγ* inflammatory signature score and (b) a low tryptophan 2,3-dioxygenase 2 (*TDO2*) gene expression score prior to the administration; and wherein the *IFNγ* inflammatory signature score is determined by measuring the expression of an a panel of inflammatory genes comprising interferon gamma (*IFNγ*) related genes ("*IFNγ* inflammatory gene panel") in a sample obtained from the subject, and subsequently calculating a score based on the expression values.

Also disclosed are methods for treating a human subject afflicted with a cancer comprising (i) identifying a subject exhibiting a combined biomarker comprising (a) a high *IFNγ* inflammatory signature score and (b) a low *TDO2* gene expression score; and, (ii) administering to the subject an anti-PD-1 antagonist (e.g., an anti-PD-1 antibody, an anti-PD-L1 antibody, or an antigen binding portion thereof) and an IDO1 inhibitor (e.g., linrodostat mesylate); wherein the *IFNγ* inflammatory signature score is determined by measuring the expression of *IFNγ* inflammatory gene panel in a sample obtained from the subject, and subsequently calculating a score based on the expression values.

Also disclosed are method for identifying a human subject afflicted with a cancer suitable for treatment with an anti-PD-1 antagonist (e.g., an anti-PD-1 antibody, an anti-PD-L1 antibody, or an antigen binding portion thereof) and an IDO1 inhibitor (e.g., linrodostat mesylate), the method comprising measuring an *IFNγ* inflammatory signature score and TDO2 gene expression in a sample obtained from the subject and; wherein the *IFNγ* inflammatory signature score is determined by measuring the expression of an *IFNγ* inflammatory gene panel in a sample obtained from the subject, and subsequently calculating a score based on the expression values. In some specific aspects, the anti-PD-1 antibody is nivolumab.

The present invention is as defined in the appended claims. It is directed to an anti-PD-1 antagonist for use in combination with an indoleamine 2,3-dioxygenase 1 (IDO1) inhibitor in a method for treating a human subject afflicted with a cancer, wherein the subject is identified as exhibiting a combined biomarker comprising (a) a high interferon gamma (IFNγ) inflammatory signature score and (b) a low tryptophan 2,3-dioxygenase 2 (TDO2) gene expression score prior to administration of the anti-PD-1 antagonist and IDO1 inhibitor; and wherein the *IFNγ* inflammatory signature score is determined by measuring the expression of a panel of inflammatory genes comprising IFNy-related genes ("*IFN*γ inflammatory gene panel") in a tumor tissue biopsy sample obtained from the subject, wherein the cancer is a bladder cancer or a squamous cell carcinoma of the head and neck (SCCHN); wherein the inflammatory genes comprise *IFNγ, CXCL10, HLA-DRA, IDO1, STATI, CCR5, CXCL11, GZMA,* and *PRF1,* and wherein the IDO1 inhibitor is selected from linrodostat mesylate ((2R)-N-(4-chlorophenyl)-2-(cis-4-(6-fluoroquinolin-4-yl)cyclohexyl)propanamide), linrodostat, indoximod, p-(3-benzofuranyl)-DL-alanine, p-[3-benzo(b)thienyl]-DL-alanine; 6-nitro-L-tryptophan, and any combination thereof. The technical disclosure set out below may in some respects go beyond the scope of the claims. Elements of the disclosure which do not fall within the scope of the claims are provided for information.

### I. Terms

In order that the present disclosure can be more readily understood, certain terms are first defined. As used in this application, except as otherwise expressly provided herein, each of the following terms shall have the meaning set forth below. Additional definitions are set forth throughout the application.

"Administering" refers to the physical introduction of a composition comprising a therapeutic agent to a subject, using any of the various methods and delivery systems known to those skilled in the art. Preferred routes of administration for immunotherapy, e.g., the administration of anti-PD-1 antibody (e.g., nivolumab) or an anti-PD-L1 antibody, include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as *in vivo* electroporation. Other non-parenteral routes include an oral, topical, epidermal or mucosal route of administration, for example, intranasally, vaginally, rectally, sublingually or topically. Administering can also be performed, for example, once, a plurality of times, and/or over one or more extended periods. In some aspects, the anti-PD-1 antibody (e.g., nivolumab) is administered intravenously. In some aspects, the IDO1 inhibitor (e.g., linrodostat) is administered orally.

An "adverse event" (AE) as used herein is any unfavorable and generally unintended or undesirable sign (including an abnormal laboratory finding), symptom, or disease associated with the use of a medical treatment. For example, an adverse event can be associated with activation of the immune system or expansion of immune system cells (*e.g.*, T cells) in response to a treatment. A medical treatment can have one or more associated AEs and each AE can have the same or different level of severity. Reference to methods capable of "altering adverse events" means a treatment regime that decreases the incidence and/or severity of one or more AEs associated with the use of a different treatment regime.

An "antibody" (Ab) shall include, without limitation, a glycoprotein immunoglobulin which binds specifically to an antigen and comprises at least two heavy (H) chains and two light (L) chains interconnected by disulfide bonds, or an antigen-binding portion thereof. Each H chain comprises a heavy chain variable region (abbreviated herein as V*_{H}*) and a heavy chain constant region. The heavy chain constant region comprises three constant domains, C*_{H1}, C*_{*H*2} and *C*_{*H*3}*.* Each light chain comprises a light chain variable region (abbreviated herein as V*_{L}*) and a light chain constant region. The light chain constant region comprises one constant domain, C*_{L}*. The V*_{H}* and V*_{L}* regions can be further subdivided into regions of hypervariability, termed complementarity determining regions (CDRs), interspersed with regions that are more conserved, termed framework regions (FRs). Each V*_{H}* and V*_{L}* comprises three CDRs and four FRs, arranged from amino-terminus to carboxy-terminus in the following order: FR1, CDR1, FR2, CDR2, FR3, CDR3, and FR4. The variable regions of the heavy and light chains contain a binding domain that interacts with an antigen. The constant regions of the antibodies can mediate the binding of the immunoglobulin to host tissues or factors, including various cells of the immune system (e.g., effector cells) and the first component (C1q) of the classical complement system. Therefore, the term "anti-PD-1 antibody" includes a full antibody having two heavy chains and two light chains that specifically binds to PD-1 and antigen-binding portions of the full antibody. Non limiting examples of the antigen-binding portions are shown elsewhere herein. In some aspects of the present disclosure, the anti-PD-1 antibody is nivolumab or an antigen-binding portion thereof.

An immunoglobulin can derive from any of the commonly known isotypes, including but not limited to IgA, secretory IgA, IgG and IgM. IgG subclasses are also well known to those in the art and include but are not limited to human IgG1, IgG2, IgG3 and IgG4. "Isotype" refers to the antibody class or subclass (e.g., IgM or IgG1) that is encoded by the heavy chain constant region genes. The term "antibody" includes, by way of example, both naturally occurring and non-naturally occurring antibodies; monoclonal and polyclonal antibodies; chimeric and humanized antibodies; human or nonhuman antibodies; wholly synthetic antibodies; and single chain antibodies. A nonhuman antibody can be humanized by recombinant methods to reduce its immunogenicity in man. Where not expressly stated, and unless the context indicates otherwise, the term "antibody" also includes an antigen-binding fragment or an antigen-binding portion of any of the aforementioned immunoglobulins, and includes a monovalent and a divalent fragment or portion, and a single chain antibody.

An "isolated antibody" refers to an antibody that is substantially free of other antibodies having different antigenic specificities (e.g., an isolated antibody that binds specifically to PD-1 is substantially free of antibodies that bind specifically to antigens other than PD-1). An isolated antibody that binds specifically to PD-1 can, however, have cross-reactivity to other antigens, such as PD-1 molecules from different species. Moreover, an isolated antibody can be substantially free of other cellular material and/or chemicals.

The term "monoclonal antibody" (mAb) refers to a non-naturally occurring preparation of antibody molecules of single molecular composition, *i.e.,* antibody molecules whose primary sequences are essentially identical, and which exhibits a single binding specificity and affinity for a particular epitope. A monoclonal antibody is an example of an isolated antibody. Monoclonal antibodies can be produced by hybridoma, recombinant, transgenic or other techniques known to those skilled in the art.

A "human antibody" (HuMAb) refers to an antibody having variable regions in which both the framework and CDR regions are derived from human germline immunoglobulin sequences. Furthermore, if the antibody contains a constant region, the constant region also is derived from human germline immunoglobulin sequences. The human antibodies of the disclosure can include amino acid residues not encoded by human germline immunoglobulin sequences (e.g., mutations introduced by random or site-specific mutagenesis *in vitro* or by somatic mutation *in vivo*)*.* However, the term "human antibody," as used herein, is not intended to include antibodies in which CDR sequences derived from the germline of another mammalian species, such as a mouse, have been grafted onto human framework sequences. The terms "human antibody" and "fully human antibody" and are used synonymously.

A "humanized antibody" refers to an antibody in which some, most or all of the amino acids outside the CDRs of a non-human antibody are replaced with corresponding amino acids derived from human immunoglobulins. In one aspect of a humanized form of an antibody, some, most or all of the amino acids outside the CDRs have been replaced with amino acids from human immunoglobulins, whereas some, most or all amino acids within one or more CDRs are unchanged. Small additions, deletions, insertions, substitutions or modifications of amino acids are permissible as long as they do not abrogate the ability of the antibody to bind to a particular antigen. A "humanized antibody" retains an antigenic specificity similar to that of the original antibody.

A "chimeric antibody" refers to an antibody in which the variable regions are derived from one species and the constant regions are derived from another species, such as an antibody in which the variable regions are derived from a mouse antibody and the constant regions are derived from a human antibody.

An "anti-antigen antibody" refers to an antibody that binds specifically to the antigen. For example, an anti-PD-1 antibody binds specifically to PD-1, and an anti-PD-L1 antibody binds specifically to PD-L1.

An "antigen-binding portion" of an antibody (also called an "antigen-binding fragment") refers to one or more fragments of an antibody that retain the ability to bind specifically to the antigen bound by the whole antibody. It has been shown that the antigen-binding function of an antibody can be performed by fragments of a full-length antibody. Examples of binding fragments encompassed within the term "antigen-binding portion" of an antibody, e.g., an anti- PD-1 antibody or an anti-PD-L1 antibody described herein, include (i) a Fab fragment (fragment from papain cleavage) or a similar monovalent fragment consisting of the V_{L}, V_{H}, LC and CH1 domains; (ii) a F(ab')2 fragment (fragment from pepsin cleavage) or a similar bivalent fragment comprising two Fab fragments linked by a disulfide bridge at the hinge region; (iii) a Fd fragment consisting of the V_{H} and CH1 domains; (iv) a Fv fragment consisting of the V_{L} and V_{H} domains of a single arm of an antibody, (v) a dAb fragment (Ward et al., (1989) Nature 341:544-546), which consists of a V_{H} domain; (vi) an isolated complementarity determining region (CDR) and (vii) a combination of two or more isolated CDRs which can optionally be joined by a synthetic linker. Furthermore, although the two domains of the Fv fragment, V_{L} and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); *see, e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies. Antigen-binding portions can be produced by recombinant DNA techniques, or by enzymatic or chemical cleavage of intact immunoglobulins.

A "cancer" refers a broad group of various diseases characterized by the uncontrolled growth of abnormal cells in the body. Unregulated cell division and growth divide and grow results in the formation of malignant tumors that invade neighboring tissues and can also metastasize to distant parts of the body through the lymphatic system or bloodstream. The term "tumor" refers to a solid cancer. The term "carcinoma" refers to a cancer of epithelial origin.

The term "IDO1" as used herein refers to indoleamine 2,3-dioxygenase 1. Indoleamine-pyrrole 2,3-dioxygenase (IDO or INDO EC 1.13.11.52) is a heme-containing enzyme that in humans is encoded by the IDO1 gene. It is one of three enzymes that catalyze the first and rate-limiting step in the kynurenine pathway, the O₂-dependent oxidation of L-tryptophan to N-formylkynurenine, the others being IDO2 and tryptophan 2,3-dioxygenase (TDO). IDO has been implicated in immune modulation through its ability to limit T-cell function and engage mechanisms of immune tolerance. IDO becomes activated during tumor development, helping malignant cells escape eradication by the immune system. Interferon gamma has an antiproliferative effect on many tumors cells at least partly because of the induction of the indoleamine 2,3-dioxygenase.

The term "IDO1 inhibitor" as used herein refers to an indoleamine 2,3-dioxygenase 1 inhibitor. IDO1 inhibitors inhibit indoleamine 2,3-dioxygenase, leading to a reduction in kynurenine levels as well as reducing proinflammatory cytokine activity. 1-Methyltryptophan is a racemic compound that weakly inhibits indoleamine dioxygenase, but is also a very slow substrate. The specific racemer 1-methyl-D-tryptophan (known as indoximod) is in clinical trials for various cancers. Epacadostat (INCB24360) and navoximod (GDC-0919) are potent inhibitors of the indoleamine 2,3-dioxygenase enzyme and are in clinical trials for various cancers. In some aspects, the IDO1 inhibitor is linrodostat (BMS-986205). In some aspects, the IDO1 inhibitor is a linrodostat salt, e.g., linrodostat mesylate. See WO2015031295, WO2016073770, and WO201809049.

The term "immunotherapy" refers to the treatment of a subject afflicted with, or at risk of contracting or suffering a recurrence of, a disease by a method comprising inducing, enhancing, suppressing or otherwise modifying an immune response. "Treatment" or "therapy" of a subject refers to any type of intervention or process performed on, or the administration of an active agent to, the subject with the objective of reversing, alleviating, ameliorating, inhibiting, slowing down or preventing the onset, progression, development, severity or recurrence of a symptom, complication or condition, or biochemical indicia associated with a disease.

"Programmed Death-1" (PD-1) refers to an immunoinhibitory receptor belonging to the CD28 family. PD-1 is expressed predominantly on previously activated T cells *in vivo,* and binds to two ligands, PD-L1 and PD-L2. The term "PD-1" as used herein includes human PD-1 (hPD-1), variants, isoforms, and species homologs of hPD-1, and analogs having at least one common epitope with hPD-1. The complete hPD-1 sequence can be found under GenBank Accession No. U64863.

"Programmed Death Ligand-1" (PD-L1) is one of two cell surface glycoprotein ligands for PD-1 (the other being PD-L2) that downregulate T cell activation and cytokine secretion upon binding to PD-1. The term "PD-L1" as used herein includes human PD-L1 (hPD-L1), variants, isoforms, and species homologs of hPD-L1, and analogs having at least one common epitope with hPD-L1. The complete hPD-L1 sequence can be found under GenBank Accession No. Q9NZQ7. The human PD-L1 protein is encoded by the human CD274 gene (NCBI Gene ID: 29126).

As used herein the term "subject" includes any human or nonhuman animal. The term "nonhuman animal" includes, but is not limited to, vertebrates such as nonhuman primates, sheep, dogs, and rodents such as mice, rats and guinea pigs. In specific aspects, the subject is a human. The terms, "subject" and "patient" are used interchangeably herein.

The use of the term "flat dose" with regard to the methods and dosages of the disclosure means a dose that is administered to a patient without regard for the weight or body surface area (BSA) of the patient. The flat dose is therefore not provided as a mg/kg dose, but rather as an absolute amount of the agent (e.g., the anti-PD-1 antibody). For example, a 60 kg person and a 100 kg person would receive the same dose of an antibody (*e.g.,* 240 mg of an anti-PD-1 antibody).

The use of the term "fixed dose" with regard to a method of the disclosure means that a therapeutic agent, e.g., an anti-PD-1 antagonist (e.g., nivolumab) or IDO1 inhibitor (e.g., linrodostat) is present in a composition, a pharmaceutical composition, in a particular (fixed) amount. In some aspects, the fixed dose is based on the weight (e.g., mg) of the therapeutic agent. In certain aspects, the fixed dose is based on the concentration (e.g., mg/ml) of the therapeutic agent. The term "weight-based dose" as referred to herein means that a dose that is administered to a patient is calculated based on the weight of the patient. For example, when a patient with 60 kg body weight requires 3 mg/kg of an anti-PD-1 antibody, one can calculate and use the appropriate amount of the anti-PD-1 antibody (*i.e.,* 180 mg) for administration.

An "immune response" is as understood in the art, and generally refers to a biological response within a vertebrate against foreign agents or abnormal, e.g., cancerous cells, which response protects the organism against these agents and diseases caused by them. An immune response is mediated by the action of one or more cells of the immune system (for example, a T lymphocyte, B lymphocyte, natural killer (NK) cell, macrophage, eosinophil, mast cell, dendritic cell or neutrophil) and soluble macromolecules produced by any of these cells or the liver (including antibodies, cytokines, and complement) that results in selective targeting, binding to, damage to, destruction of, and/or elimination from the vertebrate's body of invading pathogens, cells or tissues infected with pathogens, cancerous or other abnormal cells, or, in cases of autoimmunity or pathological inflammation, normal human cells or tissues. An immune reaction includes, *e.g.,* activation or inhibition of a T cell, *e.g.,* an effector T cell, a Th cell, a CD4⁺ cell, a CD8⁺ T cell, or a Treg cell, or activation or inhibition of any other cell of the immune system, e.g., NK cell.

An "immune-related response pattern" refers to a clinical response pattern often observed in cancer patients treated with immunotherapeutic agents that produce anticancer effects by inducing cancer-specific immune responses or by modifying native immune processes. This response pattern is characterized by a beneficial therapeutic effect that follows an initial increase in cancer burden or the appearance of new lesions, which in the evaluation of traditional chemotherapeutic agents would be classified as disease progression and would be synonymous with drug failure. Accordingly, proper evaluation of immunotherapeutic agents can require long-term monitoring of the effects of these agents on the target disease.

The terms "treat," "treating," and "treatment," as used herein, refer to any type of intervention or process performed on, or administering an active agent to, the subject with the objective of reversing, alleviating, ameliorating, inhibiting, or slowing down or preventing the progression, development, severity or recurrence of a symptom, complication, condition or biochemical indicia associated with a disease or enhancing overall survival. Treatment can be of a subject having a disease or a subject who does not have a disease (e.g., for prophylaxis).

The term "effective dose" or "effective dosage" is defined as an amount sufficient to achieve or at least partially achieve a desired effect.

A "therapeutically effective amount" or "therapeutically effective dosage" of a drug or therapeutic agent is any amount of the drug that, when used alone or in combination with another therapeutic agent, protects a subject against the onset of a disease or promotes disease regression evidenced by a decrease in severity of disease symptoms, an increase in frequency and duration of disease symptom-free periods, or a prevention of impairment or disability due to the disease affliction. A therapeutically effective amount or dosage of a drug includes a "prophylactically effective amount" or a "prophylactically effective dosage", which is any amount of the drug that, when administered alone or in combination with another therapeutic agent to a subject at risk of developing a disease or of suffering a recurrence of disease, inhibits the development or recurrence of the disease.

The ability of a therapeutic agent to promote disease regression, e.g., cancer regression can be evaluated using a variety of methods known to the skilled practitioner, such as in human subjects during clinical trials, in animal model systems predictive of efficacy in humans, or by assaying the activity of the agent in *in vitro* assays.

By way of example, an "anti-cancer agent" or combination thereof promotes cancer regression in a subject. In some aspects, a therapeutically effective amount of the therapeutic agent promotes cancer regression to the point of eliminating the cancer. In some aspects of the present disclosure the anticancer agents are administered as a combination of therapies: an intravenous therapy comprising the administration of an anti-PD-1 antibody (e.g., nivolumab), and an oral therapy comprising the administration of an IDO1 inhibitor (e.g., linrodostat mesylate). "Promoting cancer regression" means that administering an effective amount of the drug or combination thereof (administered together as a single therapeutic composition or as separate compositions in separate treatments as discussed above) , results in a reduction in cancer burden, e.g., reduction in tumor growth or size, necrosis of the tumor, a decrease in severity of at least one disease symptom, an increase in frequency and duration of disease symptom-free periods, or a prevention of impairment or disability due to the disease affliction.

In addition, the terms "effective" and "effectiveness" with regard to a treatment disclosed herein includes both pharmacological effectiveness and physiological safety. Pharmacological effectiveness refers to the ability of the drug to promote cancer regression in the patient. Physiological safety refers to the level of toxicity, or other adverse physiological effects at the cellular, organ and/or organism level (adverse effects) resulting from administration of the drug.

By way of example for the treatment of tumors, a therapeutically effective amount of an anti-cancer agent preferably inhibits cancer cell growth or tumor growth by at least about 20%, more preferably by at least about 40%, even more preferably by at least about 60%, and still more preferably by at least about 80% relative to untreated subjects. Similarly, in hematological cancers, a therapeutically effective amount of an anti-cancer agent preferably inhibits cells growth or decreases the number of circulating cancer cells by at least about 20%, more preferably by at least about 40%, even more preferably by at least about 60%, and still more preferably by at least about 80% relative to untreated subjects.

In some aspects, a therapeutically effective amount or dosage of the drug inhibits cancer cell growth or tumor growth by at least between about 20% and about 40%, by at least between about 30% and about 50%, by at least between about 40% and about 60%, by at least between about 50% and about 70%, by at least between about 60% and about 80%, by at least between about 70% and about 90%, by at least between about 30% and about 60%, by at least between about 40% and about 70%, by at least between about 50% and about 80%, by at least between about 60% and about 90%, relative to untreated subjects. In some aspects, a therapeutically effective amount or dosage of the drug completely inhibits cell growth or tumor growth, *i.e.,* inhibits cell growth or tumor growth by 100%.

In some aspects of the disclosure, cancer regression (e.g., tumor regression) can be observed and continue for a period of at least about 1 month, at least about 2 month, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 19 months, at least about 20 months, at least about 21 months, at lest about 22 months, at least about 23 months, at least about 24 months, at least about 3 years, at least about 4 years, or at least about 5 years. Notwithstanding these ultimate measurements of therapeutic effectiveness, evaluation of immunotherapeutic drugs must also make allowance for immune-related response patterns. The ability of a therapeutic agent to inhibit cancer growth, e.g., tumor growth, can be evaluated using assays described herein and other assays known in the art. Alternatively, this property of a composition can be evaluated by examining the ability of the compound to inhibit cell growth, such inhibition can be measured *in vitro* by assays known to the skilled practitioner.

In other aspects, e.g., treatment of hematological cancer, a therapeutically effective amount or dosage of the drug inhibits cancer cell growth or reduces cancer burden by at least about 20%, by at least about 25%, by at least about 30%, by at least about 35%, by at least about 40%, by at least about 45%, by at least about 50%, by at least about 55%, by at least about 60%, by at least about 65%, by at least about 70%, by at least about 75%, by at least about 80%, by at least about 85%, by at least about 90%, or by at least about 95%, relative to untreated subjects. In some aspects, a therapeutically effective amount or dosage of the drug completely inhibits cell growth or eliminates cancer burden, *i.e.,* inhibits cell growth or total population of detectable cancer cells by 100%. The ability of a compound to reduce cancer burden can be evaluated using assay described herein and other assays known in the art.

In some aspects, cancer, e.g., tumor, stabilization or regression (partial response or complete response) can be observed and continue for a period of at least about 20 days, at least about 30 days, at least about 40 days, at least about 50 days, at least about 60 days, at least about 70 days, at least about 80 days, at least about 90 days, at least about 100 days, at least about 120 days, at least about 140 days, at least about 160 days, at least about 180 days, at least about 200 days, at least about 225 days, at least about 250 days, at least about 275 days, at least about 300 days, at least about 350 days, at least about 400 days, at least about 450 days, at least about 500 days, at least about 550, or at least about 600 days.

The term "biological sample" as used herein refers to biological material isolated from a subject. The biological sample can contain any biological material suitable for determining target gene expression, for example, by sequencing nucleic acids in the cancer, e.g., tumor cells or circulating cancer cells, and identifying a genomic alteration in the sequenced nucleic acids. In some aspects, the genomic alternation is a mutation. In other aspects, the genomic alteration is a change in the expression level of a certain gene in the cancer cells with respect to a reference.

The biological sample can be any suitable biological tissue or fluid such as, for example, cancer tissue, blood, blood plasma, and serum. In one aspect, and in the present invention, the sample is a tumor tissue biopsy, e.g., a formalin-fixed, paraffin-embedded (FFPE) tumor tissue or a fresh-frozen tumor tissue or the like. In another aspect, the biological sample is a liquid biopsy that, in some aspects, comprises one or more of blood, serum, plasma, circulating tumor cells, exoRNA, ctDNA, and cfDNA.

The terms "once about every week," "once about every two weeks," or any other similar dosing interval terms as used herein mean approximate numbers. "Once about every week" can include every seven days ± one day, *i.e.,* every six days to every eight days. "Once about every two weeks" can include every fourteen days ± three days, *i.e.,* every eleven days to every seventeen days. Similar approximations apply, for example, to once about every three weeks, once about every four weeks, once about every five weeks, once about every six weeks, and once about every twelve weeks.

In some aspects, a dosing interval of once about every six weeks or once about every twelve weeks means that the first dose can be administered any day in the first week, and then the next dose can be administered any day in the sixth or twelfth week, respectively. In other aspects, a dosing interval of once about every six weeks or once about every twelve weeks means that the first dose is administered on a particular day of the first week (e.g., Monday) and then the next dose is administered on the same day of the sixth or twelfth weeks (*i.e.,* Monday), respectively.

The singular forms "a", "an" and "the" include plural referents unless the context clearly dictates otherwise. The terms "a" (or "an"), as well as the terms "one or more," and "at least one" can be used interchangeably herein. In certain aspects, the term "a" or "an" means "single." In other aspects, the term "a" or "an" includes "two or more" or "multiple."

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

The terms "about" or "comprising essentially of' refer to a value or composition that is within an acceptable error range for the particular value or composition as determined by one of ordinary skill in the art, which will depend in part on how the value or composition is measured or determined, *i.e.,* the limitations of the measurement system. For example, "about" or "comprising essentially of' can mean within 1 or more than 1 standard deviation per the practice in the art. Alternatively, "about" or "comprising essentially of" can mean a range of up to 10%. Furthermore, particularly with respect to biological systems or processes, the terms can mean up to an order of magnitude or up to 5-fold of a value. When particular values or compositions are provided in the application and claims, unless otherwise stated, the meaning of "about" or "comprising essentially of" should be assumed to be within an acceptable error range for that particular value or composition.

As used herein, the term "approximately," as applied to one or more values of interest, refers to a value that is similar to a stated reference value. In certain aspects, the term "approximately" refers to a range of values that fall within 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

As described herein, any concentration range, percentage range, ratio range or integer range is to be understood to include the value of any integer within the recited range and, when appropriate, fractions thereof (such as one tenth and one hundredth of an integer), unless otherwise indicated.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary of Biochemistry and Molecular Biology, Revised, 2000, Oxford University Press, provide one of skill with a general dictionary of many of the terms used in this disclosure.

It is understood that wherever aspects are described herein with the language "comprising," otherwise analogous aspects described in terms of "consisting of' and/or "consisting essentially of' are also provided.

Units, prefixes, and symbols are denoted in their Système International de Unites (SI) accepted form. The headings provided herein are not limitations of the various aspects of the disclosure, which can be had by reference to the specification as a whole. Accordingly, the terms defined are more fully defined by reference to the specification in its entirety.

Abbreviations used herein are defined throughout the present disclosure. Various aspects of the disclosure are described in further detail in the following subsections.

### II. Methods and Compositions for Use

The present disclosure is generally directed to methods for treating a human subject afflicted with a cancer, e.g., a tumor, comprising administering an anti-PD-1 antagonist (e.g., an anti-PD-1 antibody such as nivolumab, an anti-PD-L1 antibody, or an antigen-binding portion thereof) and an indoleamine 2,3-dioxygenase 1 (IDO1) inhibitor (e.g., linrodostat mesylate) to the subject, wherein the subject is identified as exhibiting a combined biomarker comprising (a) a high *IFNγ* inflammatory signature score and (b) a low tryptophan 2,3-dioxygenase 2 (*TDO2*) gene expression score prior to the administration; and wherein the *IFNγ* inflammatory signature score is determined by measuring the expression of an a panel of inflammatory genes comprising interferon gamma (*IFNγ*) related genes ("*IFN*γ inflammatory gene panel") in a sample obtained from the subject. *IFNγ* inflammatory signature score is calculated from the expression levels measured for every gene in the *IFNγ* inflammatory gene panel.

Accordingly, the present invention provides an anti-PD-1 antagonist for use in combination with an indoleamine 2,3-dioxygenase 1 (IDO1) inhibitor in a method for treating a human subject afflicted with a cancer, wherein the subject is identified as exhibiting a combined biomarker comprising (a) a high interferon gamma (IFNγ) inflammatory signature score and (b) a low tryptophan 2,3-dioxygenase 2 (TDO2) gene expression score prior to administration of the anti-PD-1 antagonist and IDO1 inhibitor; and wherein the *IFNy* inflammatory signature score is determined by measuring the expression of a panel of inflammatory genes comprising *IFNγ*-related genes (*"IFNγ* inflammatory gene panel") in a tumor tissue biopsy sample obtained from the subject, wherein the cancer is a bladder cancer or a squamous cell carcinoma of the head and neck (SCCHN); wherein the inflammatory genes comprise *IFNγ, CXCL10, HLA-DRA, IDO1, STATI, CCR5, CXCL11, GZMA,* and *PRF1,* and wherein the IDO1 inhibitor is selected from linrodostat mesylate ((2R)-N-(4-chlorophenyl)-2-(cis-4-(6-fluoroquinolin-4-yl)cyclohexyl)propanamide), linrodostat, indoximod, p-(3-benzofuranyl)-DL-alanine, p-[3-benzo(b)thienyl]-DL-alanine; 6-nitro-L-tryptophan, and any combination thereof

As used herein, the term *"IFNγ* related genes" refers to genes that are related to *IFNγ* mediated cell signaling. In an aspect of the present disclosure, *IFNγ* related genes encompass, e.g., *IFNγ, CXCL10, HLA-DRA, CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STATI, HLA.E, CCR5, CXCL11, GZMA, PRF1, IR2RG, CD3D, CD2, ITGAL, TAGAP, CIITA, PTPRC, CD3E, GZMK, GZMB, PDCD1, SLAMF6,* and *CXCL13.* Thus, an *"IFNγ* inflammatory gene panel" can comprise any subset of these genes. In one aspect, the *IFNγ* inflammatory gene panel comprises, consists, or consists essentially of *IFNy, CXCL10, HLA-DRA, CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STATI, HLA.E, CCR5, CXCL11, GZMA, PRF1, IR2RG, CD3D, CD2, ITGAL, TAGAP, CIITA, PTPRC, CD3E, GZMK, GZMB, PDCD1, SLAMF6,* and *CXCL13.* In another aspect, the *IFNγ* inflammatory gene panel comprises, consists, or consists essentially of *IR2RG, CXCR6, CD3D, CD2, ITGAL, TAGAP, CIITA, HLA-DRA, PTPRC, CXCL9, CCL5, NKG7, GZMA, PRF1, CCR5, CD3E, GZMK, IFNγ, HLA-E, GZMB, PDCD1, SLAMF6, CXCL13, CXCL10, IDO1, LAG3, STAT1,* and *CXCL11.* In another aspect, the *IFNγ* inflammatory gene panel comprises, consists, or consists essentially of *CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STAT1,* and *HLA.E.* In another aspect, the *IFNγ* inflammatory gene panel comprises, consists, or consists essentially of *IFNy, CXCL10, CXCL9, HLA-DRA, IDO1, STAT1, CCR5, CXCL11, GZMA,* and *PRF1.* In another aspect, the IFNγ inflammatory gene panel comprises, consists, or consists essentially of *IFNγ, CXCL10, CXCL9, HLA-DRA, IDO1,* and *STAT1.* See **TABLE 1,** which provides gene names, descriptions, and RefSeq sequence identifiers for the *IFNγ* related genes disclosed herein and TDO2. In the present invention, the inflammatory genes comprise *IFNy, CXCL10, CXCL9, HLA-DRA, IDO1, STATI, CCR5, CXCL11, GZMA,* and *PRF1.*

The Reference Sequence (RefSeq) database is an open access, annotated and curated collection of publicly available nucleotide sequences (DNA, RNA) and their protein products. This database is built by National Center for Biotechnology Information (NCBI), and, unlike GenBank, provides only a single record for each natural biological molecule (i.e. DNA, RNA or protein) for major organisms ranging from viruses to bacteria to eukaryotes. The RefSeq database can be accessed at www.ncbi.nlm.nih.gov/refseq.

**TABLE 1**

| **Gene name (synonym)** | **Description** | **RefSeq identifier** |
|---|---|---|
| CCL5 (RANTES) | Chemokine (C-C Motif) ligand 5 | NM_002985.2 |
| CCR5 | Chemokine (C-C motif) receptor 5 | NM_000579 |
| CD2 | T-Cell Surface Antigen CD2 | NM_001767.2 |
| CD274 (PD-L1) | Programmed Cell Death 1 Ligand 1 | NM_014143.2 |
| CD276 (B7-H3) | B7 Homolog 3 | NM_00102473 6.1 |
| CD3D | CD3d molecule, delta (CD3-TCR complex) | NM_000732.4 |
| CD3E | CD3e molecule, epsilon (CD3-TCR complex) | NM_000733.2 |
| CD8A | T-Cell Surface Glycoprotein CD8 Alpha Chain | NM_001145873 |
| CIITA | Class II, major histocompatibility complex, transactivator | NM_000246.3 |
| CMKLR1 | Chemokine-like receptor 1 | NM_001142343 |
| CXCL10 (IP-10) | Chemokine (C-X-C motif) ligand 10 | NM_001565.1 |
| CXCL11 | Chemokine (C-X-C motif) ligand 11 | NM_005409 |
| CXCL13 (BCA-1) | Chemokine (C-X-C motif) ligand 13 | NM_006419.2 |
| CXCL9 | Chemokine (C-X-C motif) ligand 9 | NM_002416 |
| CXCR6 | Chemokine (C-X-C motif) receptor 6 | NM_006564.1 |
| GZMA | Granzyme A | NM_006144.2 |
| GZMB | Granzyme B | NM_004131.3 |
| GZMK | Granzyme K | NM_002104.2 |
| HLA.DQA1 | Major histocompatibility complex, class II, DQ alpha 1 | NM_002122 |
| HLA.DRB1 | Major histocompatibility complex, class II, DR beta 1 | NM_002124 |
| HLA.E | Major histocompatibility complex, class I, E | NM_005516.4 |
| HLA-DRA | Major histocompatibility complex, class II, DR alpha | NM_019111.3 |
| IDO1 | Indoleamine 2,3-dioxygenase 1 | NM_002164 |
| IFNγ | Interferon gamma | NM_000619.2 |
| IR2RG | Interleukin 2 receptor, gamma | NM_000206 |
| LAG3 (CD223) | Lymphocyte activation gene 3 | NM_002286.5 |
| ITGAL | Integrin, alpha L (antigen CD11A (p180), lymphocyte function-associated antigen 1; alpha polypeptide) | NM_002209 |
| NKG7 | Natural killer cell granule protein 7 | NM_005601 |
| PDCD1 (PD-1) | Programmed Death 1 | NM_005018 |
| PDCD1LG2 (PD-L2) | Programmed Cell Death 1 Ligand 2 | NM_025239.3 |
| PRF1 | Perforin 1 | NM_005041.3 |
| PSMB10 | Proteasome (prosome, macropain) subunit, beta type, 10 | NM_002801.2 |
| PTPRC | Protein tyrosine phosphatase, receptor type, C | NM_080921 |
| SLAMF6 (NTBA) | SLAM family member 6 | NM_001184714 |
| STAT1 | Signal transducer and activator of transcription 1 | NM_007315.2 |
| TAGAP | T-cell activation RhoGTPase activating protein | NM_054114.3 |
| TIGIT | T cell immunoreceptor with Ig and ITIM domains | NM_173799.2 |
| TDO2 | Tryptophan 2,3-dioxygenase | NM_005651 |

The present disclosure also provides methods for treating a human subject afflicted with a cancer, e.g., a tumor, comprising (i) identifying a subject exhibiting a combined biomarker comprising (a) a high *IFNγ* inflammatory signature score and (b) a low *TDO2* gene expression score; and, (ii) administering to the subject an anti-PD-1 antagonist (e.g., an anti-PD-1 antibody such as nivolumab, an anti-PD-L1 antibody, or an antigen-binding portion thereof) and an IDO1 inhibitor (e.g., linrodostat mesylate); wherein the *IFNγ* inflammatory signature score is determined by measuring the expression of *IFNγ* inflammatory gene panel in a sample obtained from the subject, and subsequently calculating the score from the measured expression levels.

Also disclosure are methods for identifying a human subject afflicted with a cancer, e.g., a tumor, suitable for treatment with an anti-PD-1 antagonist (e.g., an anti-PD-1 antibody such as nivolumab, an anti-PD-L1 antibody, or an antigen-binding portion thereof) and an IDO1 inhibitor (e.g., linrodostat mesylate), the method comprising measuring an *IFNγ* inflammatory signature score and TDO2 gene expression in a sample obtained from the subject and; wherein the *IFNγ* inflammatory signature score is determined by measuring the expression of an *IFNγ* inflammatory gene panel in a sample obtained from the subject, and subsequently calculating the score from the measured expression levels.

In some aspects, the subject exhibits a combined biomarker comprising (a) a high *IFNγ* inflammatory signature score and (b) a low *TDO2* gene expression score. In some aspects, the method further comprises administering to the subject an anti-PD-1 antagonist (e.g., an anti-PD-1 antibody such as nivolumab, an anti-PD-L1 antibody, or an antigen-binding portion thereof) and an IDO1 inhibitor (e.g., linrodostat mesylate).

The present disclosure also provides an IDO1 inhibitor (e.g., linrodostat mesylate) for treating a cancer, e.g., a tumor, in combination with an anti-PD-1 antagonist (e.g., an anti-PD-1 antibody such as nivolumab, an anti-PD-L1 antibody, or an antigen-binding portion thereof) in a human subject in need thereof, wherein the subject is identified as exhibiting a combined biomarker comprising (a) a high *IFNγ* inflammatory signature score and (b) a low *TDO2* gene expression score prior to the administration; and wherein the *IFNγ* inflammatory signature score is determined by measuring the expression of an a panel of inflammatory genes comprising interferon gamma (*IFNγ*) in a sample obtained from the subject, and subsequently calculating the score from the measured expression levels.

Also disclosed is an IDO1 inhibitor (e.g., linrodostat mesylate) for identifying a human subject afflicted with a cancer suitable for treatment with an IDO1 inhibitor (e.g., linrodostat mesylate) in combination with an anti-PD-1 antagonist (e.g., an anti-PD-1 antibody such as nivolumab, an anti-PD-L1 antibody, or an antigen-binding portion thereof), wherein an *IFNγ* inflammatory signature score and *TDO2* gene expression are measured in a sample obtained from the subject; and wherein the *IFNγ* inflammatory signature score is determined by measuring the expression of an *IFNγ* inflammatory gene panel in a sample obtained from the subject, and subsequently calculating the score from the measured expression levels. In some aspects, the subject exhibits a combined biomarker comprising (a) a high *IFNγ* inflammatory signature score and (b) a low *TDO2* gene expression score.

The term "biomarker" as used herein refers to a factor that is a distinctive indicator of a biological process, biological event, and/or pathologic condition, e.g., a predictor of clinical response to treatment of a cancer, e.g., with a an IDO1 inhibitor (e.g., linrodostat) in combination with an anti-PD-1 antagonist (e.g., an anti-PD-1 antibody such as nivolumab, an anti-PD-L1 antibody, or an antigen-binding portion thereof). As used herein, the term biomarker encompasses both molecular biomarkers and measurements and/or scores derived from the measurement of such molecular biomarkers. Thus, in the context of the present disclosure, the term "biomarker" encompasses, e.g., biological biomarkers (e.g., a particular gene or genes or expression product such as an mRNA or protein) and numeric values or other quantitative or qualitative descriptors, e.g., "scores," integrating one or more measurements.

In a particular aspect, the term biomarker refers to a gene product (e.g., a nucleic or a protein) or a combination thereof that can be determined and associated, e.g., with the outcome of a treatment of cancer.

In some aspects, the biomarker is a "combined biomarker" or "composite biomarker," i.e., a biomarker comprising several discrete biomarkers. In an aspect of the present disclosure, the biomarker is a "combined biomarker" comprising two scores, i.e., a score related to a multigene interferon gamma inflammatory signature and a second score corresponding to the expression level of the TDO2 gene. The components of the combined biomarker of the present disclosure can be elevated (i.e., they can be "higher") or they can be reduced (i.e., they can be "lower") with respect to a reference level. The term "reference level" as used herein refers to the level of one or more biomarkers disclosed herein, or a score derived from the measurement of one or more biomarkers disclosed herein, wherein the level is higher or lower than a reference levels or reference score corresponding to a reference group (e.g., naive individuals, individuals associated with a particular group, such as a prognostic group, for example recurrence of the caner or non-response of the cancer to a particular therapeutic agent or combination thereof).

In some aspects, the IFNγ inflammatory signature score is determined by measuring the expression of a panel of IFNγ related inflammatory genes ("IFNγ inflammatory gene panel") in a cancer sample, e.g., a tumor sample, obtained from the subject, wherein the IFNγ inflammatory gene panel comprises 1 inflammatory gene, 2 inflammatory genes, 3 inflammatory genes, 4 inflammatory genes, 5 inflammatory genes, 6 inflammatory genes, 7 inflammatory genes, 8 inflammatory genes, 9 inflammatory genes, 10 inflammatory genes, 11 inflammatory genes, 12 inflammatory genes, 13 inflammatory genes, 14 inflammatory genes, 15 inflammatory genes, 16 inflammatory genes, 17 inflammatory genes, 18 inflammatory genes, 19 inflammatory genes, or 20 inflammatory genes, wherein the inflammatory genes are IFNγ related inflammatory genes selected from the group consisting of IFNy, CXCL10, HLA-DRA, CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STAT1, HLA.E, CCR5, CXCL11, GZMA, PRF1, IR2RG, CD3D, CD2, ITGAL, TAGAP, CIITA, PTPRC, CD3E, GZMK, GZMB, PDCD1, SLAMF6, and CXCL13.

In some aspects, the IFNγ inflammatory signature score is determined by measuring the expression of a IFNγ panel of inflammatory genes ("IFNγ inflammatory gene panel") in a cancer sample, e.g., a tumor sample, obtained from the subject, wherein the IFNγ inflammatory gene panel consists, or consists essentially of 1 inflammatory gene, 2 inflammatory genes, 3 inflammatory genes, 4 inflammatory genes, 5 inflammatory genes, 6 inflammatory genes, 7 inflammatory genes, 8 inflammatory genes, 9 inflammatory genes, 10 inflammatory genes, 11 inflammatory genes, 12 inflammatory genes, 13 inflammatory genes, 14 inflammatory genes, 15 inflammatory genes, 16 inflammatory genes, 17 inflammatory genes, 18 inflammatory genes, 19 inflammatory genes, or 20 inflammatory genes, wherein the inflammatory genes are IFNγ related inflammatory genes selected from the group consisting of IFNy, CXCL10, HLA-DRA, CXCR6, TIGIT, CD274 (PD-L1), PDCD1LG2 (PD-L2), LAG3, NKG7, PSMB10, CMKLR1, CD8A, *IDO1,* CCL5, CXCL9, HLA.DQA1, CD276, HLA.DRB1, STAT1, HLA.E, CCR5, CXCL11, GZMA, PRF1, IR2RG, CD3D, CD2, ITGAL, TAGAP, CIITA, PTPRC, CD3E, GZMK, GZMB, PDCD1, SLAMF6, and CXCL13.

In some aspects, the IFNγ inflammatory gene panel consists of less than about 25, less than about 24, less than about 23, less that about 22, less than about 21, less than about 20. Less that about 19, less than about 18, less than about 17, less than about 16, less than about 15, less than about 14, less than about 13, less than about 12, less than about 11, less than about 10, less than about 9, less than about, less than about 8, less than about 7, less than about 6, or less than about 5 IFNγ related inflammatory genes.

In some aspects, the IFNγ inflammatory gene panel consists of less than 25 IFNγ related inflammatory genes. In some aspects, the IFNγ inflammatory gene panel consists of less than 24 IFNγ related inflammatory genes. In some aspects, the IFNγ inflammatory gene panel consists of less than 23 IFNγ related inflammatory genes. In some aspects, the IFNγ inflammatory gene panel consists of less than 22 IFNγ related inflammatory genes. In some aspects, the IFNγ inflammatory gene panel consists of less than 21 IFNγ related inflammatory genes. In some aspects, the IFNγ inflammatory gene panel consists of less than 20 IFNγ related inflammatory genes. In some aspects, the IFNγ inflammatory gene panel consists of less than 19 IFNγ related inflammatory genes. In some aspects, the IFNγ inflammatory gene panel consists of less than 18 IFNγ related inflammatory genes. In some aspects, the IFNγ inflammatory gene panel consists of less than 17 IFNγ related inflammatory genes. In some aspects, the IFNγ inflammatory gene panel consists of less than 16 IFNγ related inflammatory genes. In some aspects, the IFNγ inflammatory gene panel consists of less than 15 IFNγ related inflammatory genes. In some aspects, the IFNγ inflammatory gene panel consists of less than 14 IFNγ related inflammatory genes. In some aspects, the IFNγ inflammatory gene panel consists of less than 13 IFNγ related inflammatory genes. In some aspects, the IFNγ inflammatory gene panel consists of less than 12 IFNγ related inflammatory genes. In some aspects, the IFNγ inflammatory gene panel consists of less than 11 IFNγ related inflammatory genes. In some aspects, the IFNγ inflammatory gene panel consists of less than 10 IFNγ related inflammatory genes. In some aspects, the IFNγ inflammatory gene panel consists of less than 9 IFNγ related inflammatory genes. In some aspects, the IFNγ inflammatory gene panel consists of less than 8 IFNγ related inflammatory genes. In some aspects, the IFNγ inflammatory gene panel consists of less than 7 IFNγ related inflammatory genes. In some aspects, the IFNγ inflammatory gene panel consists of less than 6 IFNγ related inflammatory genes. In some aspects, the IFNγ inflammatory gene panel consists of less than 5 IFNγ related inflammatory genes. In certain aspects, the IFNγ inflammatory gene panel consists of 4 IFNγ related inflammatory genes. In certain aspects, the IFNγ inflammatory gene panel consists of 3 IFNγ related inflammatory genes.

In some aspects, the IFNγ inflammatory gene panel comprises at least 2, 3, 4, 5, 6, 7, 8, 9, 10, 11, 12, 13, 14, 15, 16, 17, 18, 19, or 20 genes selected from the group consisting of *IFNγ, CXCL10, HLA-DRA, CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRBI, STATI, HLA.E, CCR5, CXCL11, GZMA, PRF1, IR2RG, CD3D, CD2, ITGAL, TAGAP, CIITA, PTPRC, CD3E, GZMK, GZMB, PDCD1, SLAMF6,* and *CXCL13.*

Thus, in some aspects, the IFNγ inflammatory gene panel can comprise genes related to cytolytic activity (e.g., granzyme A/B/K, and/or PRF1), cytokines/chemokines for initiation of inflammation (e.g., CXCR6, CXCL9, CCL5, and/or CCR5), T cell markers (e.g., CD3D, Cd3E, CD2, and/or IL2RG [encoding IL-2Rγ]), NK cell activity (e.g., NKG7 and/or HLA-E), antigen presentation (e.g., CIITA, and/or HLA-DRA), and/or additional immunomodulatory factors (e.g., LAG3, IDO1, and/or SLAMF6). See, e.g., Ayers et al. (2017) J. Clin. Invest. 127: 2930-40; and U.S. Appl. Publ. No. US2016-0304969.

In the present invention, the inflammatory genes comprise *IFNy, CXCL10, CXCL9, HLA-DRA, IDO1, STATI, CCR5, CXCL11, GZMA,* and *PRF1.*

In some aspects, the IFNγ inflammatory gene panel consists or consists essentially of (i) *IFNy, CXCL10, CXCL9, HLA-DRA, IDO1,* and *STAT1;*
(ii) *IFNγ, CXCL10, CXCL9, HLA-DRA, IDO1, STATI, CCR5, CXCL11, GZMA,* and *PRF1*;
(iii) *IFNγ, CXCR6, CD3D, CD2, ITGAL, TAGAP, CIITA, HLA-DRA, PTPRC, CXCL9, CCL5, NKG7, GZMA, PRF1, CCRS, CD3E, HLA-E, GZMK, GZMB, PDCD, SLAMF6, CXCL13, CXCL10, IDO1, LAG3, STAT1,* and *CXCL11*;
*(iv) IR2RG, CXCR6, CD3D, CD2, ITGAL, TAGAP, CIITA, HLA-DRA, PTPRC, CXCL9, CCL5, NKG7, GZMA, PRF1, CCR5, CD3E, GZMK, IFNG, HLA-E, GZMB, PDCD1, SLAMF6, CXCL13, CXCL10, IDO1, LAG3, STAT1,* and *CXCL11*; or
(v) any combination thereof, or any combination of the genes in (i)-(iv).

In one aspect, the IFNγ inflammatory gene panel consists of *IFNy, CXCL10, CXCL9, HLA-DRA, IDO1,* and *STATI.*

In one aspect, the IFNγ inflammatory gene panel consists of *IFNy, CXCL10, CXCL9, HLA-DRA, IDO1, STATI, CCR5, CXCL11, GZMA,* and *PRF1.*

In one aspect, the IFNγ inflammatory gene panel consists of *CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STATI,* and *HLA.E.*

In one aspect, the IFNγ inflammatory gene panel consists of *IR2RG, CXCR6, CD3D, CD2, ITGAL, TAGAP, CIITA, HLA-DRA, PTPRC, CXCL9, CCL5, NKG7, GZMA, PRF1, CCR5, CD3E, GZMK, IFNG, HLA-E, GZMB, PDCD1, SLAMF6, CXCL13, CXCL10, IDO1, LAG3, STAT1,* and *CXCL11.*

In some aspects, the IFNγ inflammatory gene panel consists of *IFNy, CXCL10, CXCL9, HLA-DRA, IDO1,* and *STAT1,* and 1 additional IFNγ related inflammatory gene, 2 additional IFNγ related inflammatory genes, 3 additional IFNγ related inflammatory genes, 4 additional IFNγ related inflammatory genes, 5 additional IFNγ related inflammatory genes, 6 additional IFNγ related inflammatory genes, 7 additional IFNγ related inflammatory genes, 8 additional IFNγ related inflammatory genes, 9 additional IFNγ related inflammatory genes, 10 additional IFNγ related inflammatory genes, 11 additional IFNγ related inflammatory genes, 12 additional IFNγ related inflammatory genes, 13 additional IFNγ related inflammatory genes, 14 additional IFNγ related inflammatory genes, 15 additional IFNγ related inflammatory genes, 16 additional IFNγ related inflammatory genes, 17 additional IFNγ related inflammatory genes, 18 additional IFNγ related inflammatory genes, 19 additional IFNγ related inflammatory genes, or 20 additional IFNγ related inflammatory genes.

In other aspects, the IFNγ inflammatory gene panel consists of *IFNy, CXCL10, CXCL9, HLA-DRA, IDO1, STATI, CCR5, CXCL11, GZMA,* and *PRF1,* and 1 additional IFNγ related inflammatory gene, 2 additional IFNγ related inflammatory genes, 3 additional IFNγ related inflammatory genes, 4 additional IFNγ related inflammatory genes, 5 additional IFNγ related inflammatory genes, 6 additional IFNγ related inflammatory genes, 7 additional IFNγ related inflammatory genes, 8 additional IFNγ related inflammatory genes, 9 additional IFNγ related inflammatory genes, 10 additional IFNγ related inflammatory genes, 11 additional IFNγ related inflammatory genes, 12 additional IFNγ related inflammatory genes, 13 additional IFNγ related inflammatory genes, 14 additional IFNγ related inflammatory genes, 15 additional IFNγ related inflammatory genes, 16 additional IFNγ related inflammatory genes, 17 additional IFNγ related inflammatory genes, 18 additional IFNγ related inflammatory genes, 19 additional IFNγ related inflammatory genes, or 20 additional IFNγ related inflammatory genes.

In other aspects, the IFNγ inflammatory gene panel consists of *CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STAT1,* and *HLA.E,* and 1 additional IFNγ related inflammatory gene, 2 additional IFNγ related inflammatory genes, 3 additional IFNγ related inflammatory genes, 4 additional IFNγ related inflammatory genes, 5 additional IFNγ related inflammatory genes, 6 additional IFNγ related inflammatory genes, 7 additional IFNγ related inflammatory genes, 8 additional IFNγ related inflammatory genes, 9 additional IFNγ related inflammatory genes, 10 additional IFNγ related inflammatory genes, 11 additional IFNγ related inflammatory genes, 12 additional IFNγ related inflammatory genes, 13 additional IFNγ related inflammatory genes, 14 additional IFNγ related inflammatory genes, 15 additional IFNγ related inflammatory genes, 16 additional IFNγ related inflammatory genes, 17 additional IFNγ related inflammatory genes, 18 additional IFNγ related inflammatory genes, 19 additional IFNγ related inflammatory genes, or 20 additional IFNγ related inflammatory genes.

In other aspects, the IFNγ inflammatory gene panel consists of *IFNy, IR2RG, CXCR6, CD3D, CD2, ITGAL, TAGAP, CIITA, HLA-DRA, PTPRC, CXCL9, CCL5, NKG7, GZMA, PRF1, CCR5, CD3E, GZMK, HLA-E, GZMB, PDCD1, SLAMF6, CXCL13, CXCL10, IDO1, LAG3, STATI, CXCL11,* and 1 additional IFNγ related inflammatory gene, 2 additional IFNγ related inflammatory genes, 3 additional IFNγ related inflammatory genes, 4 additional IFNγ related inflammatory genes, 5 additional IFNγ related inflammatory genes, 6 additional IFNγ related inflammatory genes, 7 additional IFNγ related inflammatory genes, 8 additional IFNγ related inflammatory genes, 9 additional IFNγ related inflammatory genes, 10 additional IFNγ related inflammatory genes, 11 additional IFNγ related inflammatory genes, 12 additional IFNγ related inflammatory genes, 13 additional IFNγ related inflammatory genes, 14 additional IFNγ related inflammatory genes, 15 additional IFNγ related inflammatory genes, 16 additional IFNγ related inflammatory genes, 17 additional IFNγ related inflammatory genes, 18 additional IFNγ related inflammatory genes, 19 additional IFNγ related inflammatory genes, or 20 additional IFNγ related inflammatory genes.

The *IFNγ, CXCL10, HLA-DRA, CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRBI, STATI, HLA.E, CCR5, CXCL11, GZMA, PRF1, IR2RG, CD3D, CD2, ITGAL, TAGAP, CIITA, PTPRC, CD3E, GZMK, GZMB, PDCD1, SLAMF6,* and *CXCL13* molecular biomarkers disclosed herein also include
(i) proteins or fragments thereof having at least about 70%, at least about 71%, at least about 72%, at least about 73%, at least about 74%, at least about 75%, at least about 76%, at least about 77%, at least about 78%, at least about 79%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to their corresponding wild type sequence; and
(ii) nucleic acids (e.g., mRNA) having at least about 70%, at least about 71%, at least about 72%, at least about 73%, at least about 74%, at least about 75%, at least about 76%, at least about 77%, at least about 78%, at least about 79%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% sequence identity to the respective wild type nucleic acid sequences encoding the corresponding molecular biomarker.

The molecular biomarkers disclosed herein also include isoforms and/or variants thereof. As used herein, a "variant" biomarker contains at least one amino acid sequence alteration as compared to the amino acid sequence of the corresponding wild-type polypeptide. An amino acid sequence alteration can be, for example, a substitution, a deletion, or an insertion of one or more amino acids, preferably conservative substitutions. A variant biomarker can have any combination of amino acid substitutions, deletions or insertions. In one aspect, a biomarker variant polypeptide can have an integer number of amino acid alterations such that its amino acid sequence shares at least about 60, at least about 70, at least about 80, at least about 85, at least about 90, at least about 95, at least about 97, at least about 98, at least about 99, at least about 99.5 or 100% identity with the amino acid sequence of the corresponding wild-type polypeptide.

In other aspects, a variant biomarker contains at least one nucleic acid sequence alteration as compared to the nucleic acid sequence of the corresponding DNA or RNA (e.g., mRNA). A nucleic acid sequence alteration can be, for example, a substitution, a deletion, or an insertion of one or more nucleotides, preferably conservative substitutions. A variant biomarker can have any combination of nucleic acid substitutions, deletions or insertions. In one aspect, a biomarker variant gene or gene product (e.g., mRNA) can have an integer number of nucleotide alterations such that its nucleotide sequence shares at least about 60%, at least about 70%, at least about 80%, at least about 85%, at least about 90%, at least about 95%, at least about 97%, at least about 98%, at least about 99%, at least about 99.5% or 100% identity with the nucleic acid sequence of the corresponding wild-type gene or gene product (e.g., mRNA).

In some aspects, the methods disclosed herein can comprise determining, submitting a sample taken for the subject for determination, or instructing a clinical laboratory to determine the expression level or activity of a molecular biomarkers disclosed herein or a combination thereof.

The present disclosure is directed to methods for treating a human subject afflicted with a cancer comprising administering to the subject a combination therapy comprising (i) a therapy with an anti-PD-1 antagonist, e.g., an anti-PD-1 antibody (e.g., nivolumab) or an anti-PD-L1 antibody, or an antigen binding portion thereof, generally administered intravenously, and (ii) a therapy with an IDO1 inhibitor (e.g., linrodostat mesylate) generally administered orally.

In certain aspects, the anti-PD-1 antibody or antigen binding portion thereof cross-competes with nivolumab for binding to human PD-1. In some aspects, the anti-PD-1 antibody or antigen binding portion thereof binds to the same epitope as nivolumab. In some aspects, the anti-PD-1 antibody is a chimeric, humanized or human monoclonal antibody or an antigen binding portion thereof. In some aspects, the anti-PD-1 antibody comprises a heavy chain constant region which is of a human IgG1 or IgG4 isotype.

In some aspects, the anti-PD-1 antibody comprises nivolumab, pembrolizumab, cemiplimab, or an antigen-binding portion thereof. In some aspects, the anti-PD-1 antibody is nivolumab, pembrolizumab, or cemiplimab.

In some aspects, the anti-PD-L1 antibody comprises avelumab, atezolizumab, durvalumab, or an antigen binding portion thereof. In some aspects, the anti-PD-L1 antibody is avelumab, atezolizumab, or durvalumab.

In some aspects, the anti-PD-1 antibody (e.g., nivolumab) or the anti-PD-L1 antibody is administered once every week, once every 2 weeks, once about every 3 weeks, once about every 4 weeks, once about every 5 weeks, or once about every 6 weeks. In some aspects, the anti-PD-1 antibody (e.g., nivolumab) or the anti-PD-L1 antibody is administered at a dose ranging from about 0.1 mg/kg to about 20.0 mg/kg body weight. In some aspects, the anti-PD-1 antibody (e.g., nivolumab) or the anti-PD-L1 antibody is administered at a dose of about 0.1 mg/kg, about 0.5 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg or about 20 mg/kg.

In some aspects, the anti-PD-1 antibody (e.g., nivolumab) or anti-PD-L1 antibody is administered at a dose ranging from at least about 0.1 mg/kg to at least about 10.0 mg/kg body weight, e.g., about 0.1 mg/kg, about 0.5 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg or about 20 mg/kg, once about every 1, 2, 3, 4, 5 or 6 weeks. In one aspect, the anti-PD-1 antibody (e.g., nivolumab) or anti-PD-L1 antibody is administered at a dose ranging from at least about 0.1 mg/kg to at least about 10.0 mg/kg body weight once about every 1, 2 or 3 weeks. In another aspect, the anti-PD-1 antibody (e.g., nivolumab) or anti-PD-L1 antibody is administered at a dose of at least about 3 mg/kg body weight once about every 2 weeks.

In some aspects, the anti-PD-1 antibody (e.g., nivolumab) or anti-PD-L1 antibody or an antigen-binding portion thereof is administered at a flat dose. In some aspects, the anti-PD-1 antibody (e.g., nivolumab) or anti-PD-L1 antibody or antigen-binding portion thereof is administered at a flat dose of at least about 25 mg/dose, at least about 50 mg/dose, at least about 75 mg/dose, at least about 100 mg/dose, at least about 125 mg/dose, at least about 150 mg/dose, at least about 175 mg/dose, at least about 200 mg/dose, at least about 220 mg/dose, at least about 240 mg/dose, at least about 260 mg/dose, at least about 280 mg/dose, at least about 300 mg/dose, at least about 320 mg/dose, at least about 340 mg/dose, at least about 360 mg/dose, at least about 380 mg/dose, at least about 400 mg/dose, at least about 420 mg/dose, at least about 440 mg/dose, at least about 460 mg/dose, at least about 480 mg/dose, at least about 500 mg/dose, or at least about 550 mg/dose.

In some aspects, the anti-PD-1 antibody (e.g., nivolumab) or anti-PD-L1 antibody or antigen-binding portion thereof is administered at a flat dose of about 240 mg/dose. In some aspects, the anti-PD-1 antibody (e.g., nivolumab) or anti-PD-L1 antibody or antigen-binding portion thereof is administered at a flat dose of about 480 mg/dose. In some aspects, the anti-PD-1 antibody (e.g., nivolumab) or anti-PD-L1 antibody or antigen-binding portion thereof is administered at a flat dose about once every 1, 2, 3 or 4 weeks. In some aspects, the anti-PD-1 antibody (e.g., nivolumab) or anti-PD-L1 antibody or antigen-binding portion thereof is administered at a flat dose of about 240 mg/dose once about every two weeks. In some aspects, the anti-PD-1 antibody (e.g., nivolumab) or anti-PD-L1 antibody or antigen-binding portion thereof is administered at a flat dose of about 480 mg/dose once about every four weeks.

In some aspects, the anti-PD-1 antibody (e.g., nivolumab) or anti-PD-L1 antibody is administered for as long as clinical benefit is observed or until unmanageable toxicity or disease progression occurs. In some aspects, the anti-PD-1 antibody (e.g., nivolumab) or anti-PD-L1 antibody is formulated for intravenous administration. In some aspects, the anti-PD-1 antibody (e.g., nivolumab) or anti-PD-L1 antibody is administered via bolus intravenous administration. In some aspects, the bolus in a fast bolus. In other aspects, the bolus is a slow bolus. In some aspects, the anti-PD-1 antibody (e.g., nivolumab) or anti-PD-L1 antibody is administered via intravenous infusion. In some aspects, the anti-PD-1 antibody (e.g., nivolumab) or anti-PD-L1 antibody is administered at a subtherapeutic dose.

In some aspects, the immunotherapy with an anti-PD-1 antibody (e.g., nivolumab) or anti-PD-L1 antibody, is accompanied with a second therapy comprising the administration of an IDO1 inhibitor (e.g., linrodostat mesylate) to the subject. In some aspects, the IDO1 inhibitor is a compound that selectively inhibits IDO1. In some aspects, the IDO1 inhibitor does not inhibit TDO2 enzymatic activity. In some specific aspects of the present disclosure, the IDO1 inhibitor is linrodostat ((2R)-N-(4-chlorophenyl)-2-(cis-4-(6-fluoroquinolin-4-yl)cyclohexyl) propanamide) or a salt thereof. In some aspects, the linrodostat salt is linrodostat mesylate. In some aspects, the IDO1 inhibitor is selected from the group consisting of linrodostat, 1-methyl-DL-tryptophan, p-(3-benzofuranyl)-DL-alanine, p-[3-benzo(b)thienyl]-DL-alanine; 6-nitro-L-tryptophan, epacadostat, PF-06840003 (3-(5-Fluoro-1H-indol-3-yl)pyrrolidine-2,5-dione), navoximod, IOM2983, RG-70099, TPST-8844, SRX-3217, PDX-26116, NLG-802, MK-7162, LY-3381916, LY-01013, KHK-2455, IO-102, IO-101, indoximod, HTI-1090, EOS-200271, DN-1406131, DN-016, BLH-1131, BGB-5777, BEBT-303, AN-0015, AI-001, and any combination thereof. See Prendergast et al. (2017) Cancer Res. 77(24); 6795-811.

In some aspects, the IDO1 inhibitor (e.g., linrodostat mesylate) is formulated for oral administration. In some aspects, the IDO1 inhibitor (e.g., linrodostat mesylate) is administered at a flat dose of about 100 mg. In some aspects, the IDO1 inhibitor (e.g., linrodostat mesylate) is administered at a flat dose of about 200 mg. In some aspects, the IDO1 inhibitor (e.g., linrodostat mesylate) is administered at a flat dose of about 100 mg every day. In some aspects, the IDO1 inhibitor (e.g., linrodostat mesylate) is administered at a flat dose of about 200 mg every day. In some aspects, the IDO1 inhibitor is administered at a flat dose of about 25 mg/day, about 50 mg/day, about 75 mg/day, about 100 mg/day, about 125 mg/day, about 150 mg/day, about 175 mg/day, about 200 mg/day, about 225 mg/day, about 250 mg/day, about 275 mg/day, or about 300 mg/day.

In some aspects, the IDO1 inhibitor (e.g., linrodostat mesylate) is administered at a flat dose of about 100 mg every day during the course of the treatment with the anti-PD-1 antibody (e.g., nivolumab) or anti-PD-L1 antibody. In some aspects, the IDO1 inhibitor (e.g., linrodostat mesylate) is administered at a flat dose of about 200 mg every day during the course of the treatment with the anti-PD-1 antibody (e.g., nivolumab) or anti-PD-L1 antibody. In some aspects, the IDO1 inhibitor is administered at a flat dose of about 25 mg/day, about 50 mg/day, about 75 mg/day, about 100 mg/day, about 125 mg/day, about 150 mg/day, about 175 mg/day, about 200 mg/day, about 225 mg/day, about 250 mg/day, about 275 mg/day, or about 300 mg/day during the course of the treatment with the anti-PD-1 antibody (e.g., nivolumab) or anti-PD-L1 antibody.

In a specific aspect, the anti-PD-1 antibody is nivolumab and the IDO1 inhibitor is linrodostat mesylate. In some aspects, nivolumab is administered to a subject in need thereof (e.g., a subject with a cancer) at a flat dose of about 240 mg/dose once about every two weeks and linrodostat mesylate is administered at a flat dose of about 100 mg/dose every day during the course of the treatment with nivolumab. In some aspects, nivolumab is administered to a subject in need thereof (e.g., a subject with a cancer) at a flat dose of about 480 mg/dose once about every four weeks and linrodostat mesylate is administered at a flat dose of about 100 mg/dose every day during the course of the treatment with nivolumab. In some aspects, nivolumab is administered to a subject in need thereof (e.g., a subject with a cancer) at a flat dose of about 240 mg/dose once about every two weeks and linrodostat mesylate is administered at a flat dose of about 200 mg/dose every day during the course of the treatment with nivolumab. In some aspects, nivolumab is administered to a subject in need thereof (e.g., a subject with a cancer) at a flat dose of about 480 mg/dose once about every four weeks and linrodostat mesylate is administered at a flat dose of about 200 mg/dose every day during the course of the treatment with nivolumab. In some aspects, these treatments are administered to the subject if the subject exhibits a combined biomarker comprising (a) a high *IFNy* inflammatory signature score and (b) a low *TDO2* gene expression score. In some aspects, the treatments described herein can be administered to a subject with a cancer which is refractory, e.g., the cancer is refractory following at least one prior anticancer therapy, e.g., an anticancer therapy comprising the administration of at least one anticancer agent. In some aspects, the at least one anticancer agent comprises an immunotherapy.

In some aspects, the anti-PD-1 antagonist (e.g., an anti-PD-1 antibody such as nivolumab) is administered on the same day, before or after the administration of the IDO1 inhibitor (e.g., linrodostat mesylate), e.g., immediately before or after the administration of the IDO1 inhibitor. In some aspects, the anti-PD-1 antagonist (e.g., an anti-PD-1 antibody such as nivolumab) is administered concurrently with the IDO1 inhibitor (e.g., linrodostat mesylate).

In some aspects, administering a combination therapy disclosed herein (e.g., an anticancer therapy comprising the administration of nivolumab and an IDO1 inhibitor such as linrodostat mesylate to a subject in need thereof) treats the cancer. In some aspects, administering the combination therapy reduces the cancer burden. In some aspects, cancer burden is reduced by at least about 10%, at least 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100% compared to the cancer burden prior to the administration.

In some aspects, administering the combination therapy reduces tumor volume. In some aspects, tumor volume is reduced by at least about 10%, at least 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100% compared to the tumor volume prior to the administration.

In some aspects, administering the combination therapy reduces cancer cell proliferation. In some aspects, cancer proliferation is reduced by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100% compared to the cancer cell proliferation prior to the administration.

In some aspects, administering the combination therapy reduces tumor growth. In some aspects, tumor growth is reduced by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100% compared to the tumor growth prior to the administration.

In some aspects, the subject exhibits progression-free survival of at least about one month, at least about 2 months, at least about 3 months, at least about 4 months, at least about 5 months, at least about 6 months, at least about 7 months, at least about 8 months, at least about 9 months, at least about 10 months, at least about 11 months, at least about one year, at least about eighteen months, at least about two years, at least about three years, at least about four years, or at least about five years after the initial administration of a combination therapy disclosed herein (e.g., an anticancer therapy comprising the administration of nivolumab and an IDO1 inhibitor such as linrodostat mesylate to a subject in need thereof which is administered to the subject if the subject exhibits a combined biomarker comprising (a) a high *IFNγ* inflammatory signature score and (b) a low *TDO2* gene expression score).

In some aspects, the subject exhibits stable disease after the administration of a combination therapy disclosed herein (e.g., an anticancer therapy comprising the administration of nivolumab and an IDO1 inhibitor such as linrodostat mesylate to a subject in need thereof which is administered to the subject if the subject exhibits a combined biomarker comprising (a) a high *IFNγ* inflammatory signature score and (b) a low *TDO2* gene expression score).

In some aspects, the subject exhibits a partial response after the administration of a combination therapy disclosed herein (e.g., an anticancer therapy comprising the administration of nivolumab and an IDO1 inhibitor such as linrodostat mesylate to a subject in need thereof which is administered to the subject if the subject exhibits a combined biomarker comprising (a) a high *IFNγ* inflammatory signature score and (b) a low *TDO2* gene expression score).

In some aspects, the subject exhibits a complete response after the administration of a combination therapy disclosed herein (e.g., an anticancer therapy comprising the administration of nivolumab and an IDO1 inhibitor such as linrodostat mesylate to a subject in need thereof which is administered to the subject if the subject exhibits a combined biomarker comprising (a) a high *IFNγ* inflammatory signature score and (b) a low *TDO2* gene expression score).

In some aspects, administering a combination therapy disclosed herein to a subject in need thereof (e.g., an anticancer therapy comprising the administration of nivolumab and an IDO1 inhibitor such as linrodostat mesylate to a subject in need thereof which is administered to the subject if the subject exhibits a combined biomarker comprising (a) a high *IFNγ* inflammatory signature score and (b) a low *TDO2* gene expression score) improves progression-free survival probability by at least about 10%, at least about 20%, at least about 30%, at least about 40%, at least about 50%, at least about 60%, at least about 70%, at least about 80%, at least about 90%, at least about 100%, at least about 110%, at least about 120%, at least about 130%, at least about 140%, or at least about 150%, compared to the progression-free survival probability of a subject not exhibiting a combined biomarker comprising (a) a high *IFNγ* inflammatory signature score and (b) a low *TDO2* gene expression score.

In some aspects, administering a combination therapy disclosed herein to a subject in need thereof (e.g., an anticancer therapy comprising the administration of nivolumab and an IDO1 inhibitor such as linrodostat mesylate to a subject in need thereof which is administered to the subject if the subject exhibits a combined biomarker comprising (a) a high *IFNγ* inflammatory signature score and (b) a low *TDO2* gene expression score) improves overall survival probability by at least about 25%, at least about 50%, at least about 75%, at least about 100%, at least about 125%, at least about 150%, at least about 175%, at least about 200%, at least about 225%, at least about 250%, at least about 275%, at least about 300%, at least about 325%, at least about 350%, or at least about 375%, compared to the overall survival probability of a subject not exhibiting a combined biomarker comprising (a) a high *IFNγ* inflammatory signature score and (b) a low *TDO2* gene expression score.

### III. Combined Biomarker and Gene Panel

The methods of the present disclosure relate to the predictive use of a combined biomarker which has two components: an *IFNγ* inflammatory signature score obtained from an IFNγ inflammatory gene panel, and a second score, a *TDO2* gene expression score, which is derived from the level of expression of the *TDO2* gene.

The IFNγ inflammatory signature score, as used herein, is a measurement of the combined expression level the genes present in an IFNγ inflammatory gene panel which, e.g., comprises, consists, or consists essentially of
*(i) IFNy, CXCL10, CXCL9, HLA-DRA, IDO1,* and *STAT1;*
(ii) *IFNy, CXCL10, CXCL9, HLA-DRA, IDO1, STAT1, CCR5, CXCL11, GZMA,* and *PRF1; or,*
(iii) *CXCR6, CD3D, CD2, ITGAL, TAGAP, CIITA, HLA-DRA, PTPRC, CXCL9, CCL5, NKG7, GZMA, PRF1, CCR5, CD3E, HLA-E, GZMK, GZMB, PDCD, SLAMF6, CXCL13, CXCL10, IDO1, LAG3, STAT1,* and *CXCL11*; or,
*(iv)IFNy, IR2RG, CXCR6, CD3D, CD2, ITGAL, TAGAP, CIITA, HLA-DRA, PTPRC, CXCL9, CCL5, NKG7, GZMA, PRF1, CCR5, CD3E, GZMK, HLA-E, GZMB, PDCD1, SLAMF6, CXCL13, CXCL10, IDO1, LAG3, STAT1,* and *CXCL11,*
in a sample obtained from the subject.

In principle, any biological sample comprising one or more cancer cells can be used in the methods disclosed herein. In some aspects, the sample is selected from a cancer (e.g., a tumor) biopsy, a blood sample, a serum sample, or any combination thereof. In the present invention, the sample is a tumor tissue biopsy sample. In some aspects, the sample is obtained from the stroma of a tumor. In certain aspects, the sample is a tumor biopsy collected from the subject prior to administration of the anti-PD-1 antagonist (e.g., an anti-PD-1 or anti-PD-L1 antibody) and/or prior to the administration of the IDO1 inhibitor (e.g., linrodostat mesylate). In some aspects, the sample obtained from the subject is a formalin-fixed tumor biopsy. In some aspects, the sample obtained from the subject is a paraffin-embedded tumor biopsy. In some aspects, the sample obtained from the subject is a formalin-fixed paraffin-embedded tissue. In some aspects, the sample obtained from the subject is a fresh-frozen tumor biopsy. In some aspects, the sample obtained from the subject is a blood sample, or it is obtained by processing a blood sample (e.g., the sample is a specific subpopulation of cells extracted from a blood sample).

Any method known in the art for measuring the expression of a particular gene *(e.g., TDO2*) or a panel of genes (e.g., the genes in the IFNγ inflammatory gene panel) can be used in the methods of the present disclosure. In some aspects, the expression of one or more of the inflammatory genes in the *IFNγ* inflammatory gene panel is determined by detecting the presence of mRNA transcribed from the IFNγ related inflammatory gene or the TDO2 gene, the presence of a protein encoded by the IFNγ related inflammatory gene or the TDO2 gene, or both.

In some aspects, the expression of one or more of the genes in the IFNy inflammatory gene panel and/or the TDO2 gene is determined by measuring the level of inflammatory gene mRNA, e.g., by measuring the level of one or more of IFNγ mRNA, CXCL10 mRNA, CXCL9 mRNA, HLA-DRA mRNA, IDO1 mRNA, STAT1 mRNA, CCR5 mRNA, CXCL11 mRNA, GZMA mRNA, PRF1 mRNA or TDO2 mRNA, in at least one sample obtained from the subject.

In certain aspects, the IFNγ inflammatory signature score is determined by measuring the level of IFNγ mRNA, CXCL10 mRNA, CXCL9 mRNA, HLA-DRA mRNA, IDO1 mRNA, and STAT1 mRNA in at least one sample obtained from the subject.

In certain aspects, the IFNγ inflammatory signature score is determined by measuring the level of IFNγ mRNA, CXCL10 mRNA, CXCL9 mRNA, HLA-DRA mRNA, IDO1 mRNA, STAT1 mRNA, CCR5 mRNA, CXCL11 mRNA, GZMA mRNA, and PRF1 mRNA in at least one sample obtained from the subject.

In certain aspects, the IFNγ inflammatory signature score is determined by measuring the level of CXCR6 mRNA, TIGIT mRNA, CD274 (PD-L1) mRNA, PDCD1LG2 (PD-L2) mRNA, LAG3 mRNA, NKG7 mRNA, PSMB10 mRNA, CMKLR1 mRNA, CD8A mRNA, IDO1 mRNA, CCL5 mRNA, CXCL9 mRNA, HLA.DQA1 mRNA, CD276 mRNA, HLA.DRB1 mRNA, STAT1 mRNA, and HLA.E mRNA in at least one sample obtained from the subject.

In certain aspects, the IFNγ inflammatory signature score is determined by measuring the level of IFNγ mRNA, IR2RG mRNA, CXCR6 mRNA, CD3D mRNA, CD2 mRNA, ITGAL mRNA, TAGAP mRNA, CIITA mRNA, HLA-DRA mRNA, PTPRC mRNA, CXCL9 mRNA, CCL5 mRNA, NKG7 mRNA, GZMA mRNA, PRF1 mRNA, CCR5 mRNA, CD3E mRNA, GZMK mRNA, HLA-E mRNA, GZMB mRNA, PDCD1 mRNA, SLAMF6 mRNA, CXCL13 mRNA, CXCL10 mRNA, IDO1 mRNA, LAG3 mRNA, STAT1 mRNA, and CXCL11 mRNA in at least one sample obtained from the subject.

In some aspects, the TDO2 expression score is determined by measuring the level of TDO2 mRNA in at least one sample obtained from the subject. In some aspects, all the mRNA level measurements are conducted using a single sample. In other aspects, all the mRNA level measurements are conducted using more than one sample.

Any method known in the art can be used to measure the level of mRNA from the genes in the IFNγ inflammatory gene panel and/or TDO2. In some aspects, the presence of mRNA encoding the genes in the IFNγ inflammatory gene panel and/or the *TDO2* gene is determined using reverse transcriptase PCR. In some aspects, the mRNA from the in the IFNγ inflammatory gene panel and/or TDO2 is measured using reverse transcriptase PCR. In some aspects, the mRNA from the genes in the IFNγ inflammatory gene panel and/or TDO2 is measured using RNA *in situ* hybridization.

In some aspects, the expression of one or more of the genes in the IFNγ inflammatory gene panel and/or TDO2 is determined by measuring the level of expressed protein, e.g., by measuring the protein level of one or more of IFNy, CXCL10, HLA-DRA, CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STAT1, HLA.E, CCR5, CXCL11, GZMA, PRF1, IR2RG, CD3D, CD2, ITGAL, TAGAP, CIITA, PTPRC, CD3E, GZMK, GZMB, PDCD1, SLAMF6, CXCL13, and TDO2, in at least one sample obtained from the subject.

In certain aspects, the IFNγ inflammatory signature score is determined by measuring the level of IFNγ protein, CXCL10 protein, CXCL9 protein, HLA-DRA protein, IDO1 protein, and STAT1 protein in at least one sample obtained from the subject.

In certain aspects, the IFNγ inflammatory signature score is determined by measuring the level of IFNγ protein, CXCL10 protein, CXCL9 protein, HLA-DRA protein, IDO1 protein, STAT1 protein, CCR5 protein, CXCL11 protein, GZMA protein, and PRF1 protein in at least one sample obtained from the subject.

In certain aspects, the IFNγ inflammatory signature score is determined by measuring the level of CXCR6 protein, TIGIT protein, CD274 (PD-L1) protein, PDCD1LG2 (PD-L2) protein, LAG3 protein, NKG7 protein, PSMB10 protein, CMKLR1 protein, CD8A protein, IDO1 protein, CCL5 protein, CXCL9 protein, HLA.DQA1 protein, CD276 protein, HLA.DRB1 protein, STAT1 protein, and HLA.E protein in at least one sample obtained from the subject.

In certain aspects, the IFNγ inflammatory signature score is determined by measuring the level of IFNγ protein, IR2RG protein, CXCR6 protein, CD3D protein, CD2 protein, ITGAL protein, TAGAP protein, CIITA protein, HLA-DRA protein, PTPRC protein, CXCL9 protein, CCL5 protein, NKG7 protein, GZMA protein, PRF1 protein, CCR5 protein, CD3E protein, GZMK protein, HLA-E protein, GZMB protein, PDCD1 protein, SLAMF6 protein, CXCL13 protein, CXCL10 protein, IDO1 protein, LAG3 protein, STAT1 protein, and CXCL11 protein in at least one sample obtained from the subject.

In certain aspects, the TDO2 gene expression score is determined by measuring the level of TDO2 protein in at least one sample obtained from the subject.

Any method known in the art can be used to measure the level of expressed protein corresponding to one or more of the genes in the IFNy inflammatory gene panel and/or the TDO2 gene. In some aspects, the presence of the protein encoded by the genes in the IFNy inflammatory gene panel and/or the *TDO2* gene is determined using an IHC assay. In some aspects, the expressed protein level is measured using an immunohistochemistry (IHC) assay. In certain aspects, the IHC is an automated IHC. In some aspects, the assay is a protein IHC assay including topology that is correlated to the IFNy inflammatory gene panel.

In some aspects, the expression of one or more of the genes in the IFNy inflammatory gene panel and/or the TDO2 gene is normalized relative to the expression of one or more housekeeping genes. In some aspects, the one or more housekeeping genes are made up of genes that have relatively consistent expression across various cancer types (e.g., tumor types) in various subjects.

In some aspects, raw gene expression values are normalized following standard gene expression profiling (GEP) protocols. In these aspects, the IFNy inflammatory signature score and/or the TDO2 gene expression score can be calculated as the median or average of the log2-transformed normalized and scaled expression values across all of the target genes in the IFNy inflammatory gene panel and/or the TDO2 gene, and presented on a linear scale. In certain aspects, scores have positive or negative values, depending on whether gene expression is up- or down-regulated under a particular condition.

In certain aspects, a high IFNy inflammatory signature score is characterized by an IFNy inflammatory signature score that is greater than a reference IFNy inflammatory signature score.

In some aspects, the reference IFNy inflammatory signature score is an average inflammatory signature score. In some aspects, the average IFNy inflammatory signature score is determined by measuring the expression of the genes present in the IFNy inflammatory gene panel in cancer (e.g., tumor) samples obtained from a population of subjects, and calculating the average for the population of subjects. In some aspects, the average *IFNγ* inflammatory signature score is determined by averaging the expression of the *IFNy* inflammatory gene panel genes in cancer samples obtained from the population of subjects.

In some aspects, the reference IFNy inflammatory signature score is a median inflammatory signature score. In some aspects, the median IFNy inflammatory signature score is determined by measuring the expression of the genes present in the IFNy inflammatory gene panel in cancer (e.g., tumor) samples obtained from a population of subjects, and calculating the median for the distribution in the population of subjects. In some aspects, the median IFNy inflammatory signature score is determined by measuring the expression of the genes present in the IFNγ inflammatory gene panel in cancer (e.g., tumor) samples obtained from a population of subjects, and calculating the median for the distribution in the population of subjects. In some aspects, the median *IFNγ* inflammatory signature score is determined from the distribution of the expression of the *IFNγ* inflammatory gene panel genes in cancer samples obtained from the population of subjects.

In some aspects, the reference IFNγ inflammatory signature score is a predetermined cut-off or threshold value.

In some aspects, the high IFNy inflammatory signature score is characterized by an IFNy inflammatory signature score that is higher than the average IFNy inflammatory signature score of a reference sample (e.g., a sample or set of samples obtained from a subject or group of subjects). In some aspects, the high IFNy inflammatory signature score is characterized by an IFNy inflammatory signature score that is higher than the median IFNy inflammatory signature score of a reference sample (e.g., a sample or set of samples obtained from a subject or group of subjects). In some aspects, the high IFNy inflammatory signature score is characterized by an IFNy inflammatory signature score that is higher than a predetermined cut-off or threshold value.

In some aspects, the reference sample comprises a non-tumor tissue of the subject, a corresponding non-tumor tissue of the subject, or the corresponding tissue of subjects without a tumor. In some aspects, the reference sample comprises non-tumor tissue from a population of subject, a corresponding non-tumor tissue of a population of subjects, or the corresponding tissue of a population of subjects without a tumor.

In some aspects, a high IFNy inflammatory signature score is characterized by an IFNy inflammatory signature score that is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 125%, at least about 150%, at least about 175%, at least about 200%, at least about 225%, at least about 250%, at least about 275%, or at least about 300% higher than an average IFNy inflammatory signature score.

In some aspects, a high IFNy inflammatory signature score is characterized by an IFNy inflammatory signature score that is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 125%, at least about 150%, at least about 175%, at least about 200%, at least about 225%, at least about 250%, at least about 275%, or at least about 300% higher than a median IFNy inflammatory signature score.

In some aspects, a high IFNy inflammatory signature score is characterized by an IFNy inflammatory signature score that is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 125%, at least about 150%, at least about 175%, at least about 200%, at least about 225%, at least about 250%, at least about 275%, or at least about 300% higher than a predetermined cut-off or threshold IFNγ inflammatory signature score.

In some aspects, a high IFNy inflammatory signature score is characterized by an IFNy inflammatory signature score that is at least about 1.25-fold, at least about 1.30-fold, at least about 1.35-fold, at least about 1.40-fold, at least about 1.45-fold, at least about 1.50-fold, at least about 1.55-fold, at least about 1.60-fold, at least about 1.65-fold, at least about 1.70-fold, at least about 1.75-fold, at least about 1.80-fold, at least about 1.85-fold, at least about 1.90-fold, at least about 1.95-fold, at least about 2-fold, at least about 2.25-fold, at least about 2.50-fold, at least about 2.75-fold, at least about 3-fold, at least about 3.25-fold, at least about 3.50-fold, at least about 3.75-fold, or at least about 400-fold higher than an average IFNy inflammatory signature score, a median IFNy inflammatory signature score, or a predetermined cut-off or threshold IFNγ inflammatory signature score.

In certain aspects, a high IFNy inflammatory signature score is characterized by an IFNy inflammatory signature score of at least about -0.20, at least about -0.15, at least about - 0.10, at least about -0.05, at least about 0.00, at least about 0.05, at least about 0.10, at least about 0.15, at least about 0.20, at least about 0.25, at least about 0.30, at least about 0.35, at least about 0.40, at least about 0.45, at least about 0.50, at least about 0.55, at least about 0.60, at least about 0.65, at least about 0.70, at least about 0.75, at least about 0.80, at least about 0.85, at least about 0.90, at least about 0.95, or at least about 1.00, wherein the IFNy inflammatory signature score is determined according to a method disclosed herein.

In certain aspects, the average IFNy inflammatory signature score is about -0.2, about -0.15, about -0.1, about -0.05, about 0, about 0.05, about 0.1, about 0.15, about 0.2, about 0.25, about 0.3, about 0.35, about 0.4, about 0.45, about 0.5, about 0.55, about 0.6, about 0.65, about 0.7, about 0.75, about 0.8, about 0.85, about 0.9, about 0.95, or about 1, wherein the average IFNy inflammatory signature score is determined according to a method disclosed herein.

In certain aspects, the average IFNy inflammatory signature score is -0.2, -0.15, - 0.1, -0.05, 0, 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, or 1, wherein the average IFNγ inflammatory signature score is determined according to a method disclosed herein.

In certain aspects, the median IFNy inflammatory signature score is about -0.2, about -0.15, about -0.1, about -0.05, about 0, about 0.05, about 0.1, about 0.15, about 0.2, about 0.25, about 0.3, about 0.35, about 0.4, about 0.45, about 0.5, about 0.55, about 0.6, about 0.65, about 0.7, about 0.75, about 0.8, about 0.85, about 0.9, about 0.95, or about 1, wherein the median IFNy inflammatory signature score is determined according to a method disclosed herein.

In certain aspects, the median IFNy inflammatory signature score is -0.2, -0.15, - 0.1, -0.05, 0, 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, or 1, wherein the median IFNy inflammatory signature score is determined according to a method disclosed herein.

In certain aspects, the threshold IFNγ inflammatory signature score is about -0.2, about -0.15, about -0.1, about -0.05, about 0, about 0.05, about 0.1, about 0.15, about 0.2, about 0.25, about 0.3, about 0.35, about 0.4, about 0.45, about 0.5, about 0.55, about 0.6, about 0.65, about 0.7, about 0.75, about 0.8, about 0.85, about 0.9, about 0.95, or about 1, wherein the threshold IFNγ inflammatory signature score is determined according to a method disclosed herein.

In certain aspects, the threshold IFNγ inflammatory signature score is -0.2, -0.15, - 0.1, -0.05, 0, 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, or 1, wherein the threshold IFNy inflammatory signature score is determined according to a method disclosed herein.

In certain aspects, a low *TDO2* gene expression score is characterized by a *TDO2* gene expression score that is smaller (lower) than a reference *TDO2* gene expression score.

In some aspects, the reference *TDO2* gene expression score is an average *TDO2* gene expression score. In some aspects, the average *TDO2* gene expression score is determined by measuring the expression of the TDO2 genes in cancer (e.g., tumor) samples obtained from a population of subjects, and calculating the average for the population of subjects. In some aspects, the average *TDO2* gene expression score is determined by averaging the expression of the *TDO2* gene in cancer samples obtained from the population of subjects.

In some aspects, the reference *TDO2* gene expression score is a median *TDO2* gene expression score. In some aspects, the median *TDO2* gene expression score is determined by measuring the expression of the TDO2 gene in cancer (e.g., tumor) samples obtained from a population of subjects, and calculating the median for the distribution in the population of subjects. In some aspects, the median *TDO2* gene expression score is determined by measuring the expression of the TDO2 gene present in cancer (e.g., tumor) samples obtained from a population of subjects, and calculating the median for the distribution in the population of subjects. In some aspects, the median *TDO2* gene expression score is determined from the distribution of the expression of the TDO2 gene in cancer samples obtained from the population of subjects.

In some aspects, the reference *TDO2* gene expression score is a predetermined cut-off or threshold value.

In some aspects, the low *TDO2* gene expression score is characterized by a *TDO2* gene expression score that is lower than the average *TDO2* gene expression score of a reference sample (e.g., a sample or set of samples obtained from a subject or group of subjects). In some aspects, the low *TDO2* gene expression score is characterized by a *TDO2* gene expression score that is lower than the median *TDO2* gene expression score of a reference sample (e.g., a sample or set of samples obtained from a subject or group of subjects). In some aspects, the low *TDO2* gene expression score is characterized by a *TDO2* gene expression score that is lower than a predetermined cut-off or threshold value.

In some aspects, the TDO2 reference sample comprises a non-tumor tissue of the subject, a corresponding non-tumor tissue of the subject, or the corresponding tissue of subjects without a tumor. In some aspects, the TDO2 reference sample comprises non-tumor tissue from a population of subject, a corresponding non-tumor tissue of a population of subjects, or the corresponding tissue of a population of subjects without a tumor.

In some aspects, a low *TDO2* gene expression score is characterized by a *TDO2* gene expression score that is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 125%, at least about 150%, at least about 175%, at least about 200%, at least about 225%, at least about 250%, at least about 275%, or at least about 300% lower than an average *TDO2* gene expression score.

In some aspects, a low *TDO2* gene expression score is characterized by a *TDO2* gene expression score that is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 125%, at least about 150%, at least about 175%, at least about 200%, at least about 225%, at least about 250%, at least about 275%, or at least about 300% lower than a median *TDO2* gene expression score.

In some aspects, a low *TDO2* gene expression score is characterized by a *TDO2* gene expression score that is at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 100%, at least about 125%, at least about 150%, at least about 175%, at least about 200%, at least about 225%, at least about 250%, at least about 275%, or at least about 300% lower than a predetermined cut-off or threshold IFNγ inflammatory signature score.

In some aspects, a low *TDO2* gene expression score is characterized by a *TDO2* gene expression score that is at least about 1.25-fold, at least about 1.30-fold, at least about 1.35-fold, at least about 1.40-fold, at least about 1.45-fold, at least about 1.50-fold, at least about 1.55-fold, at least about 1.60-fold, at least about 1.65-fold, at least about 1.70-fold, at least about 1.75-fold, at least about 1.80-fold, at least about 1.85-fold, at least about 1.90-fold, at least about 1.95-fold, at least about 2-fold, at least about 2.25-fold, at least about 2.50-fold, at least about 2.75-fold, at least about 3-fold, at least about 3.25-fold, at least about 3.50-fold, at least about 3.75-fold, or at least about 400-fold lower than an average *TDO2* gene expression score, a median *TDO2* gene expression score, or a predetermined cut-off or threshold *TDO2* gene expression score.

In certain aspects, a low *TDO2* gene expression score is characterized by a *TDO2* gene expression score of less that about 5, less than about 4.9, less than about 4.8, less than about 4.7, less than about 4.6, less than about 4.5, less than about 4.4, less than about 4.3, less than about 4.2, less than about 4.1, less than about 4, less than about 3.9, less than about 3.8, less than about 3.7, less than about 3.6, less than about 3.5, less than about 3.4, less than about 3.3, less than about 3.2, less than about 3.1, less than about 3, less than about 2.9, less than about 2.8, less than about 2.7, less than about 2.6, less than about 2.5, less than about 2.4, less than about 2.3, less than about 2.2, less than about 2.1, less than about 2, less than about 1.9, less than about 1.8, less than about 1.7, less than about 1.6, less than about 1.5, less than about 1.4, less than about 1.3, less than about 1.2, less than about 1.1, less than about 1, less than about 0.9, less than about 0.8, less than about 0.7, less than about 0.6, less than about 0.5, less than about 0.4, less than about 0.3, less than about 0.2, less than about 0.1, or less than about 0, wherein the *TDO2* gene expression score is determined according to a method disclosed herein.

In certain aspects, the average *TDO2* gene expression score is about 5, about 4.9, about 4.8, about 4.7, about 4.6, about 4.5, about 4.4, about 4.3, about 4.2, about 4.1, about 4, about 3.9, about 3.8, about 3.7, about 3.6, about 3.5, about 3.4, about 3.3, about 3.2, about 3.1, about 3, about 2.9, about 2.8, about 2.7, about 2.6, about 2.5, about 2.4, about 2.3, about 2.2, about 2.1, about 2, about 1.9, about 1.8, about 1.7, about 1.6, about 1.5, about 1.4, about 1.3, about 1.2, about 1.1, about 1, about 0.9, about 0.8, about 0.7, about 0.6, about 0.5, about 0.4, about 0.3, about 0.2, about 0.1, or about 0, wherein the average *TDO2* gene expression score is determined according to a method disclosed herein.

In certain aspects, the average *TDO2* gene expression score is 5, 4.9, 4.8, 4.7, 4.6, 4.5, 4.4, 4.3, 4.2, 4.1, 4, 3.9, 3.8, 3.7, 3.6, 3.5, 3.4, 3.3, 3.2, 3.1, 3, 2.9, 2.8, 2.7, 2.6, 2.5, 2.4, 2.3, 2.2, 2.1, 2, 1.9, 1.8, 1.7, 1.6, 1.5, 1.4, 1.3, 1.2, 1.1, 1, 0.9, 0.8, 0.7, 0.6, 0.5, 0.4, 0.3, 0.2, 0.1, or 0, wherein the average *TDO2* gene expression score is determined according to a method disclosed herein.

In certain aspects, the median *TDO2* gene expression score is about 5, about 4.9, about 4.8, about 4.7, about 4.6, about 4.5, about 4.4, about 4.3, about 4.2, about 4.1, about 4, about 3.9, about 3.8, about 3.7, about 3.6, about 3.5, about 3.4, about 3.3, about 3.2, about 3.1, about 3, about 2.9, about 2.8, about 2.7, about 2.6, about 2.5, about 2.4, about 2.3, about 2.2, about 2.1, about 2, about 1.9, about 1.8, about 1.7, about 1.6, about 1.5, about 1.4, about 1.3, about 1.2, about 1.1, about 1, about 0.9, about 0.8, about 0.7, about 0.6, about 0.5, about 0.4, about 0.3, about 0.2, about 0.1, or about 0, wherein the median *TDO2* gene expression score is determined according to a method disclosed herein.

In certain aspects, the median *TDO2* gene expression score is 5, 4.9, 4.8, 4.7, 4.6, 4.5, 4.4, 4.3, 4.2, 4.1, 4, 3.9, 3.8, 3.7, 3.6, 3.5, 3.4, 3.3, 3.2, 3.1, 3, 2.9, 2.8, 2.7, 2.6, 2.5, 2.4, 2.3, 2.2, 2.1, 2, 1.9, 1.8, 1.7, 1.6, 1.5, 1.4, 1.3, 1.2, 1.1, 1, 0.9, 0.8, 0.7, 0.6, 0.5, 0.4, 0.3, 0.2, 0.1, or 0, wherein the median *TDO2* gene expression score is determined according to a method disclosed herein.

In certain aspects, the threshold *TDO2* gene expression score is about 5, about 4.9, about 4.8, about 4.7, about 4.6, about 4.5, about 4.4, about 4.3, about 4.2, about 4.1, about 4, about 3.9, about 3.8, about 3.7, about 3.6, about 3.5, about 3.4, about 3.3, about 3.2, about 3.1, about 3, about 2.9, about 2.8, about 2.7, about 2.6, about 2.5, about 2.4, about 2.3, about 2.2, about 2.1, about 2, about 1.9, about 1.8, about 1.7, about 1.6, about 1.5, about 1.4, about 1.3, about 1.2, about 1.1, about 1, about 0.9, about 0.8, about 0.7, about 0.6, about 0.5, about 0.4, about 0.3, about 0.2, about 0.1, or about 0, wherein the threshold *TDO2* gene expression score is determined according to a method disclosed herein.

In certain aspects, the threshold *TDO2* gene expression score is 5, 4.9, 4.8, 4.7, 4.6, 4.5, 4.4, 4.3, 4.2, 4.1, 4, 3.9, 3.8, 3.7, 3.6, 3.5, 3.4, 3.3, 3.2, 3.1, 3, 2.9, 2.8, 2.7, 2.6, 2.5, 2.4, 2.3, 2.2, 2.1, 2, 1.9, 1.8, 1.7, 1.6, 1.5, 1.4, 1.3, 1.2, 1.1, 1, 0.9, 0.8, 0.7, 0.6, 0.5, 0.4, 0.3, 0.2, 0.1, or 0, wherein the threshold *TDO2* gene expression score is determined according to a method disclosed herein.

### IV. Anti-PD-1 Antibodies Useful for the Disclosure

In some aspects of the present disclosure, the anti-PD-1 antagonist is an anti-PD-1 antibody or antigen binding portion thereof. Anti-PD-1 antibodies that are known in the art can be used in the presently described compositions and methods. Various human monoclonal antibodies that bind specifically to PD-1 with high affinity have been disclosed in U.S. Patent No. 8,008,449. Anti-PD-1 human antibodies disclosed in U.S. Patent No. 8,008,449 have been demonstrated to exhibit one or more of the following characteristics: (a) bind to human PD-1 with a K_{D} of 1 x 10⁻⁷ M or less, as determined by surface plasmon resonance using a Biacore biosensor system; (b) do not substantially bind to human CD28, CTLA-4 or ICOS; (c) increase T-cell proliferation in a Mixed Lymphocyte Reaction (MLR) assay; (d) increase interferon-γ production in an MLR assay; (e) increase IL-2 secretion in an MLR assay; (f) bind to human PD-1 and cynomolgus monkey PD-1; (g) inhibit the binding of PD-L1 and/or PD-L2 to PD-1; (h) stimulate antigen-specific memory responses; (i) stimulate antibody responses; and (j) inhibit tumor cell growth *in vivo.* Anti-PD-1 antibodies usable in the present disclosure include monoclonal antibodies that bind specifically to human PD-1 and exhibit at least one, in some aspects, at least five, of the preceding characteristics.

Other anti-PD-1 monoclonal antibodies have been described in, for example, U.S. Patent Nos. 6,808,710, 7,488,802, 8,168,757 and 8,354,509, US Publication No. 2016/0272708, and PCT Publication Nos. WO 2012/145493, WO 2008/156712, WO 2015/112900, WO 2012/145493, WO 2015/112800, WO 2014/206107, WO 2015/35606, WO 2015/085847, WO 2014/179664, WO 2017/020291, WO 2017/020858, WO 2016/197367, WO 2017/024515, WO 2017/025051, WO 2017/123557, WO 2016/106159, WO 2014/194302, WO 2017/040790, WO 2017/133540, WO 2017/132827, WO 2017/024465, WO 2017/025016, WO 2017/106061, WO 2017/19846, WO 2017/024465, WO 2017/025016, WO 2017/132825, and WO 2017/133540.

In some aspects, the anti-PD-1 antibody is selected from the group consisting of nivolumab (also known as OPDIVO^{®}, 5C4, BMS-936558, MDX-1106, and ONO-4538), pembrolizumab (Merck; also known as KEYTRUDA^{®}, lambrolizumab, and MK-3475; *see* WO2008/156712), PDR001 (Novartis; *see* WO 2015/112900), MEDI-0680 (AstraZeneca; also known as AMP-514; *see* WO 2012/145493), cemiplimab (Regeneron; also known as REGN-2810; *see* WO 2015/112800), JS001 (TAIZHOU JUNSHI PHARMA; also known as toripalimab; *see* Si-Yang Liu et al., J. Hematol. Oncol. 10:136 (2017)), BGB-A317 (Beigene; also known as Tislelizumab; *see* WO 2015/35606 and US 2015/0079109), INCSHR1210 (Jiangsu Hengrui Medicine; also known as SHR-1210; *see* WO 2015/085847; Si-Yang Liu et al., J. Hematol. Oncol. 10:136 (2017)), TSR-042 (Tesaro Biopharmaceutical; also known as ANB011; *see* WO2014/179664), GLS-010 (Wuxi/Harbin Gloria Pharmaceuticals; also known as WBP3055; *see* Si-Yang Liu et al., J. Hematol. Oncol. 10:136 (2017)), AM-0001 (Armo), STI-1110 (Sorrento Therapeutics; *see* WO 2014/194302), AGEN2034 (Agenus; *see* WO 2017/040790), MGA012 (Macrogenics, *see* WO 2017/19846), BCD-100 (Biocad; Kaplon et al., mAbs 10(2):183-203 (2018), and IBI308 (Innovent; *see* WO 2017/024465, WO 2017/025016, WO 2017/132825, and WO 2017/133540).

In one aspect, the anti-PD-1 antibody is nivolumab. Nivolumab is a fully human IgG4 (S228P) PD-1 immune checkpoint inhibitor antibody that selectively prevents interaction with PD-1 ligands (PD-L1 and PD-L2), thereby blocking the down-regulation of antitumor T-cell functions (U.S. Patent No. 8,008,449; Wang et al., 2014 Cancer Immunol Res. 2(9):846-56).

In another aspect, the anti-PD-1 antibody is pembrolizumab. Pembrolizumab is a humanized monoclonal IgG4 (S228P) antibody directed against human cell surface receptor PD-1 (programmed death-1 or programmed cell death-1). Pembrolizumab is described, for example, in U.S. Patent Nos. 8,354,509 and 8,900,587.

Anti-PD-1 antibodies usable in the disclosed compositions and methods also include isolated antibodies that bind specifically to human PD-1 and cross-compete for binding to human PD-1 with any anti-PD-1 antibody disclosed herein, *e.g.,* nivolumab *(see, e.g.,* U.S. Patent No. 8,008,449 and 8,779,105; WO 2013/173223). In some aspects, the anti-PD-1 antibody binds the same epitope as any of the anti-PD-1 antibodies described herein, e.g., nivolumab. The ability of antibodies to cross-compete for binding to an antigen indicates that these monoclonal antibodies bind to the same epitope region of the antigen and sterically hinder the binding of other cross-competing antibodies to that particular epitope region. These cross-competing antibodies are expected to have functional properties very similar those of the reference antibody, e.g., nivolumab, by virtue of their binding to the same epitope region of PD-1. Cross-competing antibodies can be readily identified based on their ability to cross-compete with nivolumab in standard PD-1 binding assays such as Biacore analysis, ELISA assays or flow cytometry (*see, e.g.,* WO 2013/173223).

In certain aspects, the antibodies that cross-compete for binding to human PD-1 with, or bind to the same epitope region of human PD-1 antibody, nivolumab, are monoclonal antibodies. For administration to human subjects, these cross-competing antibodies are chimeric antibodies, engineered antibodies, or humanized or human antibodies. Such chimeric, engineered, humanized or human monoclonal antibodies can be prepared and isolated by methods well known in the art.

Anti-PD-1 antibodies usable in the compositions and methods of the disclosed disclosure also include antigen-binding portions of the above antibodies. It has been amply demonstrated that the antigen-binding function of an antibody can be performed by fragments of a full-length antibody.

Anti-PD-1 antibodies suitable for use in the disclosed compositions and methods are antibodies that bind to PD-1 with high specificity and affinity, block the binding of PD-L1 and or PD-L2, and inhibit the immunosuppressive effect of the PD-1 signaling pathway. In any of the compositions or methods disclosed herein, an anti-PD-1 "antibody" includes an antigen-binding portion or fragment that binds to the PD-1 receptor and exhibits the functional properties similar to those of whole antibodies in inhibiting ligand binding and up-regulating the immune system. In certain aspects, the anti-PD-1 antibody or antigen-binding portion thereof cross-competes with nivolumab for binding to human PD-1.

In some aspects, the anti-PD-1 antibody is administered at a dose ranging from 0.1 mg/kg to 20.0 mg/kg body weight once every 2, 3, 4, 5, 6, 7, or 8 weeks, e.g., 0.1 mg/kg to 10.0 mg/kg body weight once every 2, 3, or 4 weeks. In other aspects, the anti-PD-1 antibody is administered at a dose of about 2 mg/kg, about 3 mg/kg, about 4 mg/kg, about 5 mg/kg, about 6 mg/kg, about 7 mg/kg, about 8 mg/kg, about 9 mg/kg, or 10 mg/kg body weight once every 2 weeks. In other aspects, the anti-PD-1 antibody is administered at a dose of about 2 mg/kg, about 3 mg/kg, about 4 mg/kg, about 5 mg/kg, about 6 mg/kg, about 7 mg/kg, about 8 mg/kg, about 9 mg/kg, or 10 mg/kg body weight once every 3 weeks. In one aspect, the anti-PD-1 antibody is administered at a dose of about 5 mg/kg body weight about once every 3 weeks. In another aspect, the anti-PD-1 antibody, e.g., nivolumab, is administered at a dose of about 3 mg/kg body weight about once every 2 weeks. In other aspects, the anti-PD-1 antibody, e.g., pembrolizumab, is administered at a dose of about 2 mg/kg body weight about once every 3 weeks.

The anti-PD-1 antibody useful for the present disclosure can be administered as a flat dose. In some aspects, the anti-PD-1 antibody is administered at a flat dose of from about 100 to about 1000 mg, from about 100 mg to about 900 mg, from about 100 mg to about 800 mg, from about 100 mg to about 700 mg, from about 100 mg to about 600 mg, from about 100 mg to about 500 mg, from about 200 mg to about 1000 mg, from about 200 mg to about 900 mg, from about 200 mg to about 800 mg, from about 200 mg to about 700 mg, from about 200 mg to about 600 mg, from about 200 mg to about 500 mg, from about 200 mg to about 480 mg, or from about 240 mg to about 480 mg, In one aspect, the anti-PD-1 antibody is administered as a flat dose of at least about 200 mg, at least about 220 mg, at least about 240 mg, at least about 260 mg, at least about 280 mg, at least about 300 mg, at least about 320 mg, at least about 340 mg, at least about 360 mg, at least about 380 mg, at least about 400 mg, at least about 420 mg, at least about 440 mg, at least about 460 mg, at least about 480 mg, at least about 500 mg, at least about 520 mg, at least about 540 mg, at least about 550 mg, at least about 560 mg, at least about 580 mg, at least about 600 mg, at least about 620 mg, at least about 640 mg, at least about 660 mg, at least about 680 mg, at least about 700 mg, or at least about 720 mg at a dosing interval of about 1, 2, 3, 4, 5, 6, 7, 8, 9, or 10 weeks. In another aspect, the anti-PD-1 antibody is administered as a flat dose of about 200 mg to about 800 mg, about 200 mg to about 700 mg, about 200 mg to about 600 mg, about 200 mg to about 500 mg, at a dosing interval of about 1, 2, 3, or 4 weeks.

In some aspects, the anti-PD-1 antibody is administered as a flat dose of about 200 mg at about once every 3 weeks. In other aspects, the anti-PD-1 antibody is administered as a flat dose of about 200 mg at about once every 2 weeks. In other aspects, the anti-PD-1 antibody is administered as a flat dose of about 240 mg at about once every 2 weeks. In certain aspects, the anti-PD-1 antibody is administered as a flat dose of about 480 mg at about once every 4 weeks.

In some aspects, nivolumab is administered at a flat dose of about 240 mg once about every 2 weeks. In some aspects, nivolumab is administered at a flat dose of about 240 mg once about every 3 weeks. In some aspects, nivolumab is administered at a flat dose of about 360 mg once about every 3 weeks. In some aspects, nivolumab is administered at a flat dose of about 480 mg once about every 4 weeks.

In some aspects, pembrolizumab is administered at a flat dose of about 200 mg once about every 2 weeks. In some aspects, pembrolizumab is administered at a flat dose of about 200 mg once about every 3 weeks. In some aspects, pembrolizumab is administered at a flat dose of about 400 mg once about every 4 weeks.

### V. Anti-PD-L1 Antibodies Useful for the Disclosure

In certain aspects of the present disclosure, the anti-PD-1 antagonist is an anti-PD-L1 antibody or an antigen binding portion thereof. Anti-PD-L1 antibodies that are known in the art can be used in the compositions and methods of the present disclosure. Examples of anti-PD-L1 antibodies useful in the compositions and methods of the present disclosure include the antibodies disclosed in US Patent No. 9,580,507. Anti-PD-L1 human monoclonal antibodies disclosed in U.S. Patent No. 9,580,507 have been demonstrated to exhibit one or more of the following characteristics: (a) bind to human PD-L1 with a K_{D} of 1 x 10⁻⁷ M or less, as determined by surface plasmon resonance using a Biacore biosensor system; (b) increase T-cell proliferation in a Mixed Lymphocyte Reaction (MLR) assay; (c) increase interferon-γ production in an MLR assay; (d) increase IL-2 secretion in an MLR assay; (e) stimulate antibody responses; and (f) reverse the effect of T regulatory cells on T cell effector cells and/or dendritic cells. Anti-PD-L1 antibodies usable in the present disclosure include monoclonal antibodies that bind specifically to human PD-L1 and exhibit at least one, in some aspects, at least five, of the preceding characteristics.

In certain aspects, the anti-PD-L1 antibody is selected from the group consisting of BMS-936559 (also known as 12A4, MDX-1105; *see, e.g.,* U.S. Patent No. 7,943,743 and WO 2013/173223), atezolizumab (Roche; also known as TECENTRIQ^{®}; MPDL3280A, RG7446; *see* US 8,217,149; *see, also,* Herbst et al. (2013) J Clin Oncol 31(suppl):3000), durvalumab (AstraZeneca; also known as IMFINZI^{™}, MEDI-4736; *see* WO 2011/066389), avelumab (Pfizer; also known as BAVENCIO^{®}, MSB-0010718C; *see* WO 2013/079174), STI-1014 (Sorrento; *see* WO2013/181634), CX-072 (Cytomx; *see* WO2016/149201), KN035 (3D Med/Alphamab; *see* Zhang et al., Cell Discov. 7:3 (March 2017), LY3300054 (Eli Lilly Co.; *see, e.g.,* WO 2017/034916), BGB-A333 (BeiGene; *see* Desai et al., JCO 36 (15suppl*):* TPS3113 (2018)), and CK-301 (Checkpoint Therapeutics; *see* Gorelik et al., AACR: Abstract 4606 (Apr 2016)).

In certain aspects, the PD-L1 antibody is atezolizumab (TECENTRIQ^{®}). Atezolizumab is a fully humanized IgG1 monoclonal anti-PD-L1 antibody. In certain aspects, the PD-L1 antibody is durvalumab (IMFINZI^{™}). Durvalumab is a human IgG1 kappa monoclonal anti-PD-L1 antibody. In certain aspects, the PD-L1 antibody is avelumab (BAVENCIO^{®}). Avelumab is a human IgG1 lambda monoclonal anti-PD-L1 antibody.

Anti-PD-L1 antibodies usable in the disclosed compositions and methods also include isolated antibodies that bind specifically to human PD-L1 and cross-compete for binding to human PD-L1 with any anti-PD-L1 antibody disclosed herein, e.g., atezolizumab, durvalumab, and/or avelumab. In some aspects, the anti-PD-L1 antibody binds the same epitope as any of the anti-PD-L1 antibodies described herein, e.g., atezolizumab, durvalumab, and/or avelumab. The ability of antibodies to cross-compete for binding to an antigen indicates that these antibodies bind to the same epitope region of the antigen and sterically hinder the binding of other cross-competing antibodies to that particular epitope region. These cross-competing antibodies are expected to have functional properties very similar those of the reference antibody, e.g., atezolizumab and/or avelumab, by virtue of their binding to the same epitope region of PD-L1. Cross-competing antibodies can be readily identified based on their ability to cross-compete with atezolizumab and/or avelumab in standard PD-L1 binding assays such as Biacore analysis, ELISA assays or flow cytometry (*see, e.g.,* WO 2013/173223).

In certain aspects, the antibodies that cross-compete for binding to human PD-L1 with, or bind to the same epitope region of human PD-L1 antibody as, atezolizumab, durvalumab, and/or avelumab, are monoclonal antibodies. For administration to human subjects, these cross-competing antibodies are chimeric antibodies, engineered antibodies, or humanized or human antibodies. Such chimeric, engineered, humanized or human monoclonal antibodies can be prepared and isolated by methods well known in the art.

Anti-PD-L1 antibodies usable in the compositions and methods of the disclosed disclosure also include antigen-binding portions of the above antibodies. It has been amply demonstrated that the antigen-binding function of an antibody can be performed by fragments of a full-length antibody.

Anti-PD-L1 antibodies suitable for use in the disclosed compositions and methods are antibodies that bind to PD-L1 with high specificity and affinity, block the binding of PD-1, and inhibit the immunosuppressive effect of the PD-1 signaling pathway. In any of the compositions or methods disclosed herein, an anti-PD-L1 "antibody" includes an antigen-binding portion or fragment that binds to PD-L1 and exhibits the functional properties similar to those of whole antibodies in inhibiting receptor binding and up-regulating the immune system. In certain aspects, the anti-PD-L1 antibody or antigen-binding portion thereof cross-competes with atezolizumab, durvalumab, and/or avelumab for binding to human PD-L1.

The anti-PD-L1 antibody useful for the present disclosure can be any PD-L1 antibody that specifically binds to PD-L1, e.g., antibodies that cross-compete with durvalumab, avelumab, or atezolizumab for binding to human PD-1, *e.g.,* an antibody that binds to the same epitope as durvalumab, avelumab, or atezolizumab. In a particular aspect, the anti-PD-L1 antibody is durvalumab. In other aspects, the anti-PD-L1 antibody is avelumab. In some aspects, the anti-PD-L1 antibody is atezolizumab.

In some aspects, the anti-PD-L1 antibody is administered at a dose ranging from about 0.1 mg/kg to about 20.0 mg/kg body weight, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg, about 16 mg/kg, about 17 mg/kg, about 18 mg/kg, about 19 mg/kg, or about 20 mg/kg, about once every 2, 3, 4, 5, 6, 7, or 8 weeks.

In some aspects, the anti-PD-L1 antibody is administered at a dose of about 15 mg/kg body weight at about once every 3 weeks. In other aspects, the anti-PD-L1 antibody is administered at a dose of about 10 mg/kg body weight at about once every 2 weeks.

In other aspects, the anti-PD-L1 antibody useful for the present disclosure is a flat dose. In some aspects, the anti-PD-L1 antibody is administered as a flat dose of from about 200 mg to about 1600 mg, about 200 mg to about 1500 mg, about 200 mg to about 1400 mg, about 200 mg to about 1300 mg, about 200 mg to about 1200 mg, about 200 mg to about 1100 mg, about 200 mg to about 1000 mg, about 200 mg to about 900 mg, about 200 mg to about 800 mg, about 200 mg to about 700 mg, about 200 mg to about 600 mg, about 700 mg to about 1300 mg, about 800 mg to about 1200 mg, about 700 mg to about 900 mg, or about 1100 mg to about 1300 mg. In some aspects, the anti-PD-L1 antibody is administered as a flat dose of at least about 240 mg, at least about 300 mg, at least about 320 mg, at least about 400 mg, at least about 480 mg, at least about 500 mg, at least about 560 mg, at least about 600 mg, at least about 640 mg, at least about 700 mg, at least 720 mg, at least about 800 mg, at least about 840 mg, at least about 880 mg, at least about 900 mg, at least 960 mg, at least about 1000 mg, at least about 1040 mg, at least about 1100 mg, at least about 1120 mg, at least about 1200 mg, at least about 1280 mg, at least about 1300 mg, at least about 1360 mg, or at least about 1400 mg, at a dosing interval of about 1, 2, 3, or 4 weeks. In some aspects, the anti-PD-L1 antibody is administered as a flat dose of about 1200 mg at about once every 3 weeks. In other aspects, the anti-PD-L1 antibody is administered as a flat dose of about 800 mg at about once every 2 weeks. In other aspects, the anti-PD-L1 antibody is administered as a flat dose of about 840 mg at about once every 2 weeks.

In some aspects, atezolizumab is administered as a flat dose of about 1200 mg once about every 3 weeks. In some aspects, atezolizumab is administered as a flat dose of about 800 mg once about every 2 weeks. In some aspects, atezolizumab is administered as a flat dose of about 840 mg once about every 2 weeks.

In some aspects, avelumab is administered as a flat dose of about 800 mg once about every 2 weeks.

In some aspects, durvalumab is administered at a dose of about 10 mg/kg once about every 2 weeks. In some aspects, durvalumab is administered as a flat dose of about 800 mg/kg once about every 2 weeks. In some aspects, durvalumab is administered as a flat dose of about 1200 mg/kg once about every 3 weeks.

### VI. Cancers

In some aspects, the cancer is a tumor, i.e., a solid cancer. In other aspects, the cancer is a tumor of epithelial origin, i.e., a carcinoma. In some aspects, the tumor is derived from a cancer selected from the group consisting of bladder cancer, cervical cancer, lung cancer, pancreatic cancer, kidney cancer, head and neck cancer, hepatocellular carcinoma, glioblastoma, melanoma, or endometrial cancer. In some aspects, the tumor is not melanoma. In the present invention, the cancer is a bladder cancer or a squamous cell carcinoma of the head and neck (SCCHN).

In certain aspects, the tumor is derived from cancer having a high IFNy inflammatory signature score. In certain aspects, the tumor is derived from a bladder, wherein the tumor has a high IFNy inflammatory signature score. In certain aspects, the tumor is derived from a cervical cancer, wherein the tumor has a high IFNy inflammatory signature score. In certain aspects, the tumor is derived from a lung cancer, wherein the tumor has a high IFNγ inflammatory signature score. In certain aspects, the tumor is derived from a pancreatic cancer, wherein the tumor has a high IFNy inflammatory signature score. In some aspects, the tumor is derived from a kidney cancer, wherein the tumor has a high IFNy inflammatory signature score. In some aspects, the tumor is derived from a head and neck cancer, wherein the tumor has a high IFNy inflammatory signature score. In some aspects, the tumor is derived from a hepatocellular carcinoma, wherein the tumor has a high IFNy inflammatory signature score. In some aspects, the tumor is derived from a glioblastoma, wherein the tumor has a high IFNy inflammatory signature score. In some aspects, the tumor is derived from a melanoma, wherein the tumor has a high IFNy inflammatory signature score. In some aspects, the tumor is derived from an endometrial cancer, wherein the tumor has a high IFNy inflammatory signature score.

In certain aspects, the tumor is derived from cancer having a low TDO2 gene expression score. In certain aspects, the tumor is derived from a bladder, wherein the tumor has a low TDO2 gene expression score. In certain aspects, the tumor is derived from a cervical cancer, wherein the tumor has a low TDO2 gene expression score. In certain aspects, the tumor is derived from a lung cancer, wherein the tumor has a low TDO2 gene expression score. In certain aspects, the tumor is derived from a pancreatic cancer, wherein the tumor has a low TDO2 gene expression score. In some aspects, the tumor is derived from a kidney cancer, wherein the tumor has a low TDO2 gene expression score. In some aspects, the tumor is derived from a head and neck cancer, wherein the tumor has a low TDO2 gene expression score. In some aspects, the tumor is derived from a hepatocellular carcinoma, wherein the tumor has a low TDO2 gene expression score. In some aspects, the tumor is derived from a glioblastoma, wherein the tumor has a low TDO2 gene expression score. In some aspects, the tumor is derived from a melanoma, wherein the tumor has a low TDO2 gene expression score. In some aspects, the tumor is derived from an endometrial cancer, wherein the tumor has a low TDO2 gene expression score.

In certain aspects, the tumor is derived from cancer having a high IFNy inflammatory signature score and a low TDO2 gene expression score. In certain aspects, the tumor is derived from a bladder, wherein the tumor has a high IFNy inflammatory signature score and a low TDO2 gene expression score. In certain aspects, the tumor is derived from a cervical cancer, wherein the tumor has a high IFNy inflammatory signature score and a low TDO2 gene expression score. In certain aspects, the tumor is derived from a lung cancer, wherein the tumor has a high IFNy inflammatory signature score and a low TDO2 gene expression score. In certain aspects, the tumor is derived from a pancreatic cancer, wherein the tumor has a high IFNy inflammatory signature score and a low TDO2 gene expression score. In some aspects, the tumor is derived from a kidney cancer, wherein the tumor has a high IFNγ inflammatory signature score and a low TDO2 gene expression score. In some aspects, the tumor is derived from a head and neck cancer, wherein the tumor has a high IFNy inflammatory signature score and a low TDO2 gene expression score. In some aspects, the tumor is derived from a hepatocellular carcinoma, wherein the tumor has a high IFNy inflammatory signature score and a low TDO2 gene expression score. In some aspects, the tumor is derived from a glioblastoma, wherein the tumor has a high IFNy inflammatory signature score and a low TDO2 gene expression score. In some aspects, the tumor is derived from a melanoma, wherein the tumor has a high IFNy inflammatory signature score and a low TDO2 gene expression score. In some aspects, the tumor is derived from an endometrial cancer, wherein the tumor has a high IFNγ inflammatory signature score and a low TDO2 gene expression score.

In some aspects, the lung cancer is non-small cell lung cancer (NSCLC). In some aspects, the kidney cancer is renal cell carcinoma (RCC). In some aspects, the head and neck cancer is squamous cell carcinoma of the head and neck (SCCHN). In some aspects, the glioblastoma is glioblastoma multiforme (GBM).

In some aspects, the cancer is a hematological cancer. In some aspects, the hematological cancer is lymphoma. In some aspects, the lymphoma is diffuse large B-cell lymphoma (DLBCL). In some aspects, the hematological cancer (e.g., lymphoma) has a high IFNy inflammatory signature score. In some aspects, the hematological cancer (e.g., lymphoma) has a low TDO2 gene expression score. In some aspects, the hematological cancer (e.g., lymphoma) has a high IFNy inflammatory signature score and a low TDO2 gene expression score.

In certain aspects, the subject has received one, two, three, four, five or more prior cancer treatments. In other aspects, the subject is treatment-naive. In some aspects, the subject has progressed on other cancer treatments. In certain aspects, the prior cancer treatment comprised an immunotherapy. In other aspects, the prior cancer treatment comprised a chemotherapy. In some aspects, the tumor has reoccurred. In some aspects, the tumor is metastatic. In other aspects, the tumor is not metastatic. In some aspects, the tumor is locally advanced.

In some aspects, the subject has received a prior therapy to treat the tumor and the tumor is relapsed or refractory. In certain aspects, the at least one prior therapy comprises a standard-of-care therapy. In some aspects, the at least one prior therapy comprises a surgery, a radiation therapy, a chemotherapy, an immunotherapy, or any combination thereof. In some aspects, the at least one prior therapy comprises a chemotherapy. In some aspects, the subject has received a prior immuno-oncology (I-O) therapy to treat the tumor and the tumor is relapsed or refractory. In some aspects, the subject has received more than one prior therapy to treat the tumor and the subject is relapsed or refractory. In other aspects, the subject has received either an anti-PD-1 or anti-PD-L1 antibody therapy.

In some aspects, the previous line of therapy comprises a chemotherapy. In some aspects, the previous line of therapy comprises administering an anticancer agent selected from the group consisting of a platinum agent (e.g., cisplatin, carboplatin), a taxanes agent (e.g., paclitaxel, albumin-bound paclitaxel, docetaxel), vinorelbine, vinblastine, etoposide, pemetrexed, gemcitabine, bevacizumab (AVASTIN^{®}), erlotinib (TARCEVA^{®}), crizotinib (XALKORI^{®}), cetuximab (ERBITUX^{®}), and any combination thereof.

In some aspects, the subject has experienced disease progression after the at least one prior therapy. In certain aspects, the subject has received at least two prior therapies, at least three prior therapies, at least four prior therapies, or at least five prior therapies. In certain aspects, the subject has received at least two prior therapies. In one aspect, the subject has experienced disease progression after the at least two prior therapies. In certain aspects, the at least two prior therapies comprises a first prior therapy and a second prior therapy, wherein the subject has experienced disease progression after the first prior therapy and/or the second prior therapy, and wherein the first prior therapy comprises a surgery, a radiation therapy, a chemotherapy, an immunotherapy, or any combination thereof; and wherein the second prior therapy comprises a surgery, a radiation therapy, a chemotherapy, an immunotherapy, or any combination thereof. In some aspects, the first prior therapy comprises a platinum-based doublet chemotherapy, and the second prior therapy comprises a single-agent chemotherapy. In certain aspects, the single-agent chemotherapy comprises docetaxel.

### VII. Pharmaceutical Compositions and Dosages

Therapeutic agents of the present disclosure, e.g., anti-PD-1 antagonists (e.g., nivolumab) and IDO1 inhibitors (e.g., linrodostat mesylate) can be constituted in a composition, e.g., a pharmaceutical composition containing an anti-PD-1 antagonist (e.g., nivolumab) and/or an IDO1 inhibitor (e.g., linrodostat mesylate) and pharmaceutically acceptable carrier. As used herein, a "pharmaceutically acceptable carrier" includes any and all solvents, dispersion media, coatings, antibacterial and antifungal agents, isotonic and absorption delaying agents, and the like that are physiologically compatible. In some aspects, the carrier for a composition containing an antibody, e.g., an anti-PD-1 antibody such as nivolumab, is suitable for intravenous, intramuscular, subcutaneous, parenteral, spinal or epidermal administration (e.g., by injection or infusion), whereas the carrier for a composition containing a IDO1 inhibitor, e.g., linrodostat mesylate, is suitable for non-parenteral, e.g., oral, administration.

In some aspects, the subcutaneous injection is based on Halozyme Therapeutics' ENHANZE^{®} drug-delivery technology *(see* U.S. Patent No. 7,767,429). ENHANZE^{®} uses a co-formulation of an antibody with recombinant human hyaluronidase enzyme (rHuPH20), which removes traditional limitations on the volume of biologics and drugs that can be delivered subcutaneously due to the extracellular matrix *(see* U.S. Patent No. 7,767,429).

A pharmaceutical composition of the disclosure can include one or more pharmaceutically acceptable salts, anti-oxidant, aqueous and non-aqueous carriers, and/or adjuvants such as preservatives, wetting agents, emulsifying agents and dispersing agents. Therefore, in some aspects, the pharmaceutical composition for the present disclosure can further comprise recombinant human hyaluronidase enzyme, e.g., rHuPH20.

Although higher nivolumab monotherapy dosing up to 10 mg/kg every two weeks has been achieved without reaching the maximum tolerated does (MTD), the significant toxicities reported in other trials of checkpoint inhibitors plus anti-angiogenic therapy *(see, e.g.,* Johnson *et al.,* 2013; Rini *et al.,* 2011) support the selection of a nivolumab dose lower than 10 mg/kg.

Treatment is continued as long as clinical benefit is observed or until unacceptable toxicity or disease progression occurs. Nevertheless, in certain aspects, the dosages of the anti-PD-1 antagonist (e.g., anti-PD-1 antibody or anti-PD-L1 antibody), and/or the IDO1 inhibitor (e.g., linrodostat mesylate) administered are significantly lower than the approved dosage, *i.e.,* a subtherapeutic dosage, of the agent.

The anti-PD-1 antagonist (e.g., anti-PD-1 antibody or anti-PD-L1 antibody) can be administered at the dosage that has been shown to produce the highest efficacy as monotherapy in clinical trials, e.g., about 3 mg/kg of nivolumab administered once every three weeks (Topalian *et al.,* 2012a; Topalian *et al.,* 2012), or at a significantly lower dose, *i.e.,* at a subtherapeutic dose. In some aspects, the IDO1 inhibitor (e.g., linrodostat) is administered at a subtherapeutic dose.

Dosage and frequency vary depending on the half-life of the antibody (e.g., nivolumab) and IDO1 inhibitor (e.g., linrodostat mesylate) in the subject. In general, human antibodies show the longest half-life, followed by humanized antibodies, chimeric antibodies, and nonhuman antibodies. The dosage and frequency of administration can vary depending on whether the treatment is prophylactic or therapeutic. In prophylactic applications, a relatively low dosage is typically administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

Actual dosage levels of the active ingredients in the pharmaceutical compositions of the present disclosure can be varied so as to obtain amounts of the active ingredients which are effective to achieve the desired therapeutic response for a particular patient, composition, and mode of administration, without being unduly toxic to the patient.

The selected dosage levels will depend upon a variety of pharmacokinetic factors including the activity of the particular compositions of the present disclosure employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A composition of the present disclosure can be administered via one or more routes of administration using one or more of a variety of methods well known in the art. As will be appreciated by the skilled artisan, the route and/or mode of administration will vary depending upon the desired results.

### VIII. Kits, products of manufacture, and gene panels

Also within the scope of the present disclosure are kits and products of manufacture comprising (a) a dosage of an anti-PD-1 antagonist, e.g., an anti-PD-1 antibody (e.g., nivolumab) or an anti-PD-L1 antibody, and (b) a dosage of an IDO1 inhibitor (e.g., linrodostat) for therapeutic uses, e.g., the treatment of cancer. Kits typically include a label indicating the intended use of the contents of the kit and instructions for use, e.g., to administer the anti-PD-1 antagonist, e.g., an anti-PD-1 antibody (e.g., nivolumab) or an anti-PD-L1 antibody, and the IDO1 inhibitor (e.g., linrodostat) to a subject afflicted with a cancer, e.g., a tumor, if the subject is identified as having (a) a high *IFNy* inflammatory signature score and (b) a low tryptophan 2,3-dioxygenase 2 (*TDO2*) gene expression score. The term label includes any writing, or recorded material supplied on or with the kit or product of manufacture, or which otherwise accompanies the kit or product of manufacture. The term label also encompasses instructions that are distributed electronically and/or are accessible electronically, e.g., via a web server.

In one aspect, the present disclosure provides a kit or article of manufacture for treating a subject afflicted with a cancer, the kit comprising (a) a dosage of an anti-PD-1 antagonist (e.g., nivolumab), (b) a dosage of an IDO1 inhibitor (e.g., linrodostat), and (c) instructions for using the anti-PD-1 or anti-PD-L1 antibody and IDO1 inhibitor according to the methods disclosed herein. In one aspect, the present disclosure provides a kit or article of manufacture for treating a subject afflicted with a cancer, the kit comprising (a) a dosage of an anti-PD-1 antagonist (e.g., nivolumab), and (b) instructions for using the anti-PD-1 or anti-PD-L1 antibody according to the methods disclosed herein. In one aspect, the present disclosure provides a kit or article of manufacture for treating a subject afflicted with a cancer, the kit comprising (a) a dosage of an IDO1 inhibitor (e.g., linrodostat), and (b) instructions for using the IDO1 inhibitor according to the methods disclosed herein.

One skilled in the art will readily recognize that a kit of the present disclosure can contain, e.g.,
(a) an anti-PD-1 antagonist, e.g., an anti-PD-1 antibody (e.g., nivolumab) or an anti-PD-L1 antibody, in one or multiple vials, and in some aspects accompanied by additional vial(s) comprising one or more solvents;
(b) an IDO1 inhibitor (e.g., linrodostat) in one or multiple vials, and in some aspects additional vial(s) comprising one or more solvents;
(c) an assay to determine an *IFNγ*inflammatory signature score;
(d) an assay to determine a tryptophan 2,3-dioxygenase 2 (*TDO2*) gene expression score;
(e) instructions to conduct and quantitate the *IFNγ*inflammatory signature score assay and/or the *TDO2* gene expression score assay;
(f) instructions to administer the anti-PD-1 antagonist and/or the IDO1 inhibitor;
(g) instructions to determine whether to administer the anti-PD-1 antagonist and/or the IDO1 inhibitor to the subject according to the methods disclosed herein (e.g., if the subject identified as having (i) a high IFNyinflammatory signature score and (ii) a low tryptophan 2,3-dioxygenase 2 (*TDO2*) gene expression score);
(h) any combination thereof.

One skilled in the art will also readily recognize that the kit or product of manufacture can comprise any of the components disclosed above or combinations thereof, and that they can be readily incorporated into one of the established kit formats which are well known in the art or as part of companion diagnostics.

Accordingly, this disclosure provides a kit or product of manufacture for treating a subject afflicted with a cancer, e.g., a tumor, the kit or product of manufacture comprising: (a) a dosage ranging from 0.1 to 10 mg/kg body weight of an anti-PD-1 antibody (e.g., nivolumab) or a dosage ranging from 0.1 to 20 mg/kg body weight of an anti-PD-L1 antibody; and (b) instructions for using the anti-PD-1 antibody (e.g., nivolumab) or the anti-PD-L1 antibody according to the methods disclosed herein.

This disclosure further provides a kit or products of manufacture for treating a subject afflicted with a cancer, e.g., a tumor, the kit comprising: (a) a dosage ranging from about 4 mg to about 500 mg of an anti-PD-1 antibody (e.g., nivolumab) or a dosage ranging from about 4 mg to about 2000 mg of an anti-PD-L1 antibody; and (b) instructions for using the anti-PD-1 antibody (e.g., nivolumab) or the anti-PD-L1 antibody according to the methods disclosed herein.

In some aspects, this disclosure provides a kit or product of manufacture for treating a subject afflicted with a cancer, e.g., a tumor, the kit or product of manufacture comprising: (a) a dosage ranging from 200 mg to 800 mg of an anti-PD-1 antibody (e.g., nivolumab) or a dosage ranging from 200 mg to 1800 mg of an anti-PD-L1 antibody; and (b) instructions for using the anti-PD-1 antibody (e.g., nivolumab) or the anti-PD-L1 antibody according to the methods disclosed herein.

In certain aspects for treating human patients afflicted with a cancer, e.g., a tumor, the kit or product of manufacture comprises an anti-human PD-1 antibody disclosed herein, e.g., nivolumab or pembrolizumab, or an antigen-binding portion thereof. In certain aspects for treating human patients afflicted with a cancer, e.g., a tumor, the kit or product of manufacture comprises an anti-human PD-L1 antibody disclosed herein, e.g., atezolizumab, durvalumab, or avelumab, or an antigen-binding portion thereof.

In some aspects, the kit or product of manufacture further includes an IDO1 inhibitor (e.g., linrodostat) and instructions to administer (a) the anti-PD-1 antibody (e.g., nivolumab) or the anti-PD-L1 antibody and (b) the IDO1 inhibitor (e.g., linrodostat) to a subject identified as having (a) a high *IFNγ* inflammatory signature score and (b) a low tryptophan 2,3-dioxygenase 2 (*TDO2*) gene expression score, according to the methods disclosed herein.

In some aspects, the kit further includes an inflammatory gene panel assay disclosed herein, e.g., a gene panel assay to quantitate an *IFNy* inflammatory signature score and/or a gene assay to quantitate a tryptophan 2,3-dioxygenase 2 (*TDO2*) gene expression score. In some aspects, the kit or product of manufacture further includes instructions to administer (a) the anti-PD-1 antibody (e.g., nivolumab) or the anti-PD-L1 antibody and (b) the IDO1 inhibitor (e.g., linrodostat) to a subject identified as having (a) a high *IFNy* inflammatory signature score and (b) a low tryptophan 2,3-dioxygenase 2 (*TDO2*) gene expression score, according to the methods disclosed herein.

In some aspects, the present disclosure provides a gene panel comprising at least the *IFNy* and *TDO2* genes, for use in
(i) identifying a subject suitable for therapy with a combination comprising an anti-PD-1 antagonist and an IDO1 inhibitor;
(ii) determining the prognosis of a subject undergoing therapy with a combination comprising an anti-PD-1 antagonist and an IDO1 inhibitor;
(iii) initiating, suspending, or modifying the administration of a combination comprising an anti-PD-1 antagonist and an IDO1 inhibitor; or,
(iv) a combination thereof.

In some aspects, the gene panel comprises
*(i) IFNy, CXCL10, CXCL9, HLA-DRA, IDO1, STAT1,* and *TDO2;*
(ii) *IFNγ, CXCL10, CXCL9, HLA-DRA, IDO1, STAT1, CCR5, CXCL11, GZMA, PRF1,* and *TDO2;*
(iii) *CXCR6, TIGIT, CD274 (PD-L1), PDCD1LG2 (PD-L2), LAG3, NKG7, PSMB10, CMKLR1, CD8A, IDO1, CCL5, CXCL9, HLA.DQA1, CD276, HLA.DRB1, STAT1, HLA.E,* and *TDO2;* or,
(iv) *IFNγ, IR2RG, CXCR6, CD3D, CD2, ITGAL, TAGAP, CIITA, HLA-DRA, PTPRC, CXCL9, CCL5, NKG7, GZMA, PRF1, CCR5, CD3E, GZMK, HLA-E, GZMB, PDCD1, SLAMF6, CXCL13, CXCL10, IDO1, LAG3, STAT1, CXCL11,* and *TDO2.*

In some aspects, the present disclosure provides a set of reagents (e.g., antibodies) or array (e.g., an oligonucleotide array) for detection and/or quantification of the genes in the gene panel and/or their expression products (e.g., mRNA or protein), and instructions to use the reagents or array as predictive biomarkers according to the methods of the present disclosure.

The following examples are offered by way of illustration.

### EXAMPLES

### Example 1

### Biomarker Samples and Assessments

A clinical trial was developed to examine the effect of combination therapy with nivolumab and the IDO1 inhibitor linrodostat in patients with advanced tumors (Clinical Trial Identifier NCT02658890).

Part 1 of the clinical trial involved a dose escalation study in select previously treated advanced tumors. Prior treatment immune checkpoint inhibitors and therapy targeting T-cell co-stimulation was permitted. Patients were treated with 240mg of nivolumab (intravenous, once every two weeks) and linrodostat was given orally, every day, with varying dosages (25mg, 50mg, 100mg, 200mg, 400mg, 600mg, or 800mg). Part 2 of the clinical trial involved a dose expansion study. These patients were treated with either nivolumab monotherapy (240mg, intravenous, once every two weeks) or nivolumab and linrodostat combination therapy (480mg nivolumab, intravenous, once every four weeks and 100mg or 200mg linrodostat, orally, every day).

Serum and tumor samples were taken from patients participating in the dose expansion phase of the study (part 2). Tumor and serum samples were obtained from patients with solid tumors (bladder, cervical, melanoma, lung, pancreatic, renal cell, head and neck) or lymphoma at baseline (cycle 1, day 1; C1D1) and during treatment (cycle 1, day 15; C1D15). These samples were subsequently used in quantification of KYN and tryptophan levels by liquid chromatography-mass spectroscopy. Solid tumor samples at baseline were also obtained and formalin-fixed, paraffin-embedded. These samples were used to quantify IFN-γ signature genes, IDO1, and TDO2 expression levels by RNA-sequencing. **FIG. 1A** and **FIG. 1B** show the percentage of each tumor type obtained out of the total patient population.

### Example 2

### Association of IFNγ Signature with Clinical Response

IFNγ gene signatures that are predictive of the response to PD-1 checkpoint blockade in melanoma have previously been established (Ayers et al. J Clin Invest. 2017;127(8):2930-2940). In this example, the association between the expression of IFNγ signature genes and the clinical response to nivolumab and linrodostat combination therapy was examined. The expression of IFNγ signature genes was quantified by RNA sequencing from formalin-fixed, paraffin embedded solid tumor samples. Clinical response was determined by separating the tumors into five groups: non evaluable (NE), progressive disease (PD), stable disease (SD), partial response (PR), and complete response (CR). Expression of IFN-γ signature genes was associated with an improved clinical response from patients treated with nivolumab and linrodostat **(****FIG. 2A** and **FIG. 2B****).**

### Example 3

### Association of IFN-γ Signature with Progression-Free Survival and Overall Survival

The association of expression of IFNγ signature genes and survival of patients treated with nivolumab and linrodostat was evaluated. Patients were separated into three groups based on IFNγ signature gene expression: low, medium, or high expression. Patient survival was monitored over 200 day increments up to 600 days. High IFNγ signature was associated with improved progression free survival **(****FIG. 3A** and **TABLE 2)** and overall survival **(****FIG. 3B** and **TABLE 3)** in immuno-oncology naive patients treated with nivolumab and linrodostat.

**TABLE 2. Number of surviving patients based on progression-free survival**

| | **Baseline (Day 0 of Treatment)** | **Day 200 of Treatment** | **Day 400 of Treatment** | **Day 600 of Treatment** |
|---|---|---|---|---|
| **IFNγ High** | 27 | 15 | 3 | 1 |
| **IFNγ Medium** | 26 | 5 | 3 | 1 |
| **IFNy Low** | 26 | 2 | 0 | 0 |

**TABLE 3. Number of surviving patients based on overall survival**

| | **Baseline (Day 0 of Treatment)** | **Day 200 of Treatment** | **Day 400 of Treatment** | **Day 600 of Treatment** |
|---|---|---|---|---|
| **IFNγ High** | 27 | 20 | 7 | 1 |
| **IFNγ Medium** | 26 | 10 | 4 | 1 |
| **IFNγ Low** | 26 | 11 | 3 | 0 |

### Example 4

### Impact of Tumor TDO2 Expression on KYN Reduction

The level of KYN expression was examined in TDO2 high and TDO2 low tumors from patients treated with nivolumab and linrodostat.

Tumor and serum samples were obtained from patients at baseline (cycle 1, day 1; C1D1) and during treatment (cycle 1, day 15; C1D15). Low tumor *TDO2* expression was associated with greater reduction of KYN and reduction of KYN to normal levels in tumor **(****FIG. 4A****)** and serum **(****FIG. 4B****).** High tumor *TDO2* expression was associated with less suppression of KYN, particularly in tumors.

### Example 5

### Association of TDO2 Expression with Response

Since linrodostat selectively inhibits IDO1 but not TDO2 enzymatic activity, high TDO2 expression can drive resistance to nivolumab and linrodostat combination therapy. Therefore, the association between TDO2 expression and clinical response was evaluated. High or low TDO2 expression levels were determined by the median. Based on the objective response rate, 26% of patients with low TDO2 expression responded to treatment while only 13% of patients with high TDO2 expression responded to treatment **(TABLE 4).** Low TDO2 expression was observed among responders in non-melanoma samples with nivolumab and linrodostat treatment **(****FIG. 5A****).** No association between TDO2 expression and response was observed with nivolumab monotherapy **(****FIG. 5B****).**

**TABLE 4. Association of TDO2 Expression and Objective Response Rate**

| ***TDO2* Expression** | **Objective Response Rate** |
|---|---|
| | All immuno-oncology naive samples (n=79) |
| **Low** | 26% |
| **High** | 13% |

### Example 6

### Association of IFNy Signature and TDO2 Expression with Objective Response Rate

The association of IFN-γ and TDO2 with the objective response rate was evaluated for all tumors. High IFNγ signature and low TDO2 expression were associated with the highest response rate to nivolumab and linrodostat treatment in immuno-oncology naive patients **(****FIG. 6A****).** Low IFNγ signature and high TDO2 expression were associated with no response to nivolumab and linrodostat treatment in immuno-oncology naive patients. Additionally, in the non-melanoma subset (n=49), a composite biomarker of IFNγ signature and TDO2 gene expression was more significantly associated with response (P = 0.021) than the IFNγ signature alone (P = 0.063) **(****FIG. 6B****).**

### Example 7

### IFN-γ Signature and TDO2 Expression as a Composite Biomarker Improves Upon IFNγ Alone in Non-Melanoma Cohort

The sensitivity and specificity of expression IFNγ signature genes and TDO2 as a composite biomarker were compared to expression of IFNγ signature genes alone. In the non-melanoma subset (n=49), the composite biomarker, IFNγ and TDO2 expression, performed numerically better **(****FIG. 7A****)** as a predictor of response than IFNγ signature alone in immuno-oncology naive patients (n=79) **(****FIG. 7B****).** In the melanoma subset (n=30), IFNy signature and TDO2 expression [AUC 79% (95% CI, 62-95)] showed no difference in response prediction compared with IFNγ signature alone [AUC 77% (95% CI, 59-95)]. Therefore, IFNγ signature and TDO2 gene expression can function as a composite biomarker to identify patients with certain tumor types more likely to respond to linrodostat mesylate and nivolumab treatment.

## Claims

1. An anti-PD-1 antagonist for use in combination with an indoleamine 2,3-dioxygenase (IDO1) inhibitor in a method for treating a human subject afflicted with a cancer, wherein the subject is identified as exhibiting a combined biomarker comprising (a) a high interferon gamma (*IFN*γ) inflammatory signature score and (b) a low tryptophan 2,3-dioxygenase 2 (*TDO2*) gene expression score prior to administration of the anti-PD-1 antagonist and IDO1 inhibitor;
wherein the *IFNy* inflammatory signature score is determined by measuring the expression of a panel of inflammatory genes comprising *IFNγ*-related genes ("*IFN*γ inflammatory gene panel") in a tumor tissue biopsy sample obtained from the subject;
wherein the cancer is a bladder cancer or a squamous cell carcinoma of the head and neck (SCCHN);
wherein the inflammatory genes comprise *IFNγ, CXCL10, CXCL9, HLA-DRA, IDO1, STAT1, CCR5, CXCL11, GZMA,* and *PRF1*; and
wherein the IDO1 inhibitor is selected from linrodostat mesylate ((2R)-N-(4-chlorophenyl)-2-(cis-4-(6-fluoroquinolin-4-yl)cyclohexyl)propanamide), linrodostat, indoximod, p-(3-benzofuranyl)-DL-alanine, p-[3-benzo(b)thienyl]-DL-alanine; 6-nitro-L-tryptophan, and any combination thereof.

2. The anti-PD-1 antagonist for use of claim 1, wherein the high *IFN*γ inflammatory signature score is **characterized by** an *IFNy* inflammatory signature score that is greater than an average *IFN*γ inflammatory signature score, wherein the average *IFNy* inflammatory signature score is determined by averaging the expression of the genes of the *IFN*γ inflammatory gene panel in cancer samples obtained from a population of subjects afflicted with the cancer.

3. The anti-PD-1 antagonist for use of claim 1, wherein the high *IFN*γ inflammatory signature score is **characterized by** an *IFNγ* inflammatory signature score that is higher than the average *IFNy* inflammatory signature score of a reference sample, wherein the reference sample comprises a non-tumor tissue of the subject, a corresponding non-tumor tissue of the subject, or the corresponding tissue of subjects without a tumor.

4. The anti-PD-1 antagonist for use of any one of claims 1 to 3, wherein the *IFNγ* high inflammatory signature score is **characterized by** an IFNy inflammatory signature score that is at least about 50% higher than the average IFNyinflammatory signature score.

5. The anti-PD-1 antagonist for use of any one of claims 1 to 4, wherein the low *TDO2* gene expression score is **characterized by** a *TDO2* gene expression that is smaller than an average *TDO2* gene expression score, wherein the average *TDO2* gene expression score is determined by averaging the expression of the *TDO2* gene in cancer samples obtained from a population of subjects afflicted with the cancer.

6. The anti-PD-1 antagonist for use of any one of claims 1 to 4, wherein the low *TDO2* gene expression score is **characterized by** a *TDO2* gene expression that is lower than the average *TDO2* gene expression score of a reference sample, wherein the reference sample comprises a non-tumor tissue of the subject, a corresponding non-tumor tissue of the subject, or the corresponding tissue of subjects without a tumor.

7. The anti-PD-1 antagonist for use of claim 5 or 6, wherein the low *TDO2* gene expression score is **characterized by** a *TDO2* gene expression score that is at least about 50% lower than the average *TDO2* gene expression score.

8. The anti-PD-1 antagonist for use of any one of claims 1 to 7, wherein the sample is a formalin-fixed paraffin-embedded tissue or a fresh-frozen tissue.

9. The anti-PD-1 antagonist for use of any one of claims 1 to 8, wherein the expression of the genes in the IFNy inflammatory gene panel and/or *TDO2* gene expression is determined by detecting the presence of a gene mRNA, the presence of a protein encoded by the gene, or both, optionally wherein;
(i) the presence of mRNA encoding the genes in the IFNγ inflammatory gene panel and/or the *TDO2* gene is determined using reverse transcriptase PCR; or
(ii) the presence of the protein encoded by the genes in the IFNγ inflammatory gene panel and/or the *TDO2* gene is determined using an IHC assay.

10. The anti-PD-1 antagonist for use of any one of claims 1 to 9, wherein the anti-PD-1 antagonist is (i) an anti-PD-1 antibody, optionally comprising nivolumab, pembrolizumab, or an antigen-binding portion thereof, or (ii) an anti-PD-L1 antibody, optionally comprising avelumab, atezolizumab, durvalumab, or an antigen-binding portion thereof.

11. The anti-PD-1 antagonist for use of any one of claims 1 to 10, wherein the anti-PD-1 antagonist is nivolumab and the IDO1 inhibitor is linrodostat mesylate ((2R)-N-(4-chlorophenyl)-2-(cis-4-(6-fluoroquinolin-4-yl)cyclohexyl)propanamide).

12. The anti-PD-1 antagonist for use of claim 10 or 11, wherein:
(i) the anti-PD-1 or anti-PD-L1 antibody is to be administered at a dose of at least about 3 mg/kg body weight once about every 2 weeks; or
(ii) the anti-PD-1 or anti-PD-L1 antibody is to be administered at a flat dose of about 240 mg once about every two weeks, or about 480 mg once about every four weeks.

13. The anti-PD-1 antagonist for use of any one of claims 1 to 12, wherein the anti-PD-1 antagonist is an anti-PD-1 antibody, which is to be administered intravenously at a dose of 240 mg every two weeks or 480 mg every four weeks, and the IDO1 inhibitor is to be administered orally at a dose of 100 mg or 200 mg every day.

14. The anti-PD-1 antagonist for use of any one of claims 1 to 13, wherein the cancer is relapsed, refractory, locally advanced, metastatic, or any combination thereof.

## Patentansprüche

1. Anti-PD-1-Antagonist zur Verwendung in Kombination mit einem Indolamin-2,3-Dioxygenase- (IDO1) Inhibitor in einem Verfahren zur Behandlung eines an Krebs erkrankten Subjekts, wobei das Subjekt vor einer Verabreichung des Anti-PD-1-Antagonisten und des IDO1-Inhibitors identifiziert wird, einen kombinierten Biomarker aufzuweisen, der (a) einen hohen Interferon-gamma- (*IFNγ*) Entzündungssignaturwert und (b) einen niedrigen Tryptophan-2,3-Dioxygenase-2- (*TDO2*) Genexpressionswert umfasst;
wobei der *IFNγ*-Entzündungssignaturwert durch Messung der Expression eines Panels von Entzündungsgenen, das IFNy-bezogene Gene ("*IFN*γ*-*Entzündungsgenpanel") umfasst, in einer vom Subjekt gewonnenen Tumorgewebeprobe bestimmt wird;
wobei es sich bei dem Krebs um einen Blasenkrebs oder ein Plattenepithelkarzinom des Kopfes und Halses (SCCHN) handelt;
wobei die Entzündungsgene *IFNγ*, *CXCL10, CXCL9, HLA-DRA, IDO1, STAT1, CCR5, CXCL11, GZMA* und *PRF1* umfassen; und
wobei der IDO1-Inhibitor aus Linrodostatmesylat-((2R)-N-(4-chlorphenyl)-2-(cis-4-(6-fluorchinolin-4-yl)cyclohexyl)propanamid), Linrodostat, Indoximod, p-(3-Benzoftiranyl)-DL-alanin, p-[3-Benzo(b)thienyl]-DL-alanin; 6-Nitro-L-tryptophan und einer Kombination davon ausgewählt ist.

2. Anti-PD-1-Antagonist zur Verwendung nach Anspruch 1, wobei der hohe *IFNγ-*Entzündungssignaturwert durch einen *IFNγ*-Entzündungssignaturwert gekennzeichnet ist, der größer ist als ein durchschnittlicher IFNy-Entzündungssignaturwert, wobei der durchschnittliche *IFNγ*-Entzündungssignaturwert durch Mittelung der Expression der Gene des IFNy-Entzündungsgenpanels in Krebsgewebeproben bestimmt wird, die aus einer Population von an Krebs erkrankten Subjekten gewonnen wurden.

3. Anti-PD-1-Antagonist zur Verwendung nach Anspruch 1, wobei der hohe *IFNγ-*Entzündungssignaturwert durch einen *IFNγ*-Entzündungssignaturwert gekennzeichnet ist, der höher ist als der durchschnittliche *IFNγ-*Entzündungssignaturwert einer Referenzprobe, wobei die Referenzprobe ein nicht-tumoriges Gewebe des Subjekts, ein entsprechendes nicht-tumoriges Gewebe des Subjekts oder das entsprechende Gewebe von Subjekten ohne einen Tumor umfasst.

4. Anti-PD-1-Antagonist zur Verwendung nach einem der Ansprüche 1 bis 3, wobei der *IFNγ*-Entzündungssignaturwert durch einen *IFNγ*-Entzündungssignaturwert gekennzeichnet ist, der mindestens etwa 50% höher ist als der durchschnittliche *IFNγ-*Entzündungssignaturwert.

5. Anti-PD-1-Antagonist zur Verwendung nach einem der Ansprüche 1 bis 4, wobei der niedrige TD02-Genexpressionswert durch eine *TDO2*-Genexpression gekennzeichnet ist, die kleiner ist als ein durchschnittlicher *TDO2*-Genexpressionswert, wobei der durchschnittliche TDO2-Genexpressionswert durch Mittelung der Expression des *TDO2-*Gens in Krebsgewebeproben bestimmt wird, die aus einer Population von an dem Krebs erkrankten Subjekten gewonnen wurden.

6. Anti-PD-1-Antagonist zur Verwendung nach einem der Ansprüche 1 bis 4, wobei der niedrige TD02-Genexpressionswert durch eine *TDO2-*Genexpression gekennzeichnet ist, die niedriger ist als der durchschnittliche *TDO2*-Genexpressionswert einer Referenzprobe, wobei die Referenzprobe ein nicht-tumoriges Gewebe des Subjekts, ein entsprechendes nicht-tumoriges Gewebe des Subjekts oder das entsprechende Gewebe von Subjekten ohne einen Tumor umfasst.

7. Anti-PD-1-Antagonist zur Verwendung nach Anspruch 5 oder 6, wobei der niedrige TDO2-Genexpressionswert durch einen TDO2-Genexpressionswert gekennzeichnet ist, der mindestens etwa 50 % niedriger ist als der durchschnittliche *TDO2-*Genexpressionswert.

8. Anti-PD-1-Antagonist zur Verwendung nach einem der Ansprüche 1 bis 7, wobei es sich bei der Probe um ein formalinfixiertes, paraffineingebettetes Gewebe oder ein frisch gefrorenes Gewebe handelt.

9. Anti-PD-1-Antagonist zur Verwendung nach einem der Ansprüche 1 bis 8, wobei die Expression der Gene im IFNy-Entzündungsgenpanel und/oder die TDO2-Genexpression durch einen Nachweis der Anwesenheit einer Gen-mRNA, der Anwesenheit eines von dem Gen kodierten Proteins oder beider bestimmt wird, wobei optional;
(i) das Vorhandensein von mRNA, die für die Gene in dem *IFNγ-*Entzündungsgenpanel und/oder das TDO2-Gen kodiert, mittels reverser Transkriptase-PCR bestimmt wird; oder
(ii) das Vorhandensein des Proteins, das von den Genen im *IFNγ-*Entzündungsgenpanel und/oder dem TDO2-Gen kodiert wird, mittels eines IHC-Tests bestimmt wird.

10. Anti-PD-1-Antagonist zur Verwendung nach einem der Ansprüche 1 bis 9, wobei es sich bei dem Anti-PD-1-Antagonisten um (i) einen Anti-PD-1-Antikörper handelt, der optional Nivolumab, Pembrolizumab oder einen Antigen-bindenden Teil davon umfasst, oder (ii) einen Anti-PD-L1-Antikörper, der optional Avelumab, Atezolizumab, Durvalumab oder einen Antigen-bindenden Teil davon umfasst.

11. Anti-PD-1-Antagonist zur Verwendung nach einem der Ansprüche 1 bis 10, wobei es sich bei dem Anti-PD-1-Antagonisten um Nivolumab und bei dem IDO1-Inhibitor um Linrodostatmesylat-((2R)-N-(4-chlorphenyl)-2-(cis-4-(6-fluorchinolin-4-yl)cyclohexyl)propanamid) handelt.

12. Anti-PD-1-Antagonist zur Verwendung nach Anspruch 10 oder 11, wobei:
(i) der Anti-PD-1- oder Anti-PD-L1-Antikörper in einer Dosis von mindestens etwa 3 mg/kg Körpergewicht etwa alle 2 Wochen zu verabreichen ist; oder
(ii) der Anti-PD-1- oder Anti-PD-L1-Antikörper in einer festen Dosis von etwa 240 mg einmal alle zwei Wochen oder etwa 480 mg einmal alle vier Wochen zu verabreichen ist.

13. Anti-PD-1-Antagonist zur Verwendung nach einem der Ansprüche 1 bis 12, wobei es sich bei dem Anti-PD-1-Antagonisten um einen Anti-PD-1-Antikörper handelt, der intravenös in einer Dosis von 240 mg alle zwei Wochen oder 480 mg alle vier Wochen zu verabreichen ist, und der IDO1-Inhibitor oral in einer Dosis von 100 mg oder 200 mg täglich zu verabreichen ist.

14. Anti-PD-1-Antagonist zur Verwendung nach einem der Ansprüche 1 bis 13, wobei der Krebs wiederkehrend, widerstandsfähig, lokal fortgeschritten, metastasiert oder eine Kombination davon ist.

## Revendications

1. Antagoniste anti-PD-1 pour utilisation en combinaison avec un inhibiteur de l'indoleamine 2,3-dioxygénase (IDO1) dans un procédé de traitement d'un sujet humain atteint d'un cancer, dans lequel le sujet est identifié comme présentant un biomarqueur combiné comprenant (a) un score élevé de signature inflammatoire de l'interféron gamma (*IFNγ*) et (b) un faible score d'expression du gène de la tryptophane 2,3-dioxygénase 2 (*TDO2*) avant l'administration de l'antagoniste anti-PD-1 et de l'inhibiteur de l'IDO1;
dans lequel le score de signature inflammatoire *IFNγ* est déterminé en mesurant l'expression d'un panel de gènes inflammatoires comprenant des gènes liés à *l'IFNγ* (« panel de gènes inflammatoires *IFNγ* ») dans un échantillon de biopsie de tissu tumoral prélevé sur le sujet ;
dans lequel le cancer est un cancer de la vessie ou un carcinome épidermoïde de la tête et du cou (SCCHN) ;
dans lequel les gènes inflammatoires comprennent *IFNγ, CXCL10, CXCL9, HLA-DRA, IDO1, STAT1, CCR5, CXCL11, GZMA* et *PRF1* ; et
dans lequel l'inhibiteur de l'IDO1 est sélectionné parmi le mésylate de linrodostat ((2R)-N-(4-chlorophényl)-2-(cis-4-(6-fluoroquinolin-4-yl)cyclohexyl)propanamide), le linrodostat, l'indoximod, la p-(3-benzoftiranyl)-DL-alanine, la p-[3-benzo(b)thienyl]-DL-alanine ; le 6-nitro-L-tryptophane, et toute combinaison de ceux-ci

2. Antagoniste anti-PD-1 pour utilisation selon la revendication 1, dans lequel le score élevé de signature inflammatoire *IFNγ* est **caractérisé par** un score de signature inflammatoire *IFNγ* qui est supérieur à un score de signature inflammatoire *IFNγ* moyen, dans lequel le score de signature inflammatoire *IFNy* moyen est déterminé par calcul de la moyenne de l'expression des gènes du panel de gènes inflammatoires *IFNy* dans des échantillons de cancer prélevés sur une population de sujets atteints du cancer.

3. Antagoniste anti-PD-1 pour utilisation selon la revendication 1, dans lequel le score élevé de signature inflammatoire *IFNγ* est **caractérisé par** un score de signature inflammatoire *IFNγ* qui est supérieur au score de signature inflammatoire *IFNγ* moyen d'un échantillon de référence, dans lequel l'échantillon de référence comprend un tissu non tumoral du sujet, un tissu non tumoral correspondant du sujet ou le tissu correspondant de sujets sans tumeur.

4. Antagoniste anti-PD-1 pour utilisation selon l'une quelconque des revendications 1 à 3, dans lequel le score élevé de signature inflammatoire *IFNγ* est **caractérisé par** un score de signature inflammatoire *IFNγ* qui est au moins environ 50 % supérieur au score de signature inflammatoire *IFNγ* moyen.

5. Antagoniste anti-PD-1 pour utilisation selon l'une quelconque des revendications 1 à 4, dans lequel le faible score d'expression du gène *TDO2* est **caractérisé par** une expression du gène *TDO2* qui est inférieure à un score d'expression moyen du gène *TDO2,* dans lequel le score d'expression moyen du gène *TDO2* est déterminé par calcul de la moyenne de l'expression du gène *TDO2* dans des échantillons de cancer prélevés sur une population de sujets atteints du cancer.

6. Antagoniste anti-PD-1 pour utilisation selon l'une quelconque des revendications 1 à 4, dans lequel le faible score d'expression du gène *TDO2* est **caractérisé par** une expression du gène *TDO2* qui est inférieure au score d'expression moyen du gène *TDO2* d'un échantillon de référence, dans lequel l'échantillon de référence comprend un tissu non tumoral du sujet, un tissu non tumoral correspondant du sujet ou le tissu correspondant de sujets sans tumeur.

7. Antagoniste anti-PD-1 pour utilisation selon la revendication 5 ou 6, dans lequel le faible score d'expression du gène *TDO2* est **caractérisé par** un score d'expression du gène *TDO2* qui est au moins environ 50 % inférieur au score d'expression moyen du gène *TDO2.*

8. Antagoniste anti-PD-1 pour utilisation selon l'une quelconque des revendications 1 à 7, dans lequel l'échantillon est un tissu fixé au formol et inclus dans de la paraffine ou un tissu frais congelé.

9. Antagoniste anti-PD-1 pour utilisation selon l'une quelconque des revendications 1 à 8, dans lequel l'expression des gènes dans le panel de gènes inflammatoires *IFNy* et/ou l'expression du gène *TDO2* est déterminée par détection de la présence d'un ARNm de gène, la présence d'une protéine codée par le gène, ou des deux, éventuellement dans lequel :
(i) la présence d'ARNm codant pour les gènes dans le panel de gènes inflammatoires *IFNγ* et/ou le gène *TDO2* est déterminée par PCR à transcription inverse ; ou
(ii) la présence de la protéine codée par les gènes dans le panel de gènes inflammatoires *IFNγ* et/ou le gène *TDO2* est déterminée par un test en IHC.

10. Antagoniste anti-PD-1 pour utilisation selon l'une quelconque des revendications 1 à 9, dans lequel l'antagoniste anti-PD-1 est (i) un anticorps anti-PD-1, comprenant éventuellement du nivolumab, du pembrolizumab ou une partie de liaison à l'antigène de celui-ci, ou (ii) un anticorps anti-PD-L1, comprenant éventuellement de l'avelumab, de l'atezolizumab, du durvalumab ou une partie de liaison à l'antigène de celui-ci.

11. Antagoniste anti-PD-1 pour utilisation selon l'une quelconque des revendications 1 à 10, dans lequel l'antagoniste anti-PD-1 est le nivolumab et l'inhibiteur de l'IDO1 est le mésylate de linrodostat ((2R)-N-(4-chlorophényl)-2-(cis-4-(6-fluoroquinolin-4-yl)cyclohexyl)propanamide).

12. Antagoniste anti-PD-1 pour utilisation selon la revendication 10 ou 11, dans lequel :
(i) l'anticorps anti-PD-1 ou anti-PD-L1 est à administrer en une dose d'au moins environ 3 mg/kg du poids corporel environ toutes les 2 semaines ; ou
(ii) l'anticorps anti-PD-1 ou anti-PD-L1 est à administrer en une dose fixe d'environ 240 mg, une fois toutes les deux semaines environ, ou d'environ 480 mg une fois toutes les quatre semaines environ.

13. Antagoniste anti-PD-1 pour utilisation selon l'une quelconque des revendications 1 à 12, dans lequel l'antagoniste anti-PD-1 est un anticorps anti-PD-1, qui est à administrer par voie intraveineuse en une dose de 240 mg toutes les deux semaines ou de 480 mg toutes les quatre semaines, et l'inhibiteur de l'IDO1 est à administrer par voie orale en une dose de 100 mg ou de 200 mg par jour.

14. Antagoniste anti-PD-1 pour utilisation selon l'une quelconque des revendications 1 à 13, dans lequel le cancer est récidivant, réfractaire, localement avancé, métastatique ou toute combinaison de ceux-ci.
